# EUROPÄISCHE PATENTSCHRIFT

(11) **EP 1 786 823 B1**
(45) Veröffentlichungstag und Bekanntmachung des Hinweises auf die Patenterteilung: **16.01.2008**
(21) Anmeldenummer: 05786302.9
(22) Anmeldetag: 02.09.2005
(51) Int. Cl.: C07F 5/02, C07D 215/38, C07D 215/22, C07D 237/32, C07D 239/84, C07C 69/02, A61K 31/4704, A61K 31/517, A61K 31/502, A61P 29/00

(54) **ALKYLIDEN-TETRAHYDRONAPHTHALINDERIVATE, VERFAHREN ZU IHRER HERSTELLUNG UND IHRE VERWENDUNG ALS ENTZÜNDUNGSHEMMER**
ALKYLIDENE TETRAHYDRONAPHTHALENE DERIVATIVES, METHOD FOR THEIR PRODUCTION AND THEIR USE AS ANTI-INFLAMMATORY AGENTS
DERIVES D'ALKYLIDENE-TETRAHYDRONAPHTALENE, PROCEDES POUR LEUR PRODUCTION ET LEUR UTILISATION COMME ANTI-INFLAMMATOIRES

(30) Priorität: 09.09.2004 DE 102004044680
(43) Veröffentlichungstag der Anmeldung: 23.05.2007
(73) Patentinhaber: Bayer Schering Pharma Aktiengesellschaft, 13353 Berlin (DE)
(72) Erfinder: BÄURLE, Stefan, 10245 Berlin (DE); BERGER, Markus, 13347 Berlin (DE); JAROCH, Stefan, 10629 Berlin (DE); KROLIKIEWICZ, Konrad, 12357 Berlin (DE); NGUYEN, Duy, 10179 Berlin (DE); REHWINKEL, Hartmut, 10961 Berlin (DE); SCHÄCKE, Heike, 10115 Berlin (DE); SCHMEES, Norbert, 13469 Berlin (DE); SKUBALLA, Werner, 13465 Berlin (DE)
(86) Internationale Anmeldenummer: PCT/EP2005/009623
(87) Internationale Veröffentlichungsnummer: WO 2006/027236

(56) Entgegenhaltungen:
- WO-A-02/10143
- WO-A-96/20930
- WO-A-03/082280
- WO-A-20/05034939
- EVANS D A ET AL: "C2-Symmetric Copper(II) Complexes as Chiral Lewis Acids. Catalytic Enantioselective Carbonyl-Ene Reactions with Glyoxylate and Pyruvate Esters" JOURNAL OF THE AMERICAN CHEMICAL SOCIETY, AMERICAN CHEMICAL SOCIETY, WASHINGTON, DC, US, Bd. 122, 2000, Seiten 7936-7943, XP002221658 ISSN: 0002-7863
- CLEGHORN L A T ET AL: "Three-component bimetallic (Pd/In) mediated cascade allylation of C=X functionality - Part 1. Scope and Class 1 examples with aldehydes and ketones" JOURNAL OF ORGANOMETALLIC CHEMISTRY, ELSEVIER-SEQUOIA S.A. LAUSANNE, CH, Bd. 687, Nr. 2, 7. Dezember 2003 (2003-12-07), Seiten 483-493, XP004475449 ISSN: 0022-328X

## Beschreibung

Die Erfindung betrifft Alkyliden-Tetrahydronaphthalinderivate, Verfahren zu ihrer Herstellung und ihre Verwendung als Entzündungshemmer.

Aus dem Stand der Technik WO02/10143 und WO 03/082827 sind offenkettige nicht steroidale Entzündungshemmer bekannt. Diese Verbindungen zeigen im Experiment Wirkdissoziationen zwischen antiinflammatorischen und unerwünschten metabolischen Wirkungen und sind den bisher beschriebenen, nichtsteroidalen Glucocorticoiden überlegen oder weisen zumindest eine ebenso gute Wirkung auf.

Die Selektivität der Verbindungen des Standes der Technik gegenüber den anderen Steroidrezeptoren ist jedoch noch verbesserungsbedürftig.
Daher war es Aufgabe der vorliegenden Erfindung, Verbindungen zur Verfügung zu stellen, deren Selektivität gegenüber den anderen Steroidrezeptoren verbessert, mindestens jedoch vergleichbar ist.

Diese Aufgabe wird durch die Verbindungen der vorliegenden Erfindung, dargelegt in den Patentansprüchen, gelöst.
Die vorliegende Erfindung betrifft Verbindungen der allgemeinen Formel (I), 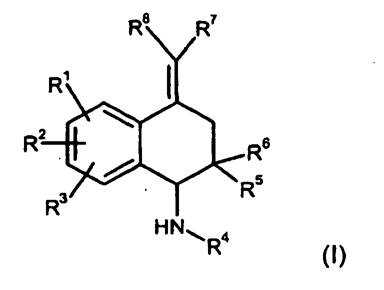 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
   oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe,
   eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹²-Gruppen), (C₁-C₅)-Alkoxygruppen, Hydroxygruppen, Halogenatome, (C₁-C₃)Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R¹² eine (C₁-C₅)-Alkyl-, oder eine Benzyl-Gruppe bedeutet,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinylgruppe eine gegebenenfalls durch eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe,
   eine Heteroaryl(C₁-C₈)alkylgruppe oder eine Heteroaryl(C₂-C₈)alkenylgruppe
   wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann, oder gemeinsam mit dem Kohlenstoffatom der Methylengruppe einen (C₃-C₆)-Cycloalkylring, oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-2 Hydroxygruppen, 1-4 Halogenatome substituiert ist, bedeuten.

Ein weiterer Gegenstand der Erfindung sind Verbindungen der allgemeinen Formel I, worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
   oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe,
   eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹²-Gruppen), (C₁-C₅)-Alkoxygruppen, Hydroxygruppen, Halogenatome, (C₁-C₃)Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R¹² eine (C₁-C₅)-Alkyl-oder eine Benzyl-Gruppe bedeutet,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinylgruppe eine gegebenenfalls durch
   eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe,
   eine Heteroaryl(C₁-C₈)alkylgruppe oder eine Heteroaryl(C₂-C₈)alkenylgruppe
   wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann, oder gemeinsam mit dem Kohlenstoffatom der Methylengruppe einen (C₃-C₆)-Cycloalkylring, oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-2 Hydroxygruppen, 1-4 Halogenatome substituiert ist, bedeuten.

Ein weiterer Gegenstand der Erfindung sind Verbindungen der allgemeinen Formel I, worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
   oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe,
   eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR⁶-Gruppen), (C₁-C₅)-Alkoxygruppen, Hydroxygruppen, Halogenatome, (C₁-C₃)Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkeflylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinylgruppe eine gegebenenfalls durch
   eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe,
   eine Heteroaryl(C₁-C₈)alkylgruppe oder eine Heteroaryl(C₂-C₈)alkenylgruppe
   wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann, oder gemeinsam mit dem Kohlenstoffatom der Methylengruppe einen (C₃-C₆)-Cycloalkylring, oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist, bedeuten.

Ein weiterer Gegenstand der Erfindung sind Stereoisomere der allgemeinen Formel (1), worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, wobei n = 1 oder 2 ist und die endständigen Atome mit direkt benachbarten Ring-Kohlenstoffatomen verknüpft sind, oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Hydroxygruppen, 1-3 Halogenatome, 1-2 (C₁-C₃)-Exoalkylidengruppen, substituierte 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppen über eine beliebige Position mit dem Amin des Tetrahydronaphthalinsystems verknüpft sein und gegebenenfalls an einer oder mehreren Stellen hydriert sein können,
R⁵ eine Hydroxygruppe
R⁶ eine (C₁-C₅)-Alkylgruppe oder eine gegebenenfalls teilweise oder vollständig fluorierte (C₁-C₅)-Alkylgruppe, eine Arylgruppe, eine Aryl(C₁-C₈)alkylgruppe, Aryl(C₂-C₈)alkenylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe
R⁷ und R⁸ unabhängig voneinander ein Wasserstoffatom, ein Halogenatom, eine Methyl- oder Ethylgruppe, die mit OR¹⁰, SR¹⁰, N(R¹⁰)₂ substituiert sein dürfen, oder gemeinsam mit dem Kohlenstoffatom der Methylengruppe einen (C₃-C₆)-Cycloalkylring oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist, bedeuten.

Ein Gegenstand der vorliegenden Erfindung sind Stereoisomere der allgemeinen Formel (I), worin
R¹ und R² unabhängig voneinander ein Wasserstoffatom, eine Hydroxy-Gruppe, ein Halogenatom, eine gegebenenfalls substituierte (C₁-C₅)-Alkylgruppe, eine (C₁-C₅)-Alkoxygruppe,
   oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
   wobei n = 1 oder 2 ist und die endständigen Atome mit direkt benachbarten Ring-Kohlenstoffatomen verknüpft sind,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe,
R⁴ eine C₁-C₁₀-Alkylgruppe, eine durch 1-3 Hydroxygruppen, Halogenatome substituierte C₁-C₁₀-Alkylgruppe, eine gegebenenfalls durch eine oder mehrere Gruppen ausgewählt aus 1-2 Ketogruppen, 1-2 (C₁-C₅)-Alkylgruppen, 1-2 (C₁-C₅)-Alkoxygruppen, 1-3 Hydroxygruppen, 1-3 Halogenatome, 1-2 (C₁-C₃)-Exoalkylidengruppen substituierte Phenyl-, Naphthyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, lsochinolinyl-, Chinolonyl-, Isochinolonyl-, Indazolyl-, Benzothiazolyl- , Chinazolinyl-, Chinoxalinyl-, Cinnolinyl-, Phthalazinyl, 1,7-oder 1,8-Naphthyridinyl-, Dihydroindolonyl-, Dihydroisoindolonyl-, Benzimidazol- oder Indolylgruppe,
   wobei diese Gruppen über eine beliebige Position mit dem Amin des Tetrahydronaphthalinsystems verknüpft sein können und gegebenenfalls an einer oder mehreren Stellen hydriert sein können
R⁵ eine Hydroxygruppe
R⁶ eine (C₁-C₅)-Alkylgruppe oder eine gegebenenfalls teilweise oder vollständig fluorierte (C₁-C₅)-Alkylgruppe
R⁷ und R⁸ unabhängig voneinander ein Wasserstoffatom, ein Halogenatom, eine Methyl- oder Ethylgruppe, die mit OR¹⁰, SR¹⁰, N(R⁹R¹⁰) substituiert sein dürfen, oder gemeinsam mit dem Kohlenstoffatom der Methylengruppe einen (C₃-C₆)-Cycloalkylring oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist, bedeuten.

Ein weiterer Gegenstand der vorliegenden Erfindung sind Stereoisomere der allgemeinen Formel (I), worin
R¹ und R¹ unabhängig voneinander ein Wasserstoffatom, eine Hydroxy-Gruppe, ein Halogenatom, eine (C₁-C₅)-Alkylgruppe, eine (C₁-C₅)-Alkoxygruppe, oder zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
   wobei n = 1 oder 2 ist und die endständigen Atome mit direkt benachbarten Ring-Kohfenstoffatomen verknüpft sind,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe,
R⁴ eine gegebenenfalls mit C₁-C₅-Alkyl, Halogen, Hydroxy, C₁-C₅-Alkoxy, Ketogruppen, (C₁-C₃)-Exoalkylidengruppen substituierte Phenyl-, Naphthyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl-, Dihydroisoindolonyl-, Benzimidazol- oder Indolylgruppe,
   wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine (C₁-C₅)-Alkylgruppe oder eine gegebenenfalls teilweise oder vollständig fluorierte (C₁-C₅)-Alkylgruppe
R⁷ und R⁸ unabhängig voneinander ein Wasserstoffatom, ein Halogenatom, eine Methyl- oder Ethylgruppe, oder gemeinsam mit dem Kohlenstoffatom der Methylengruppe einen (C₃-C₆)-Cycloalkylring oder
R¹ und R⁸ gemeinsam einen annelierten fünf- bis achtglierigen gesättigten oder ungesättigten Carbo- oder Heterozyklus, bedeuten.

Besonders bevorzugt sind Verbindungen der allgemeinen Formel I, worin
R¹ und R² unabhängig voneinander ein Wasserstoffatom, eine Hydroxy-Gruppe, ein Halogenatom, eine (C₁-C₅)-Alkoxygruppe,
R³ ein Wasserstoffatom, ein Halogenatom,
R⁴ eine gegebenenfalls mit C₁-C₅-Alkyl, Halogen, Ketogruppen, substituierte Chinolinyl-, Chinolonyl-, Chinazolinyl-, Phthalazinonylgruppe,
   wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine gegebenenfalls teilweise oder vollständig fluorierte (C₁-C₅)-Alkylgruppe
R⁷ und R⁸ unabhängig voneinander ein Wasserstoffatom, eine Methyl- oder Ethylgruppe, oder
R¹ und R⁸ gemeinsam einen annelierten fünf- bis achtgliedrigen gesättigten oder ungesättigten Carbo- oder Heterozyklus, bedeuten.

Ganz besonders bevorzugt sind Verbindungen der allgemeinen Formel 1, worin
R¹ und R² unabhängig voneinander ein Wasserstoffatom, eine Hydroxy-Gruppe, ein Fluor- oder Chloratom, eine Methoxygruppe,
R³ ein Wasserstoffatom oder ein Chloratom,
R⁴ eine gegebenenfalls mit einer oder mehren Gruppen ausgewählt aus Methyl-, Hydroxy-, Ketogruppe oder Fluoratom, substituierte Chinolinyl-, Chinolonyl-, Chinazolinyl- oder Phtalazinonylgruppe
   wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine Trifluormethylgruppe
R⁷ und R⁸ unabhängig voneinander ein Wasserstoffatom, eine Methyl- oder Ethylgruppe, oder
R¹ und R⁸ gemeinsam einen annelierten sechsgliedrigen Heterozyklus, bedeuten, der gegebenenfalls durch eine Hydroxygruppe substituiert ist.

Ein weiterer Gegenstand der Erfindung sind Stereoisomere der allgemeinen Formel (I) gemäß Anspruch 1, worin R¹ und R⁸ gemeinsam einen annelierten sechsgliedrigen Heterozyklus, bedeuten, der ein Sauerstoffatom und ein Boratom enthält und der gegebenenfalls durch eine Hydroxygruppe substituiert ist.

Die Bezeichnung Halogenatom oder Halogen bedeutet ein Fluor-, Chlor-, Brom-oder lodatom. Bevorzugt ist ein Fluor-, Chlor- oder Bromatom. Besonders bevorzugt sind das Fluor- und das Chloratom.

Die in den Ansprüchen genannten Alkylgruppen, insbesondere R¹, R², R³, R⁶, R⁷, R⁸, R⁹, R¹⁰, R¹¹ und R¹² können geradkettig oder verzweigt sein und beispielsweise für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe stehen. Eine C₁-C₃-Alkylgruppe ist bevorzugt.
Sie können gegebenenfalls substituiert sein durch eine Gruppe ausgewählt aus 1-3 Hydroxy-, 1-3 Halogenatome, 1-3 (C₁-C₃)Alkoxy-, und/oder 1-3- COOR¹¹ Gruppen. Bevorzugt sind Hydroxygruppen.

Die Alkylgruppe R⁴ hat die im vorstehenden Absatz genannte Bedeutung, jedoch sind die möglichen Substituenten ausgewählt aus der Gruppe Hydroxy, Halogen, (C₁-C₅)-Alkyloxy.

Die Alkylgruppen R⁷ und R⁸ haben die im ersten, die Alkylgruppen betreffenden, Absatz genannte Bedeutung, jedoch sind die möglichen Substituenten ausgewählt aus der Gruppe OR¹⁰, SR¹⁰ und N(R⁹R¹⁰), wobei R⁹ und R¹⁰ Wasserstoff, C₁-C₅-Alkyl oder (CO)C₁-C₅-Alkyl bedeutet und Alkyl ebenfalls wie oben definiert ist.

Die Alkoxygruppen können geradkettig oder verzweigt sein und für eine Methoxy-, Ethoxy-, n-Propoxy-, iso-Propoxy-, n-Butoxy, iso-Butoxy, tert.-Butoxy-oder n-Pentoxy-, 2,2-Dimethylpropoxy-, 2-Methylbutoxy- oder 3-Methylbutoxygruppe stehen. Eine Methoxy- oder Ethoxygruppe ist bevorzugt.

Die Alkylthiogruppen können geradkettig oder verzweigt sein und für eine Methylthio-, Ethylthio-, n-Propylthio-, iso-Propylthio-, n-Butylthio, iso-Butylthio, tert.-Butylthio- oder n-Pentylthio-, 2,2-Dimethylpropylthio-, 2-Methylbutylthio-oder 3-Methylbutylthiogruppe stehen. Eine Methylthio- oder Ethylthiogruppe ist bevorzugt.

Für eine teilweise oder vollständig fluorierte Alkylgruppe, die geradkettig oder verzweigt sein kann, kommen beispielsweise die teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl, C₃F₇, C₃H₂F₅, C₄F₉, C₅F₁₁. Von diesen bevorzugt sind die Trifluormethyl- oder die Pentafluorethylgruppe. Die Reagenzien sind käuflich oder die publizierten Synthesen der entsprechenden Reagenzien gehören zum Stand der Technik.
Die Arylsubstituenten R¹ und R² können einen Ring bilden, indem beide Arylsubstituenten gemeinsam eine Kette bedeuten ausgewählt aus den Gruppen -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, -NH-N=CH-, wobei n = 1 oder 2 ist. Die endständigen Atome der oben aufgeführten Gruppen sind mit direkt benachbarten Aryl-Ring-Kohlenstoffatomen verknüpft, so dass ein annelierter Ring entsteht.

Der Substituent NR⁹R¹⁰ bedeutet beispielsweise NH₂, NH(CH₃), N(CH₃)₂, NH(C₂H₅), N(C₂H₅)₂, NH(C₃H₇), N(C₃H₇)₂, NH(C₄H₉), N(C₄H₉)₂, NH(C₅H₁₁), N(C₅H₁₁)₂, NH(CO)CH₃, NH(CO)C₂H₅, NH(CO)C₃H₇, NH(CO)C₄H₉, NH(CO)C₅H₁₁.

Die Cycloalkylgruppe bedeutet eine durch eine oder mehrere Gruppen ausgewählt aus Hydroxygruppen, Halogenatomen, (C₁-C₅)-Alkylgruppen, (C₁-C₅)-Alkoxygruppen gegebenenfalls substituierte gesättigte zyklische Gruppe mit 3 bis 7 Ringkohlenstoffatomen wie beispielsweise Cyclopropyl, Methylcyclopropyl, Cyclobutyl, Methylcyclobutyl, Cylopentyl, Methylcyclopentyl, Cyclohexyl, Methylcyclohexyl, Cycloheptyl, Methylcycloheptyl.

Die Cycloalkylalkylgruppe bedeutet beispielsweise -(CH₂)-Cycloalkyl, - (C₂H₄)-Cycloalkyl, -(C₃H₆)-Cycloalkyl, -(C₄H₈)-Cycloalkyl, -(C₅H₁₀)Cycloalkyl, wobei Cycloalkyl definiert ist wie oben beschrieben.

Cycloalkylalkenylgruppe bedeutet beispielsweise -(CH=CH)-Cycloalkyl, - [C(CH₃)=CH]-Cycloalkyl, -[CH=C(CH₃)]-Cycloalkyl, -(CH=CH-CH₂)-Cycloalkyl, -(CH₂-CH=CH)-Cycloalkyl, -(CH=CH-CH₂-CH₂)-Cycloalkyl, -(CH₂-CH=CH-CH₂)-Cycloalkyl, -(CH₂-CH₂-CH=CH)-Cycloalkyl, -(C(CH₃)=CH-CH₂)-Cycloalkyl, -(CH=C(CH₃)-CH₂)-Cycloalkyl.

Als (C₁-C₃)-Exoalkylidengruppe ist eine Gruppe zu verstehen, die über eine Exodoppelbindung an das System (Ring oder Kette) gebunden ist. Bevorzugt ist Exomethylen.

Die Heterocyclylgruppe ist nicht aromatisch und kann beispielsweise Pyrrolidin, Imidazolidin, Pyrazolidin, Piperidin sein. Als Substituenten kommen Hydroxygruppen, Halogenatomen, (C₁-C₅)-Alkylgruppen, (C₁-C₅)-Alkoxygruppen in Frage.
Unter Heterozyklylalkylgruppen sind Heterozyklylgruppen zu verstehen, die übereine C₁-C₅-Alkylgruppe an das Grundgerüst gebunden sind, wobei die Alkylgruppe geradkettig oder verzweigt sein kann.

Heterocyclylalkenylgruppen sind Heterozyklylgruppen, die über eine ungesättigte C₂-C₅-Alkylgruppe an das Gerüst gebunden sind, wobei die Alkenylengruppen geradkettig oder verzweigt sein können.
Die Arylgruppe R⁴ und R⁶ kann Phenyl oder Naphthyl sein.
Als Substituenten für beide Gruppen kommen C₁-C₃-Alkyl, Hydroxy, C₁-C₃-Alkoxy, C₁-C₃-Alkylthio, Halogen, Cyano, COO(C₁-C₅)alkyl, COOH, N(R⁹R¹⁰), Nitro, in Betracht. Der Substitutionsgrad kann ein oder mehrfach sein, mehrere gleiche oder verschiedene Substituenten beinhalten. Mono- oder disubstituierte Phenyl- und Naphthylgruppen R⁴ sind bevorzugt.
Die Arylgruppen können teilweise hydriert sein und dann zusätzlich oder alternativ zu den oben aufgeführten Substituenten auch Keto, (C₁-C₃)-Exoalkyliden tragen. Unter teilweise hydriertem Phenyl ist z.B. Cyclohexadienyl, Cyclohexenyl, Cyclohexyl zu verstehen. Ein teilweise hydriertes substituiertes Naphthalinsystem ist beispielsweise 1-Tetraton oder 2-Tetralon.
Die Arylalkylgruppe ist eine Arylgruppe, die über eine C₁-C₈-Alkylgruppe an ein Grundgerüst gebunden ist, wobei die Alkylgruppe geradkettig oder verzweigt sein kann. Beispielsweise seien Benzyl oder Phenethylen genannt.

Eine Arylalkenylgruppe ist eine Arylgruppe, die über eine C₂-C₈-Alkenylgruppe an ein Grundgerüst gebunden ist, wobei die Alkenylgruppe geradkettig oder verzweigt sein kann.

Die Arylalkinylgruppe ist eine Arylgruppe, die über eine C₂-C₈-Alkinylgruppe an das grundgerüst gebunden ist, wobei die Alkinylgruppe geradkettig oder verzweigt sein kann.

Unter mono-oder bizyklische Heteroarylgruppe R⁴ und R⁶, die an einer oder mehreren Stellen hydriert sein kann, sind alle mono- oder bizyklischen aromatischen Ringsysteme zu verstehen, die mindestens ein Heteroatom und höchstens sieben Heteroatome enthalten. Bevorzugt werden Ringsysteme mit 1-5 Heteroatomen. Als Heteroatome kommen 1-4 Stickstoffatome, 1-2 Sauerstoffatome und 1-2 Schwefelatome in Frage, die in allen Unterkombinationen im Ringsystem auftreten können, solange sie die für das jeweilige Heteroatom festgelegte Anzahl und in der Summe die Höchstzahl von sieben Heteroatomen nicht überschreiten. Beispielsweise gehören damit Verbindungen der Formel I worin R⁴ oder R⁶ Furanyl-, Thiophenyl-, Pyrazolyl, Pyrrolyl-, Oxazolyl-, Thiazolyl-, Imidazolyl-, Isoxazolyl-, Isothiazolyl-, Oxadiazolyl-, Triazolyl-, Tetrazolyl-, Thiadiazolyl-, Pyridyl-, Pyrimidinyl-, Pyridazinyl-, Pyrazinyl-, Triazinyl-, Azaindolizinyl-, Phthalidyl-, Thiophthalidyl-, Indolyl-, Isoindolyl-, Dihydroindolyl-, Dihydroisoindolyl-, Indazolyl-, Benzothiazolyl-, Indolonyl, Dihydroindolonyl-, Isoindolonyl-, Dihydroisoindolonyl-, Benzofuranyl-, Benzimidazolyl-, Indolizinyl-, Isobenzofuranyl-, Azaindolyl-, Azaisoindolyl-, Furanopyridyl-, Furanopyrimidinyl-, Furanopyrazinyl-, Furanopyidazinyl-, Dihydrobenzofuranyl-, Dihydrofuranopyridyl-, Dihydrofuranopyrimidinyl-, Dihydrofuranopyrazinyl-, Dihydrofuranopyridazinyl-, Dihydrobenzofuranyl-, Dihydroisochinolinyl-, Dihydrochinolinyl-, Benzoxazinonyl- , Phthalazinonyl-, Chinolinyl-, Isochinolinyl-, Chinolonyl-, Isochinolonyl-, Chinazolinyl-, Chinoxalinyl-, Cinnolinyl-, Phthalazinyl, 1,7- oder 1,8-Naphthyridinyl-, bedeutet, zur vorliegenden Erfindung und stellen eine besondere Ausführungsform der Erfindung dar.
Sind die Heteroarylgruppen teilweise oder vollständig hydriert so gehören Verbindungen der Formel I worin R⁴ Tetrahydropyranyl, 2H-Pyranyl, 4H-Pyranyl, Piperidyl, Tetrahydropyridyl, Dihydropyridyl, 1H-Pyridin-2-onyl, 1H-Pyridin-4-onyl, 4-Aminopyridyl, 1H-Pyridin-4-ylidenaminyl, Chromanyl, Thiochromanyl, Decahydrochinolinyl, Tetrahydrochinolinyl, Dihydrochinolinyl, 5,6,7,8-Tetrahydro-1 H-chinolin-4-onyl, Decahydroisochinolinyl, Tetrahydroisochinolinyl, Dihydroisochinolinyl, 3,4-Dihydro-2H-benz[1,4]oxazinyl, 1,2-Dihydro[1,3]benzoxazin-4-onyl, 3,4-Dihydrobenz[1,4]oxazin-4-onyl, 3,4-Dihydro-2H-benzo[1,4]thiazinyl, 4H-Benzo[1,4]thiazinyl, 1,2,3,4-Tetrahydrochinoxalinyl, 1H-Cinnolin-4-onyl, 3H-Chinazolin-4-onyl, 1H-Chinazolin-4-onyl, 3,4-Dihydro-1 H-chinoxalin-2-onyl, 2,3-1,2,3,4-Tetrahydro[1,5]naphthyridinyl, Dihydro-1H-[1,5]naphthyridyl, 1H-[1,5]naphthyrid-4-onyl, 5,6,7,8-Tetrahydro-1H-naphthyridin-4-onyl, 1,2-Dihydropyrido[3,2-d][1,3]oxazin-4-onyl, Octahydro-1 H-indolyl, 2,3-Dihydro-1H-indolyl, Octahydro-2H-isoindolyl, 1,3-Dihydro-2H-isoindolyl, 1,2-Dihydroindazolyl, 1 H-Pyrrolo[2,3-b]pyridyl, 2,3-Dihydro-1H-pyrrolo[2,3-b]pyridyl, 2,2-Dihydro-1H-pyrrolo[2,3-b]pyridin-3-onyl bedeutet, zur vorliegenden Erfindung.

Handelt es sich um eine Heteroarylalkylgruppe, so ist darunter eine gegebenenfalls auch teilweise hydrierte Heteroarylgruppe wie oben beschrieben zu verstehen, die über eine C₁-C₈-Alkylgruppe, die geradkettig oder verzweigt sein kann, an das Grundgerüst gebunden ist.

Unter einer Heteroarylalkenylgruppe ist eine gegebenenfalls auch teilweise hydrierte Heteroarylgruppe wie oben beschrieben zu verstehen, die über eine C₂-C₈-Alkenylgruppe, die geradkettig oder verzweigt sein kann, an das Grundgerüst gebunden ist.

Als Hydroxyschutzgruppe R¹¹ können die dem Fachmann bekannten Schutzgruppen wie z.B. Trimethylsilyl, tert.-Butyldimethylsilyl, Phenyldimethylsilyl, Benzyl, je nach Notwendigkeit vorhanden sein.
Die Hydroxygruppe in R⁵ kann geschützt durch eine der üblichen Hydroxyschutzgruppen als z. B. Benzylether, Silylether wie z.B. (CH₃)₃Si-O-, (Phenyl)(CH₃)₂Si-O-, (tert.butyl)(CH₃)₂Si-O- oder als C₁-C₅-Alkylether oder C₁-C₅-Alkylester oder Benzylester vorliegen.
Als Rest R⁵ ist die Hydroxygruppe bevorzugt.

Wenn R¹ und R⁸ einen fünf-bis achtgliedrigen Carbo- oder Heterozyklus (auch substituiert) bilden, so liegt dann ein trizyklisches System vor.
Als Heteroatome kommen Stickstoff, Sauerstoff, Schwefel oder Bor in Frage. Für den Fall, dass der gebildete Heterozyklus Bor enthält, ist dies ein besonderer Aspekt der vorliegenden Erfindung. Liegt ein sechsgliedriger Heterozyklus vor, so bilden die Reste R¹ und R⁸ zwei Glieder, während das bereits bestehende Tetralinsystem die übrigen 4 Glieder bildet. R¹ ist dafür an das Kohlenstoffatom, das dem Brücken-Kohlenstoffatom direkt benachbart ist, gebunden und R⁸ ist dann der Z-Substituent der exo-Doppelbindung.

Ein weiterer Gegenstand der Erfindung sind Verbindungen der allgemeinen Formel I worin R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe,
(C₃-C₇)Cycloalkyl(C₂-C₈)alkenylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe bedeutet.

Ein bevorzugter Gegenstand sind Verbindungen der allgemeinen Formel I, worin R⁴ eine gegebenenfalls mit C₁-C₅-Alkyl, Halogen, Hydroxy, C₁-C₅-Alkoxy substituierte Phenyl- oder Naphthyl-, Phthalidyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl, Dihydroindolonyl-, Dihydroisoindolonyl-, Benzimidazol- oder Indolylgruppe ist.
Ein weiterer bevorzugter Gegenstand der Erfindung sind Verbindungen der allgemeinen Formel I, worin R⁴ eine Chinolinyl-, Chinolonyl -, Chinazolinyl-, eine Chinazolonyl-, eine Phthalazinyl- oder eine Phthalazinonylgruppe bedeutet. Besonders bevorzugt sind Verbindungen der allgemeinen Formel I, worin R⁴ eine Chinolinyl-, Chinolonyl -, Chinazolinyl-, oder eine Phthalazinonylgruppe bedeutet.

Enthält ein oben genannter Heterozyklus eine Ketogruppe, so sind alle chemisch sinnvollen Regioisomeren wie beispielsweise 2H-Phthalazin-1-on und Phthalazin-2-on ebenfalls Gegenstand der Erfindung.

Ein besonderer Gegenstand der Erfindung sind Verbindungen der allgemeinen Formel I, worin R⁶ eine (C₁-C₅)-Alkylgruppe oder eine gegebenenfalls teilweise oder vollständig fluorierte (C₁-C₅)-Alkylgruppe, eine Arylgruppe, eine Aryl(C₁-C₈)alkylgruppe, Aryl(C₂-C₈)alkenylgruppe, eine (C₃-C₇)Cycloalkylgruppe, eine(C₃-C₇)Cycloalkyl(C₁-C₈)alkylgruppe, (C₃-C₇)Cycloalkyl(C₂-C₈)Alkenyl gruppe bedeutet.

Ein weiterer Gegenstand der Erfindung sind Verbindungen der allgemeinen Formel I, in denen R⁶ eine (C₁-C₃)-Alkylgruppe oder eine gegebenenfalls teilweise oder vollständig fluorierte (C₁-C₃)-Alkylgruppe darstellt. Besonders bevorzugt sind die vollständig fluorierten Alkylgruppen, insbesondere die CF₃-Gruppe.

Ein weiterer Gegenstand der Erfindung sind Verbindungen der Formel I, worin R⁴ eine C₁-C₁₀-Alkylgruppe, die gegebenenfalls substituiert sein kann durch 1-3 Hydroxygruppen, Halogenatome, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen. 1-3 Halogenatome, 1-2 (C₁-C₃)Exoalkylidengruppen substituierte 1-4 Stickstoffatome und/oder 1-2-Sauerstoffatome und/oder 1-2 Schwefelatome enthaltende mono- oder bizyklische Heteroarylgruppe, wobei diese Gruppen über eine beliebige Position mit dem Stickstoffatom verknüpft sein können und gegebenenfalls an einer oder mehreren Stellen hydriert sein können, bedeutet.
Besonders bevorzugt sind Verbindungen der Formel I worin R⁴ eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹²-Gruppen), (C₁-C₅)-Alkoxygruppen, Hydroxygruppen, Halogenatome, (C₁-C₃)Exoalkylidengruppen substituierte, gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R¹² eine (C₁-C₅)-Alkyl-oder eine Benzyl-Gruppe bedeutet, bedeutet.

Ein bevorzugter Gegenstand der Erfindung sind Verbindungen der allgemeinen Formel I, worin R⁴ eine gegebenenfalls mit C₁-C₅-Alkyl, Halogen, Hydroxy, C₁-C₅-Alkoxy, Keto oder (C₁-C₃)Exoalkyliden substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazol- oder Indolylgruppe bedeutet. Ganz besonders bevorzugt sind Verbindungen der allgemeinen Formel I, worin R⁴ eine Chinolinyl, Chinolonyl, Chinazolinyl- oder Phthalazinonylgruppe bedeuten.

Die erfindungsgemäßen Verbindungen der allgemeinen Formel I können durch das Vorhandensein von Asymmetriezentren als Stereoisomere vorliegen Gegenstand der vorliegenden Erfindung sind alle möglichen Diastereomere (z.B.: RR, RS, SR, SS), sowohl als Racemate, als auch in enantiomerenreiner Form.
Des weiteren können die erfindungsgemäßen Verbindungen als E-/Z-Isomere vorliegen. Sowohl die getrennten E- bzw. Z-Isomere als auch deren Gemische sind Gegenstand der vorliegenden Erfindung. E-/Z-Gemische können mit üblichen Methoden wie zum Beispiel Chromatographie getrennt werden.

Die erfindungsgemäßen Verbindungen können auch in Form von Salzen mit physiologisch verträglichen Anionen vorliegen, beispielsweise in der Form des Hydrochlorides, Sulfates, Nitrates, Phosphates, Pivalates, Maleates, Fumarates, Tartrates, Benzoates, Mesylates, Citrates oder Succinates.

Die erfindungsgemäßen Verbindungen werden hergestellt,
a) indem nach im Stand der Technik bekannten Methoden, beispielsweise durch Cyclopropanierung der Verbindungen der Formel IV, die offenkettigen Vorstufen der allgemeinen Formel (II) generiert werden, worin die Reste R¹, R², R³, R⁴, R⁵, und R⁶ die in Anspruch 1 angegebenen Bedeutungen haben, und verbindungen der Formel IV wie in Anspruch 16 definiert sind, 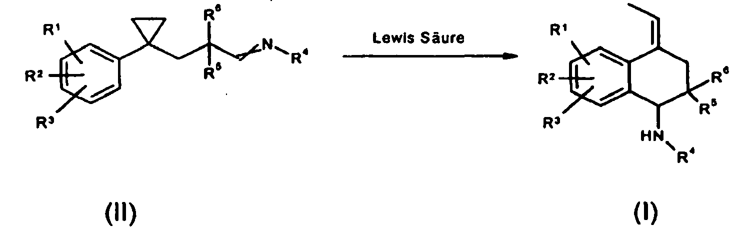 die dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (I) zyklisiert und umgelagert werden
b) indem nach im Stand der Technik bekannten Methoden hergestellten Styrole der allgemeinen Formel (III) durch eine gegebenenfalls enantioselektiv geführte En-Reaktion mit chiralen Lewis Säuren in die Verbindungen der allgemeinen Formel (IV) überführt werden, wobei R⁵ eine Hydroxygruppe bedeutet, die gegebenenfalls gemäß der anderen für R⁵ in Anspruch 1 definierten Bedeutungen in dem Fachmann bekannter Weise in eine Schutzgruppe überführt werden kann. Durch Reduktion und Aminierung wird nach dem Fachmann bekannten Methoden das Imin (V) erzeugt, 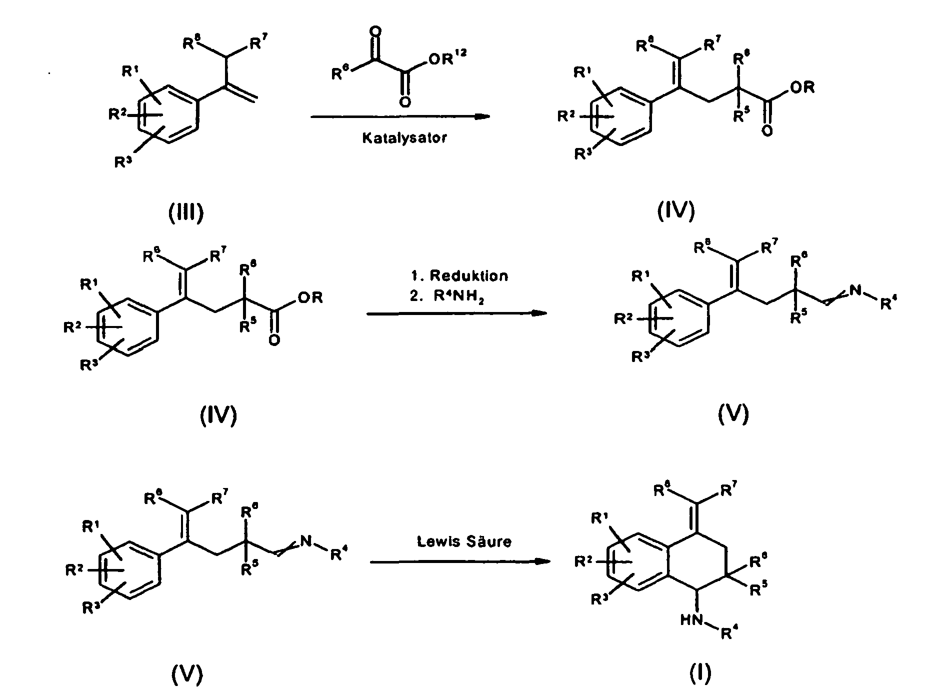 das dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (I) zyklisiert wird. Die allgemein in den oben aufgeführten Formeln definierten Reste R¹, R², R³, R⁴, R⁵, R⁶, R⁷ und R⁸ haben die in Anspruch 1 angegebenen Bedeutungen und mit R¹² in der Bedeutung von (C₁-C₅)-Alkyl, Benzyl.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR)) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe Affinität zum GR. So wurde für die Verbindung aus Beispiel 1 ein IC₅₀(GR) = 20 nM und IC₅₀(PR) > 1 µM und für die Verbindung aus Beispiel 2 ein IC₅₀(GR) = 30 nM und IC₅₀(PR) > 1µM gemessen.
Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Transkriptionsfaktoren, z.B. AP-1 und NFkappa-B, bewirkt (zur Übersicht siehe Cato ACB and Wade E, BioEssays **18**, 371-378 1996).

Die erfindungsgemäßen Verbindungen der allgemeinen Formel I hemmen die durch Lipopolysaccharid (LPS) ausgelöste Sekretion des Cytokins IL-8 in der menschlichen Monozytenzelline THP-1. Die Konzentration der Cytokine wurde im Überstand mittels kommerziell erhältlicher ELISA-Kits bestimmt. Die Verbindung aus Beispiel 1 zeigte eine Inhibition IC₅₀(IL8) = 6,4 nM bei einer 90%igen Effizienz bezüglich [³H]-Dexamethason als Standard.

Die anti - inflammatorische Wirkung der Verbindungen der allgemeinen Formel I wurde im Tierexperiment durch Testen in der Crotonöl - induzierten Entzündung in der Ratte und der Maus getestet (J. Exp. Med. (1995), **182**, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel I hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Verbindungen der allgemeinen Formel I induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Verbindungen der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS)
   - Bronchiectasis
   - Alle Formen der restriktiven Lungenerkrankungen, vor allem allergische Alveolitis,
   - Alle Formen des Lungenödems, vor allem toxisches Lungenödem, z.B. Bestrahlungs-Pneumonitis
   - Sarkoidosen und Granulomatosen, insbesondere Morbus Boeck
(ii) Rheumatische Erkrankungen / Autoimmunerkrankungen /Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Traumatische Arthritiden
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Rheumatische Weichteilerkrankungen
(iii) Allergien oder pseudoallerg. Erkrankungen, die mit entzündlichen, und /oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Giant cell arteriitis
(v) Dermatologische Erkrankungen, die mit entzündlichen, allergischen und /oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimune Pemphigus vulgaris, bullöses Pemphigoid
   - Erkrankungen des lichenoiden Formenkreises,
   - Pruritus (z. B. allergischer Genese)
   - Seborrhoisches Ekzem
   - Rosacea-Formenkreis
   - Pemphigus vulgaris
   - Erythema exsudativum multiforme
   - Balanitis
   - Vulvitis
   - Manifestation von Gefäßerkrankungen
   - Haarausfall wie Alopecia areata
   - Cutane Lymphome
   - Parapsoriasis
(vi) Nierenerkrankungen, die mit entzündlichen, allergischen und /oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und /oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und /oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und /oder proliferativen Prozessen einhergehen:
   - Himödem, vor allem Tumor-bedingtes Himödem
   - Multiple Sklerose
   - akute Encephalomyelitis
   - Meningitis
   - verschieden Formen von Krampfanfällen, z.B. BNS-Krämpfe
   - Akute Rückenmarksverletzung
   - Schlaganfall
(xiii) Bluterkrankungen, die mit entzündlichen, allergischen und / oder proliferativen Prozessen einhergehen wie z. B.:
   - Erworbene hämolytische Anämie
   - Idopathische Thrombocytopenia
(xiv) Tumorerkrankungen, die mit entzündlichen, allergischen und / oder proliferativen Prozessen einhergehen wie z. B.:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen wie z. B.:
   - Endokrine Orbitopathie
   - Thyreotoxische Krise
   - Thyreoiditis de Quervain
   - Hashimoto Thyreoiditis
   - Morbus Basedow
   - Endokrine Ophthalmopathy
   - Granulomatous thyroiditis
   - Struma lymphomatosa
   - Grave Krankheit
(xvi) Organ- und Gewebstransplantationen , Graft-versus-host-disease(xvii) Schwere Schockzustände, z.B anaphylaktischer Schock , systemic inflammatory response syndrome (SIRS)
(xviii)Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc.
(xix)Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xx) Schmerzen bei entzündlicher Genese, z.B. Lumbago
(xxi) Verschiedene Erkrankungen.

Darüber hinaus können die erfindungsgemäßen Verbindungen der allgemeinen Formel 1 zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Die Erfindung berifft ferner eine Kombinationstherapie, wobei eine oder mehrere Verbindungen der vorliegenden Erfindung, oder deren pharmazeutisch akzeptables Salz oder ein eine oder mehrere Verbindungen enthaltendes pharmazeutisches Mittel oder eine, eine oder mehrere Verbindungen enthaltende, Formulierung gleichzeitig oder nacheinander verabreicht wird oder als Kombinationspräparat mit einem oder mehreren anderen therapeutisch wirksamen Mitteln oder wirksamen Mitteln zur Behandlung von einer oder mehreren Zuständen, verabreicht wird.

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutischen Wirkungen bei den oben genannten Krankheitszuständen ist die geeignete Dosis unterschiedlich und hängt beispielsweise von der Wirkstärke der Verbindung der allgemeinen Formel 1, dem Wirt, der Art der Verabreichung und der Art und der Schwere der zu behandelnden Zustände, sowie der Verwendung als Prophylaktikum oder Therapeutikum ab.

Die Erfindung liefert weiterhin
(i) die Verwendung eines der erfindungsgemäßen Verbindung gemäß Formel I oder deren Gemisch zur Herstellung eines Medikaments zur Behandlung von einer ERKRANKUNG;
(ii) ein Verfahren zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eines der erfindungsgemäßen Verbindungen oder deren Gemisch und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als **Tropfen, Salben und Tinkturen in entsprechenden pharmazeutischen** Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01 %-20% betragen, um eine ausreichende pharmakologische Wirkung zu erzielen.

Die Erfindung umfaßt ebenfalls die erfindungsgemäßen Verbindungen der allgemeinen Formel I als therapeutischen Wirkstoff. Weiterhin gehört zur Erfindung die erfindungsgemäßen Verbindungen der allgemeinen Formel I als therapeutischen Wirkstoff zusammen mit pharmazeutisch verträglichen und annehmbaren Hilfsstoffen und Trägerstoffen.
Ebenfalls umfaßt die Erfindung eine pharmazeutische Zusammensetzung, die eine der pharmazeutisch aktiven, erfindungsgemäßen Verbindungen oder deren Gemisch oder deren pharmazeutisch verträgliches Salz und pharmazeutisch verträgliche Hilfsstoffe und Trägerstoffe enthält.

### Experimenteller Teil

### Experimenteller Teil

### Beispiel 1

### (cis,Z)-4-Ethyliden-6-fluor-1-[(2-methylchinolin-5-yl)amino]-2-(trifluormethyl)-1,2,3,4-tetrahydronaphthalin-2,5-diol

*3-[1-(3-Fluor-2-methoxyphenyl)-cyclopropyl]-2-oxopropionsäure-ethylester* Zu 26 g (180 mmol) 2,6-Difluoranisol und 14,6 ml (198 mmol) Cyclopropylcyanid in 500 mL Toluen werden bei 0°C über 40 min 396 ml einer 0,5 molaren (198 mmol) Lösung von Bis-(trimethylsilyl)-kaliumamid in Toluen getropft. Man rührt 18 Stunden bei Raumtemperatur und versetzt unter Eiskühlung mit Wasser und 1 M Schwefelsäure. Die organische Phase wird abgetrennt und die wässrige Phase mehrfach mit Ethylacetat extrahiert. Es wird mit Sole gewaschen, mit Natriumsulfat getrocknet und im Vakuum eingeengt. Nach chromatographischer Reinigung an Kieselgel (Hexan /Ethylacetat 10%-20%) erhält man 12,7 g 1-(3-Fluor-2-methoxyphenyl)-cyclopropylnitril. 12.7 g (66.1 mmol) des Nitrils werden in Toluol bei - 78°C langsam mit 82.7 ml (99.2 mmol) Diisobutylaluminiumhydrid Lösung (20% in Toluol) versetzt und nach 3 h bei - 78°C wurden 11.1 ml Isopropanol zugetropft. Man lässt auf -5°C erwärmen und gibt 150 ml einer 10%igen wässrigen Weinsäure Lösung zu. Nach Verdünnen mit Ether wird kräftig gerührt, die organische Phase wird abgetrennt und die wässrige Phase mehrfach mit Ethylacetat extrahiert. Es wird mit Sole gewaschen, mit Natriumsulfat getrocknet und im Vakuum eingeengt. Man erhält 11.8 g Aldehyd als gelbes Öl. Eine Lösung von 16.3 g (60.7 mmol) 2-Diethylphosphono-2-ethoxyessigsäure-ethylester in 60 ml Tetrahydrofuran wird unter Eiskühlung innerhalb von 20 Minuten mit 33.4 ml (66.8 mmol) einer 2 M Lösung von Lithiumdüsopropylamid in Tetrahydrofuran-Heptan-Toluol versetzt und 30 Minuten bei 0 °C gerührt. Innerhalb von 30 Minuten wird eine Lösung von 11.8 g (60.7 mmol) I in 61 ml Tetrahydrofuran bei 0°C zugetropft. Nach 20 Stunden bei RT wird Eiswasser zugegeben und mehrfach mit Ether und Ethylacetat extrahiert. Man wäscht mit gesättigter Ammoniumchlorid Lösung, trocknet über Natriumsulfat und engt ein. Das Rohprodukt wird mit 170 ml 2 M Natronlauge in 170 ml Ethanol über 15 Stunden bei Raumtemperatur verseift. Man erhält 13,9 g Säure, die mit 87 ml 2 N Schwefelsäure bei 90°C über 16 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchlorid Lösung und Entfernen des Lösungsmittels werden 10.2 g der rohen Ketosäure erhalten. 10.2 g (40.6 mmol) *3-[1-(3-Fluor 2-methoxyphenyl)-cyclopropyl]-2-oxopropionsäure* und 4.5 ml (85.3 mmol) Schwefelsäure (96%ig) werden in 200 ml Ethanol eine Stunde unter Rückfluss erhitzt. Der Ansatz wird im Vakuum eingeengt, der Rückstand in Eiswasser gegeben und mit gesättigter Natriumhydrogencarbonat Lösung basisch gestellt. Es wird mehrfach mit Essigester extrahiert, mit gesättigter Natriumchlorid Lösung gewaschen, getrocknet (Natriumsulfat) und im Vakuum eingeengt. Nach chromatographischer Reinigung an Kieselgel (Hexan / Ethylacetat 20%) erhält man 9.6 g 3-[1-(3-Fluor-2-methoxyphenyl)-cyclopropyl]-2-oxopropionsäure-ethylester.
¹H-NMR (CDCl₃): δ = 0.90 (m, 4H), 1.29 (t, 3H), 3.09 (s, 2H), 3.99 (d, 3H), 4.20 (q, 2H), 6.87 (ddd, 1H), 6.95 (ddd, 1H), 7.07 (d, 1 H).

### 3-[1-(3-Fluor-2-methoxyphenyl)-cyclopropyl]-2-hydroxy-2-(trifluormethyl)propanal

9.6 g (34.3 mmol) 3-[1-(3-Fluor-2-methoxyphenyl)-cyclopropyl]-2-oxopropionsäure-ethylester und 34.5 ml (233mmol) (Trifluormethyl)-trimethylsilan in 343 ml DMF werden mit 46.9 g Cäsiumcarbonat bei 0°C versetzt. Es wird 2 h bei 0°C gerührt und anschließend wird die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Nach chromatographischer Reinigung an Kieselgel (Hexan / Ethylacetat 10%-40%) erhält man 10.4 g 3-[1-(3-Fluor-2-methoxyphenyl)-cyclopropyl]-2-hydroxy-2-(trifluormethyl)-propansäure-ethylester als gelbes Öl. Dieses Öl wird in 297 ml Diethylether bei 0°C mit 2.25 g (59.4 mmol) Lithiumaluminiumhydrid versetzt und noch 1 Stunden bei RT gerührt. Zum Ansatz werden bei 0°C vorsichtig 20 ml gesättigte Ammoniumchlorid Lösung gegeben und es wird 15 Minuten kräftig nachgerührt. Man extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan / Ethylacetat 10%-50%) erhält man 5.6 g 3-[1-(3-Fluor-2-methoxyphenyl)-cyclopropyl]-2-(trifluormethyl)-propan-1,2-diol. Zu 5.6 g (18.1 mmol) Diol in 100 ml Dichlormethan und 61 ml DMSO gibt man 12.4 ml (89 mmol) Triethylamin und portionsweise über 10 min 11 g (70 mmol) Pyridin SO₃ Komplex. Man rührt über 3 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-50%) erhält man 5.9 g Produkt.
¹H-NMR (CDCl₃): δ = 0.68-0.76 (m, 2H), 0.90-1.02 (m, 2H), 2.03 (d, 1H), 2.91 (d, 1H), 3.85 (s, 1H), 4.03 (s, 3H), 6.80 (d, 1H), 6.87 (ddd, 1H), 6.98 (dd, 1H), 9.26 (s, 1H).

200 mg (0.65 mmol) 3-[1-(3-Fluor-2-methoxyphenyl)-cyclopropyl]-2-hydroxy-2-trifluormethylpropanal, 120 mg (0.76 mmol) 5-Amino-2-methylchinolin und 0.3 ml Titantetraethylat werden in 25 mL Toluol 2 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 270 mg 3-[1-(3-Fluor-2-methoxyphenyl)-cyclopropyl]-1-[(2-methyl-chinolin-5-yl)imino]-2-(trifluormethyl)propan-2-ol als Rohprodukt. Zu 270 mg (0.6 mmol) Imin in 27 mL CH₂Cl₂ werden bei -30 °C 5.5 mL (5.5 mmol) einer 1 M Bortribromid-Lösung getropft. Man lässt auf Raumtemperatur erwärmen und rührt 20 Stunden. Der Ansatz wird mit ges. NaHCO₃ versetzt, die Phasen werden getrennt, die wäßrige Phase mit CH₂Cl₂ extrahiert, die vereinigten organischen Phasen getrocknet (Na₂SO₄) und im Vakuum eingeengt. Säulenchromatographie an Kieselgel (Hexan/Essigester 0-70%) und anschliessende HPLC (Kromasil C18, Wasser / Acetonitril 30 - 60%) liefern 24 mg Produkt.
¹H-NMR (300 MHz, CD₃OD): δ = 1.74 (d, 3H), 2.52 (d, 1H), 2.69 (s, 3H), 3.12 (d, 1H), 4.55 (s, 1H), 5.81 (q, 1H), 6.34 (d, 1H), 6.73 (dd, 1H), 6.94 (dd, 1H), 7.26 (d, 1H), 7.35 (d, 1H), 7.42 (t, 1H).

### Beispiel 2

### (cis, Z)-5-{[4-Ethyliden-6-fluor-2,5-dihydroxy-2-(trifluormethyl)-1,2,3,4-tetrahydronaphthalin-1-yl]amino}-chinolin-2(1H)-on

### 5-Aminochinolin-2(1H)-on:

4.5 g 5-Nitrochinolin-2(1H)-on (Chem. Pharm. Bull. (1981), 29, pp. 651-56) werden in 200 ml Essigester und 500 ml Methanol in Anwesenheit von 450 mg Palladium auf Aktivkohle als Katalysator unter Normaldruck mit Wasserstoff bis zur vollständigen Umsetzung hydriert. Der Katalysator wird durch Filtration durch Kieselgur entfernt und die Reaktionslösung im Vakuum eingeengt. Man erhält 3.8 g der Titelverbindung als gelben Feststoff.
¹H-NMR (DMSO): δ = 5.85 (bs, 2H), 6.27 (d, 1 H), 6.33 (d, 1H), 6.43 (d, 1H), 7.10 (t, 1 H), 8.07 (d, 1 H), 11.39 (br, 1 H)

Zu 300 mg (0.98 mmol) 3-[1-(3-Fluor-2-methoxyphenyl)-cyclopropyl]-2-hydroxy-2-(trifluormethyl)propanal und 188 mg (1.18 mmol) 5-Aminochinolin-2(1H)-on in 3 ml Toluol werden 0.41 ml (2.0 mmol) Titantetraethytat gegeben und die Mischung wird über eine Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Das so roh erhaltene 5-({3-[1-(3-Fluor-2-methoxyphenyl)-cyclopropyl]-2-(trifluormethyl)propan-1-yl}-imino)-chinolin-2(1H)-on wird säulenchromatographisch an Kieselgel (Hexan / Ethylacetat 50%) gereinigt. Die so erhaltenenen 235 mg (0.52 mmol) Imin werden in 10 ml Dichlormethan aufgenommen und auf -60°C gekühlt. Man tropft über 10 Minuten 8.9 ml (8.9 mmol) einer 1 M Bortribromid Lösung in Dichlor-methan zu und lässt über Nacht erwärmen. Man gießt die Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und Chromatographie an Kieselgel (Hexan / Ethylacetat 0 - 50%) und anschliessender HPLC (Kromasil C18, Wasser / Acetonitril 30 - 60%) erhält man 10.3 mg des gewünschten Produktes.
¹H-NMR (300 MHz, CD₃OD): δ = 1.76 (d, 3H), 2.53 (d, 1H), 3.13 (d, 1H), 4.53 (s, 1H), 5.82 (q, 1H), 6.19 (d, 1H), 6.57 (d, 1H), 6.69 (d, 1H), 6.77 (dd, 1H), 6.98 (dd, 1H), 7.29 (t, 1H), 8.24 (d, 1H)

### Beispiel 3

### 5-{[2-Hydroxy-4-propyliden-2-(trifluormethyl)-1,2,3,4-tetrahydronaphthalin-1-yl]amino}-chinolin-2(1H)-on

### 2-Hydroxy-4-phenyl-2-(trifluormethyl)-hept-4-enal

Zu 400 mg (1.4 mmol) 1,1'-Bi-2-naphthol werden 1.4 ml (0.7 mmol) einer 0.5 M Titantetraisopropylat Lösung in Toluol gegeben und man rührt die rote Lösung für 2 Stunden bei Raumtemperatur. 1.1 g (7.6 mmol) 2-Phenyl-1-penten und 2.5 g (15.2 mmol) Ethyltrifluorpyruvat werden zugegeben und man erhitzt die Mischung über 8 Stunden auf 100°C. Nach dem Abkühlen wird sofort säulenchromatographisch an Kieselgel (Hexan / Ethylacetat 15 %) gereinigt und man erhält 1.85 g 2-Hydroxy-4-phenyl-2-(trifluormetyl)-hept-4-ensäure-ethylester als E/Z Gemisch. 0.75 g (2.3 mmol) (*E*)-2-Hydroxy-4-phenyl-2-(trifluormetyl)-hept-4-ensäure-ethylester werden in 20 ml Diethylether auf -5°C gekühlt und über 10 Minuten werden portionsweise 175 mg (4.6 mmol) Lithiumaluminiumhydrid fest zugegeben. Man rührt 1 Stunden bei 0°C und gießt in gesättigte Ammoniumchlorid Lösung. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Ethylacetat 0-15%) liefert 0.42 g (*E*)-2-Hydroxy-4-phenyl-2-(trifluormetyl)-hept-4-enal und 0.12 g Alkohol.
¹H-NMR (300 MHz, CDCl₃): δ = 1.06 (t, 3H), 2.26 (dq, 2H), 3.26 (d, 1H), 3.33 (d, 1H), 3.69 (s, 1H), 5.79 (t, 1H), 7.20-7.33 (m, 5H), 9.22 (s, 1H)

Zu 200 mg (0.73 mmol) 2-Hydroxy-4-phenyl-2-(trifluormethyl)-hept-4-enal und 120 mg (0.73 mmol) 5-Amino-chinolin-2(1H)-on in 5 ml Toluol werden 0.3 ml (1.5 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 310 mg 5-{[2-Hydroxy-4-phenyl-2-(trifluormethyl)-hept-4-en-1-yl]imino}-chinolin-2(1H)-on als Rohprodukt. Davon werden 155 mg (0.36 mmol) in 5 ml Dichlormethan aufgenommen und auf -78°C gekühlt. Man tropft über 5 Minuten 1.8 ml (1.8 mmol) einer 1 M Titantetrachlorid Lösung in Dichlormethan zu und entfernt nach 10 Minuten das Kühlbad. Nach weiteren 30 Minuten gießt man die auf Raumtemperatur erwärmte Lösung auf eine Mischung von Eis und gesättigter Natriumhydrogencarbonat Lösung und rührt 10 Minuten lang heftig. Es wird mit Ethylacetat extrahiert, mit gesättigter Natriumchloridlösung gewaschen und über Natriumsulfat getrocknet. Nach Einengen und Chromatographie an Kieselgel (Hexan / Ethylacetat 0 - 50%) erhält man 12 mg des gewünschten Produktes.
¹H-NMR (300 MHz, CD₃OD): δ= 1.17 (t, 3H), 2.32 (dq, 2H), 2.85 (d, 1H), 3.07 (d, 1H), 5.18 (s, 1H), 6.32 (t, 1H), 6.52 (d, 1H), 6.60 (d, 1H), 6.71 (d, 1H), 7.18 (d, 1H), 7.26 (t, 2H), 7.37 (t, 1H), 7.69 (d, 1H), 8.25 (d, 1H)

### Beispiel 4

### (cis,Z)-5-{[4-Ethyliden-6-fluor-2,5-dihydroxy-2-(trifluormethyl)-1,2,3,4-tetrahydronaphthalin-1-yl]amino}-2-methylphthalazin-1-on

### 5-Amino-2-methyl-phthalazin-1-on:

### 3-Brom-4-nitro-phthalid

5.37 g 4-Nitrophthalid (Tetrahedron Lett. (2001), 42, pp. 1647-50), 8.04 g *N-*Bromsuccinimid und 196 mg Benzoylperoxyd werden in 80 ml Benzotrifluorid unter Rückfluß und Lichteinwirkung bis zur vollständigen Umsetzung erhitzt. Es wird auf Wasser gegeben, mit Dichlormethan extrahiert, mehrfach mit Wasser gewaschen, getrocknet und das Lösungsmittel im Vakuum entfernt. Man erhält 7.24 g 3-Brom-4-nitro-phthalid als Feststoff.
¹H-NMR (300 MHz, CDCl₃), δ = 7.26 (s, 1 H), 7.88 (t, 1H), 8.3 (d, 1 H), 8.56 (d, 1 H)

### 5-Nitro-phthalazin-1-on:

18.25 g Hydrazinsulfat und 14.88 g Natriumcarbonat werden in 300 ml DMF bei 100°C für 1 h gerührt. Dann werden 7.24 g 3-Brom-4-nitro-phthalid in 100 ml DMF zugegeben und es wird weitere 4 h bei 100°C gerührt. Es wird auf Wasser gegeben, mit Essigester mehrfach extrahiert und die org. Phase mit Wasser und Sole gewaschen. Es wird getrocknet und das Lösungsmittel im Vakuum entfernt. Nach Umkristallisation aus Essigester erhält man 2.35 g 5-Nitro-phthalazin-1-on als Feststoff.
¹H-NMR (300 MHz, DMSO-d₆), δ = 8.05 (t, 1H), 8.57-8.66 (m, 2H), 8.73 (s, 1H), 13.13 (bs, 1H)

### 2-Methyl-5-nitro-phthafazin-1-on

1.6 g 5-Nitro-phthalazin-1-on und 2.31 g Kaliumcarbonat werden 10 min. bei Raumtemperatur in 60 ml DMF gerührt. Es werden 1.1 ml Methyliodid zugegeben und man rührt über Nacht. Es wird auf Wasser gegeben, mit Essigester mehrfach extrahiert und die org. Phase mit Wasser und Sole gewaschen. Es wird getrocknet und das Lösungsmittel im Vakuum entfernt. Man erhält 1.57 g 2-Methyl-5-nitro-phthalazin-1-on als gelben Feststoff.
¹H-NMR (300 MHz, DMSO-d₆), δ = 3.73 (s, 3H), 8.05 (t, 1H), 8.62 (d, 2H), 8.75 (s, 1H)

### 5-Amino-2-methyl-phthalazin-1-on

1.57 g 2-Methyl-5-nitro-phthalazin-1-on und 130 mg Palladium auf Aktivkohle werden in 45 ml Essigester suspendiert und mit Wasserstoff unter Normaldruck hydriert. Es wird durch Kieselgur filtriert und das Lösungsmittel im Vakuum entfernt. Man erhält 1.26 g 5-Amino-2-methyl-phthalazin-1-on als gelben Feststoff.
¹H-NMR (300 MHz, CDCl₃), = 3.81 (s, 3H), 7.0 (d, 1H), 7.5 (t, 1H), 7.8 (d, 1H). 8.16 (s, 1H)

Zu 430 mg (1.4 mmol) 3-[1-(3-Fluor-2-methoxyphenyl)-cyclopropyl]-2-hydroxy-2-(trifluormethyl)propanal und 253 mg (1.44 mmol) 5-Amino-2-methyl-phthalazin-1-on in 50 ml Toluol werden 0.6 ml (2.4 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Die so roh erhaltenen 650 mg 5-({3-[1-(3-Fluor-2-methoxyphenyl)-cyclopropyl]-2-(trifluormethyl)propan-1-yl}-imino)-2-methyl-phthalazin-1-on werden in 55 ml Dichlormethan aufgenommen und auf -30°C gekühlt. Man tropft über 10 Minuten 12 ml (12 mmol) einer 1 M Bortribromid Lösung in Dichlormethan zu und refluxiert die Lösung im Anschluß über 8 Stunden. Man gießt die erkaltete Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und Chromatographie an Kieselgel (Hexan / Ethylacetat 0 - 50%) und anschließender HPLC (Kromasil C18, Wasser / Acetonitril 30-60%) erhält man 75 mg des gewünschten Produktes.
¹H-NMR (300 MHz, CD₃OD); δ = 1.76 (d, 3H), 2.55 (d, 1 H), 3.13 (d, 1H), 3.84 (s, 3H), 4.59 (s, 1H), 5.85 (q, 1 H), 6.74- 6.84 (m, 3H), 7.56 (t, 1H), 7.63 (d, 1H), 8.56 (s, 1 H).

### Beispiel 5

### (cis,Z)- 4-Ethyliden-6-fluor-1-[(2-methylchinazolin-5-yl)amino]-2-(trifluormethyl)-1,2,3,4-tetrahydronaphthalin-2,5-diol

### 5-Amino-2-methylchinazolin

12,7 g (62 mmol) 2-Methyl-5-nitro-3H-chinazolin-4-on (M.T. Bogert, V.J. Chambers J. Org Chem. 1905, 649-658) und 37,5 g Phosphorpentachlorid werden in 75 ml Phosphorylchlorid über 20 Stunden unter Rückfluss erhitzt. Nach dem Abkühlen gießt man in ges. NaHCO₃ Lösung und extrahiert mit Ethylacetat. Die organische Phase wird getrocknet und das Lösungsmittel entfernt. Man erhält 14 g 4-Chlor-2-methyl-5-nitrochinazolin, von denen 4.5 g (20.2 mmol) in 225 ml Ethylacetat und 22.5 ml Triethylamin gelöst werden. Man gibt 2 g Palladium auf Kohle zu und rührt bei Eiskühlung 4 Stunden unter einer Wasserstoffatmosphäre bei Normaldruck. Die Lösung wird mittels Filtration über Celite vom Katalysator befreit, wobei mit 200 ml Ethanol nachgewaschen wird, und eingedampft. Nach Chromatographie an Kieselgel mit Essigester-Ethanol (0-10%) werden 530 mg des Produkts erhalten.
¹H-NMR (300 MHz, CDCl₃); δ = 2.87 (s, 3H), 4.52 (br., 2H), 6.77 (d, 1H), 7.33 (d, 1H), 7.65 (t, 1H), 9.40 (s, 1H).

Zu 500 mg (1.63 mmol) 3-[1-(3-Fluor-2-methoxyphenyl)-cyclopropyl]-2-hydroxy-2-(trifluormethyl)propanal und 300 mg (1.88 mmol) 5-Amino-2-methyl-chinazolin in 75 ml Toluol werden 0.8 ml (3.2 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Nach Chromatographie an Kieselgel (Hexan / Ethylacetat 0 - 60%) erhält man 350 mg 3-[1-(3-Fluor-2-methoxyphenyl)-cyclopropyl]-1-[(2-methyl-chinazol-5-yl)imino]-2-(trifluormethyl)propan-2-ol, die in 36 ml Dichlormethan aufgenommen und auf -30°C gekühlt werden. Man tropft über 10 Minuten 7.9 ml (7.9 mmol) einer 1 M Bortribromid Lösung in Dichlormethan zu und refluxiert die Lösung im Anschluß über 5 Stunden. Man gießt die erkaltete Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und HPLC (Kromasil C18, Wasser /Acetonitril 30 - 60%) erhält man 86 mg des gewünschten Produktes.
¹H-NMR (300 MHz, CDCl₃); δ = 1.83 (d, 3H), 2.66 (d, 1H), 2,81 (s, 3H), 3.22 (d, 1H), 5.03 (d, 1H), 4.67 (d, 1 H), 5.85 (d, 1H), 5.95 (q, 1H), 6.55 (d, 1H), 6.85 (dd, 1H), 6.95 (d, 1H), 7.28 (d, 1H), 7.66 (t, 1H), 9.35 (s, 1 H).

**Beispiel 5A** / **5B** (*rac,cis,Z*)*-* 4-Ethyliden-6-fluor-1-[(2-methylchinazolin-5-yl)amino]-2-(trifluormethyl)-1,2,3,4-tetrahydronaphthalin-2,5-diol wird mittels präparativer chiraler HPLC (Chiracel OD 20µ) in die enanantiomerenreinen Verbindungen gespalten:
(+)-Enantiomer: analytische HPLC: Rₜ = 8.0 min (Chiralcel OD 10µ, 250x4.6 mm, Hexan / Ethanol 10%, 1 ml/min Fluß)
(-)-Enantiomer: analytische HPLC: Rₜ = 11.1 min (Chiralcel OD 10µ, 250x4.6 mm, Hexan / Ethanol 10%, 1 ml/min Fluß)

### Beispiel 6

### (cis,Z)-6-Chlor-4-ethyliden-1-[(2-methylchinazolin-5-yl)amino]-2-(trifluormethyl)-1,2,3,4-tetrahydronaphthalin-2,5-diol

### 3,3,3-Trifluor-2-[1-(3-chlor-2-methoxyphenyl)-cyclopropylmethyl]-2-hydroxypropionaldehyd

### 1-(3-Chlor-2-methoxy-phenyl)-cyclopropancarbonitril:

5,50 g (30,36 mmol) (3-Chlor-2-methoxy-phenyl)acetonitril werden in 50 ml DMF gelöst. Bei 0°C werden unter Schutzgas innerhalb von 30 Minuten 2,96 g (73,93 mmol) einer Natriumhydridsuspension (55-60%) zugegeben. Nach 30minütigem Nachrühren bei 0°C werden 7,16 g (38,09 mmol) 1,5-Dibromethan zugetropft. Nach dem Rühren über Nacht bei Raumtemperatur wird das Reaktionsgemisch auf Wasser gegeben und dreimal mit Methyl-tert.butylether extrahiert. Die vereinigten organischen Extrakte werden wie üblich mit Sole gewaschen und getrocknet. Der nach dem Abfiltrieren des Trocknungsmittels und Abrotieren des Lösungsmittels verbleibende Rückstand wird an Kieselgel (Laufmittel Ethylacetat/Hexan) chromatographiert. Isoliert werden 4,36 g (69,2%) der gewünschten Verbindung. ¹H-NMR (300 MHz, CDCl₃): δ = 1,34 (2H), 1,70 (3H), 4,13 (3H), 7,02 (1H), 7,18 (1H), 7,39 (1 H).

### 1-(3-Chlor-2-methoxy-phenyl)-cyclopropancarbaldehyd:

4,36 g (21 mmol) 1-(3-Chlor-2-methoxy-phenyl)-cyclopropancarbonitril werden in 75 ml Toluol gelöst und die Reaktionsmischung auf -70°C gekühlt. Bei dieser Temperatur werden 26,14 ml (31,50 mmol) einer 1,2 molaren DIBAH Lösung in Toluol zugetropft und der Ansatz zwei Stunden bei dieser Temperatur nachgerührt. Anschließend werden vorsichtig 239,2 ml einer 10%igen Weinsäure zugetropft, wobei die Temperatur rasch auf -5°C bis +5°C ansteigt. Nach kurzem Nachrühren wird Methyl-tert.-butylether zugegeben und der Ansatz kräftig gerührt. Die organische Phase wird abgetrennt und die wässrige Phase zweimal mit Methyl-tert.butylether nachextrahiert. Die vereinigten organischen Extrakte werden mit Sole gewaschen und über Natriumsulfat getrocknet. Nach dem Abfiltrieren des Trocknungsmittels und Abrotieren des Lösungsmittels werden 5,42 g (>100%) des Aldehyds erhalten, der roh weiter eingesetzt wird.
¹H-NMR (300 MHz, CDCl₃): δ = 1,39 (2H), 1,68 (3H), 3,86 (3H), 7,00-7,12 (2H), 7,37 (1H), 9,20 (1H).
(E/Z)-3-[1-(3-Chlor-2-methoxy-phenyl)-cyclopropyl]-2-ethoxy-acrylsäureethylester:

Bei 0°C werden zu 2,5 g (24,47 mmol) Diisopropylamin in 5,9 ml Tetrahydrofuran langsam 15,3 ml (24,25 mmol) Butyllithium (1,6 molar in Hexan) zugetropft und dreißig Minuten nachgerührt. Das so erzeugte LDA wird zu 5,63 g (20,98 mmol) (Diethoxy-phosphoryl)-ethoxy-essigsäureethylester, vorgelegt in 17,3 ml Tetrahydrofuran, zugetropft, und zwar bei 0°C. Nach 20minütigem Rühren werden 4,42 g (20,98 mmol) des im vorigen Abschnitt beschriebenen Aldehyds, gelöst in 17,3 ml THF, bei 0°C zum deprotonierten Phosphonat getropft. Nach dem Rühren über Nacht bei Raumtemperatur wird die Reaktionsmischung mit Wasser versetzt und dreimal mit Methyl-tert.butylether extrahiert. Die vereinigten organischen Extrakte werden wie üblich weiter aufgearbeitet und der verbleibende Rückstand an Kieselgel (Laufmittel Ethylacetat/Hexan) chromatographiert. Isoliert werden 4,83 g (70,9%) der gewünschten Verbindung als E/Z Gemisch. Aus diesem Grund sind im nachfolgenden ¹H-NMR nur die Lage der Signale und nicht die Integration angegeben. ¹H-NMR (300 MHz, CDCl₃): δ = 0,85-0,99, 1,09, 1,16-1,40, 3,49, 3,73, 3,94, 4,00, 4,10-4,29, 5,70, 6,08, 6.90-7,00, 7,19-7,30.

### (E/Z)-3-[1-(3-Chlor-2-methoxy-phenyl)-cyclopropyl]-2-ethoxy-acrylsäure:

4,83 g (14,87 mmol) (E/Z)-3-[1-(3-Chlor-2-methoxy-phenyl)-cyctopropyl]-2-ethoxy-acrylsäureethylester werden mit 140 ml einer 1 M NaOH in Ethanol/Wasser 2:1 versetzt und über Nacht bei Raumtemperatur gerührt. Das Ethanol wird am Rotationsverdampfer abgezogen, die Mischung mit Wasser verdünnt und dreimal mit Methyl-tert.butylether extrahiert. Die organischen Phasen werden nach DC Kontrolle verworfen. Die wässrige Phase wird mit einer 1 M HCl angesäuert und dreimal mit Methyl-tert.butylether extrahiert. Die vereinigten organischen Extrakte werden mit Sole gewaschen und über Natriumsulfat getrocknet. Der nach dem üblichen Procedere erhaltene Rückstand (4,33 g = 98,2%) wird roh in die nächste Stufe eingesetzt. Aus diesem Grund sind im nachfolgenden ¹H-NMR nur die Lage der Signale und nicht die Integration angegeben. ¹H-NMR (300 MHz, CDCl₃): δ = 0,88, 1,00, 1,13-1,49, 3,50, 3,80, 3,95, 4,00, 5,93, 6,22, 6,92-7,00, 7,19-7,30, 7,40.

### 3-[1-(3-Chlor-2-methoxy-phenyl)-cyclopropyl]-2-oxo-propionsäure:

4,33 g (14,59 mmol) (E/Z)3-[1-(3-Chlor-2-methoxy-phenyl)-cyclopropyl]-2-ethoxy-acrylsäure werden in 65 ml Schwefelsäure (1M) gegeben und über Nacht bei 90°C gerührt. Nach dem Abkühlen wird mit etwas Wasser verdünnt und mit Kaliumcarbonat basisch gestellt. Es wird dreimal mit Methyl-tert.butylether extrahiert und die vereinigten organischen Extrakte nach DC Kontrolle verworfen. Die wässrige Phase wird mit 1 M Salzsäure angesäuert und mit Methyl-tert.butylether extrahiert. Nach dem weiteren üblichen Aufarbeiten werden 3,40 g (86,7%) der gewünschten α-Ketocarbonsäure erhalten. ¹H-NMR (300 MHz, CDCl₃): δ = 0,97 (2H), 1,05 (2H), 3,19 (2H), 4,05 (3H), 6,99 (1H), 7,20-7,30 (2H).

### 3-[1-(3-Chlor-2-methoxy-phenyl)-cyclopropyl]-2-oxo-propionsäureethylester:

15,12 g (56,27 mmol) 3-[1-(3-Chlor-2-methoxy-phenyl)-cyclopropyl]-2-oxo-propionsäure werden in 350 ml Ethanol gelöst und mit 6,3 ml Schwefelsäure (conc.) versetzt. Nach fünfstündigem Kochen am Rückfluß wird die Reaktionsmischung zur Trockene einrotiert. Der Rückstand wird in 700 ml gesättigte Natriumhydrogencarbonatlösung aufgenommen und dreimal mit Ethylacetat extrahiert. Die vereinigten organischen Extrakte werden mit Sole gewaschen, über Natriumsulfat getrocknet und das Lösungsmittel nach dem Abfiltrieren des Trockenmittels abrotiert. Isoliert werden nach Chromatographie des Rückstandes an Kieselgel (Laufmittel Ethylacetat/Hexan) 12,36 g (74%) des gewünschten Esters. ¹H-NMR (300 MHz, CDCl₃): δ = 0,90-0,98 (4H), 1,30 (3H), 3,19 (2H), 3,97 (3H), 4,20 (2H), 6,95 (1H), 7,20-7,30 (2H).

### 2-[1-(3-Chlor-2-methoxy-phenyl)-cyclopropylmethyl]-3,3,3-trifluor-2-trimethylsilyloxy-propionsäureethylester:

6,18 g (20,83 mmol) 3-[1-(3-Chlor-2-methoxy-phenyl)-cyclopropyl]-2-oxo-propionsäureethylester und 3,55 g (24,99 mmol) (Trifluormethyl)-trimethylsilan werden in 33 ml Tetrahydrofuran gelöst. Nach Zugabe von 51 mg Tetrabutylammoniumfluoridtrihydrat wird über Nacht bei Raumtemperatur gerührt. Die Reaktionsmischung wird mit Methyl-tert.butylether verdünnt, zunächst mit Wasser und anschließend mit Sole gewaschen. Nach dem üblichen Procedere (Trocknen und Abfiltrieren des Lösungsmittels) wird der Rückstand an Kieselgel (Laufmittel Ethylacetat/Hexan) chromatographiert. Isoliert werden 5,65 g (66,4%) der gewünschten, allerdings verunreinigten Verbindung.

### 2-[1-(3-Chlor-2-methoxy-phenyl)-cyclopropylmethyl]-3,3,3-trifluor-2-hydroxy-propionsäureethylester:

5,65 g (1,75 mmol) der im vorigen Abschnitt beschriebenen Verbindung wird in 76 ml Tetrahydrofuran gelöst und mit 4,34 g (13,75 mmol) Tetrabutylammoniumfluoridtrihydrat versetzt. Nach einstündigem Rühren bei Raumtemperatur wird wie üblich aufgearbeitet und der nach dem Einrotieren des Lösungsmittels verbleibende Rückstand an Kieselgel (Hexan / Ethylacetat 0-30%) chromatographiert. Isoliert werden 3,24 g (64,3%) der gewünschten Verbindung. ¹H-NMR (300 MHz, CDCl₃): δ = 0,59-0,69 (1H), 0,73-0,82 (1H), 0,98-1,20 (5H), 1,75 (1H), 3,09 (1H), 3,39-3,51 (1H), 3,80 (1H), 3,85-4,03 (4H), 6,92 (1H), 7,08 (1H), 7,25 (1H).

### 3,3,3-Trifluor-2-[1-(3-chlor-2-methoxyphenyl)-cyclopropylmethyl]-2-hydroxypropionaldehyd:

0,850 g (2,317 mmol) des im vorigen Abschnitt beschriebenen Esters werden in 8 ml Diethylether gelöst und bei 0°C unter Schutzgas portionsweise mit 66 mg (1,74 mmol) Lithiumaluminiumhydrid versetzt. Nach zweistündigem Nachrühren bei 0 bis 5°C werden bei 0°C vorsichtig 2,7 ml gesättigte Natriumhydrogencarbonatlösung zugetropft. Nach dreimaligem Extrahieren mit Methyl-tert.butylether werden die vereinigten organischen Extrakte wie üblich gewaschen. Der letztlich erhaltene Rückstand wird am Flashmaster chromatographiert. Isoliert werden 750 mg (65,5%) eines Gemisches, das zu 64% aus dem gewünschten Aldehyd und zu 36% aus dem Ausgangsester besteht.

Zu 300 mg (0.93 mmol) 3,3,3-Trifluor-2-[1-(3-chlor-2-methoxyphenyl)-cyclopropylmethyl]-2-hydroxy-propionaldehyd und 155 mg (0.98 mmol) 5-Amino-2-methylchinazolin in 28 ml Toluol werden 0.39 ml (1.9 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Das so roh erhaltene 3-[1-(3-Chlor-2-methoxyphenyl)-cyclopropyl]-1-[(2-methyl-chinazol-5-yl)imino]-2-(trifluormethyl)propan-2-ol wird in 23 ml Dichlormethan aufgenommen und auf -20°C gekühlt. Man tropft über 10 Minuten 7.8 ml (7.8 mmol) einer 1 M Bortribromid Lösung in Dichlor-methan zu und refluxiert die Lösung im Anschluß über 3 Stunden. Man gießt die erkaltete Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen, Chromatographie an Kieselgel (1. Trennung: Hexan / Ethylacetat 15 - 20%, 2. Trennung: Hexan / 2-Propanol 10%) erhält man 4 mg des gewünschten Produktes.
¹H-NMR (300 MHz, CD₃OD); δ = 1.75 (d, 3H), 2.58 (d, 1H), 2,83 (s, 3H), 3.15 (d, 1H), 4.65 (s, 1H), 5.87 (q, 1H), 6.50 (d, 1 H), 6.84 (d, 1H), 7.19 (d, 1H), 7.25 (d, 1H), 7.71 (t, 1H), 9.65 (s, 1H).

### Beispiel 7

### (cis,Z)-6-Chlor-4-ethyliden-1-[(7-fluor-2-methylchinazolin-5-yl)amino]-2-(trifluormethyl)-1,2,3,4-tetrahydronaphthalin-2,5-diol

### 5-Amino-7-fluor-2-methychinazolin

17 g (70,5 mmol) 3,6-Difluor-2-N-pivaloylaminobenzaldehyd (L. Florvall, I Fagervall, L.-G- Larsson, S.B. Ross, Eur.J. Med. Chem. 34 (1999) 137-151), 9,2 g Acetamidin Hydrochlorid, 13,4g Kaliumcarbonat und 10,4 g Molekularsieb (4A) werden in 70 ml Butyronitril zusammengegeben. Man erhitzt unter heftigem Rühren 17 Stunden auf 145°C und entfernt das Lösungsmittel im Vakuum. Nach Chromatographie des Rückstands an Kieselgel mit Hexan/Ethylacetat (0-70%) erhält man 4,5 g 7-Fluor-5-N-pivaloylamino-2-methychinazolin.
1g (3,82 mmol) 7-Fluor-5-N-pivaloylamino-2-methychinazolin werden in 74 ml Toluol gelöst und auf -70°C gekühlt. Über 30 min werden 9,5 ml (11,4 mmol) einer 1,2 M Disobutylaluminiumhydrid Lösung in Toluol zugetropft. Man lässt die Reaktionsmischung auf -40°C erwärmen und rührt 4 Stunden bei -40°C. Es wird langsam Wasser zugegeben und 30 Minuten bei Raumtemperatur gerührt bis sich ein Niederschlag bildet, der mittels Filtration durch Celite entfernt wird. Man trennt die Phasen, wäscht mit gesättigter Natriumchlorid Lösung und trocknet über Natriumsulfat. Nach Chromatographie an Kieselgel mit Hexan-Essigester (0-100%) werden 64 mg des Produkts erhalten.
¹H-NMR (300 MHz, CDCl₃); δ = 2.83 (s, 3H), 4.67 (br., 2H), 6.50 (dd, 1H), 6.93 (dd, 1H), 9.23 (s, 1 H).

Analog Beispiel 6 werden 15 mg des entsprechend gebildeten 3-[1-(3-Chlor-2-methoxyphenyl)-cyclopropyl]-1-[(7-fluor-2-methyl-chinazol-5-yl)imino]-2-(trifluormethyl)propan-2-ol mit BBr₃ Lösung behandelt und eine Stunde refluxiert. Nach Aufarbeitung und Chromatographie an Kieselgel (Ethylacetat) erhält man 1.6 mg gewünschtes Produkt.
¹H-NMR (300 MHz, CDCl₃); δ = 1.82 (d, 3H), 2.60 (d, 1H), 2,81 (s, 3H), 3.24 (d, 1H), 4.60 (d, 1H), 5.94 (q, 1H), 6.08 (d, 1 H), 6.24 (dd, 1H), 6.88 (d, 1H), 6.90 (dd, 1H), 7.27 (d, 1H), 9.25 (s, 1 H).

### Beispiel 8

### (cis,Z)- 4-Ethyliden-6-fluor-1-[(7-fluor-2-methylchinazolin-5-yl)amino]--2-(trifluormethyl)-1,2,3,4-tetrahydronaphthalin-2,5-diol

Analog Beispiel 5 wird aus 800 mg (2.61 mmol) 3-[1-(3-Fluor-2-methoxyphenyl)-cyclopropyl]-2-hydroxy-2-(trifluormethyl)propanal und 500 mg (2.82 mmol) 5-Amino-7-fluor-2-methyl-chinazolin quantitativ 1-[(7-Fluor-2-methyl-chinazol-5-yl)imino]-3-[1-(3-fluor-2-methoxyphenyl)-cyclopropyl]-2-(trifluormethyl)propan-2-ol hergestellt. Die Behandlung mit 24 ml (24 mmol) BBr₃ Lösung und anschliessendes Refluxieren über 14 Stunden ergeben 130 mg gewünschtes Produkt nach analoger Chromatographie und HPLC.
¹H-NMR (300MHz, CD₃OD); δ = 1.77 (d, 3H), 2.57 (d, 1H), 2,80 (s, 3H), 3.14 (d, 1H), 4.64 (s, 1H), 5.86 (q,1 H), 6.26 (dd, 1 H), 6.77- 6.97 (m, 2H), 7.02 (dd, 1 H), 9.57 (s, 1 H).

**Beispiel 8A /8B** (*rac,cis,Z*)-4-Ethylen-6-Fluor-1-[(7-fluor-2-methylchinazolin-5-yl)amino]-2-(trifluormethyl)-1,2,3,4-tetrahydronaphthalin-2,5-diol wird mittels präparativer chiraler HPLC (Chiracel OD-H 5µ) in die enanantiomerenreinen Verbindungen gespalten:
(+)-Enantiomer: analytische HPLC: Rₜ = 10.4 min (Chiralcel OD 10µ, 250x4.6 mm, Hexan / Ethanol 7%, 1 ml/min Fluß)
(-)-Enantiomer: analytische HPLC: Rₜ = 16.5 min (Chiralcel OD 10µ, 250x4.6 mm, Hexan / Ethanol 7%, 1 ml/min Fluß)

### Beispiel 9

### (cis, Z)-4-Ethyliden-6-fluor-1-[(8-fluor-2-methylchinazolin-5-yl)amino]-2-(trifluormethyl)-1,2,3,4-tetrahydronaphthalin-2,5-diol

### 5-Amino-8-fluor-2-methylchinazolin

Zu einer Lösung von 3.35 g (20.25 mmol) Chloralhydrat und 21.27 g (149.7 mmol) Natriumsulfat in 72 ml Wasser wird eine 50°C warme Lösung von 2.4 g (18.6 mmol) 2,5-Difluoranilin in 11 ml Wasser und 1.6 ml konz. Salzsäure (37%) gegeben, die bei dieser Temperatur vorher 1 h gerührt wurde. Man rührt weitere 30 min bei RT und erhitzt nach der Zugabe von 4.09 g (58.9 mmol) Hydroxylammoniumchlorid in 19 ml Wasser über 45 min auf 125°C und hält diese Temperatur für 5 min. Nach dem Abkühlen und einer weiteren Stunde wird der ausgefallene hellbraune Niederschlag abfiltriert, mit Wasser gewaschen und getrocknet. Man erhält 3.0 g (15.0 mmol) des Hydroxylimins als Zwischenprodukt, die portionsweise in 15 ml konz. Schwefelsäure bei 60°C gelöst werden. Nach vollständiger Zugabe erhitzt man 2 Stunden auf 80°C und 4 Stunden auf 90°C. Man läßt abkühlen und gießt die Lösung auf 100 g Eis. Man extrahiert mit Ethylacetat, wäscht die organische Phase mit Wasser, trocknet über Natriumsulfat und engt ein. Nach Chromatographie an Kieselgel mit Hexan-Essigester (0-45 %) werden 1,2 g (7.1 mmol) des 4,7-Difluorisatins erhalten. Zum Isatin in 30 ml einer 1 molaren Natronlauge werden über 10 min 1.8 ml einer 30%igen Wasserstoffperoxid Lösung getropft. Nach 2 Stunden Rühren bei RT wird auf 0°C gekühlt und es werden 5 ml einer 4 molaren Salzsäure zugegeben und mit 50 ml Wasser verdünnt. Man extrahiert mit Ethylacetat, trocknet über Natriumsulfat, engt ein und erhält so quantitativ 1.27 g der 3,6-Difluoranthranilsäure, die ohne weitere Aufreinigung umgesetzt wird.
Die 3,6-Difluoranthranilsäure wird in 8 ml Essigsäureanhydrid 45 min lang auf 100°C erhitzt. Nach dem Abkühlen wird die entstandene Essigsäure und überschüssiges Essigsäureanhydrid azeotrop mit Toluol im Vakuum entfernt. Der Rückstand wird unter Eiskühlung mit 40 ml einer 25%igen Ammoniaklösung versetzt und 72 Stunden gerührt. Man verdünnt mit Wasser und säuert mit Essigsäure an. Man extrahiert mit Ethylacetat, wäscht die organische Phase mit Wasser, trocknet über Natriumsulfat und engt ein. Die so erhaltenen 1,03 g (5.25 mmol) 5,8-Difluor-2-methyl-3H-chinazolin-4-on und 6 g Phosphor-pentachlorid werden in 20 ml Phosphorylchlorid über 12 h auf 125°C erhitzt. Nach dem Abkühlen gießt man in ges. NaHCO₃ Lösung und extrahiert mit Ethylacetat. Die organische Phase wird getrocknet und das Lösungsmittel entfernt. Man erhält quantitativ 1.7 g 4-Chlor-5,8-difluor-2-methylchinazolin, die in 60 ml Ethylacetat und 5 ml Triethylamin gelöst werden. Man gibt 600 mg Palladium auf Kohle zu und schüttelt 2 h (480 ml Wasserstoffaufnahme) unter einer Wasserstoffatmosphäre bei Normaldruck. Die Lösung wird mittels Filtration über Celite vom Katalysator befreit, wobei mit 100 ml Ethanol nachgewaschen wird, und eingedampft. Nach Chromatographie an Kieselgel mit Hexan-Essigester-Ethanol (0-40 %) werden 550 mg 5,8-Difluor-2-methylchinazolin erhalten. Zu 240 mg (1.3 mmol) 5,8-Difluor-2-methylchinazolin, 300 mg (1.13 mmol) 18-Krone-6 in 10 ml DMF werden 890 mg (13.7 mmol) Natriumazid gegeben und man erhitzt die Mischung über 8 h auf 125°C. Das Lösungsmittel wird im Vakuum entfernt und man chromatographiert an Kieselgel mit Ethylacetat und erhält 52 mg Produkt.
¹H-NMR (300 MHz, CDCl₃); δ = 2.92 (s, 3H), 4.31 (br., 2H), 6.67 (dd, 1H), 7.38 (dd, 1H), 9.37 (s, 1H).
Zu 400 mg (1.3 mmol) 3-[1-(3-Fluor-2-methoxyphenyl)-cyclopropyl]-2-hydroxy-2-(trifluormethyl)propanal und 266 mg (1.5 mmol) 5-Amino-8-fluor-2-methylchinazolin in 50 ml Toluol werden 0.62 ml (2.9 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Die so roh erhaltenen 600 mg 1-[(7-Fluor-2-methyl-chinazol-5-yl)imino]-3-[1-(3-fluor-2-methoxyphenyl)-cyclopropyl]-2-(trifluormethyl)propan-2-ol werden in 60 ml Dichlormethan aufgenommen und auf 0°C gekühlt. Man tropft über 10 Minuten 13.5 ml (13.5 mmol) einer 1 M Bortribromid Lösung in Dichlormethan zu und refluxiert die Lösung im Anschluß über 4.5 Stunden. Man gießt die erkaltete Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und Chromatographie an Kieselgel (Hexan / 2-Propanol 0-15%) erhält man 300 mg des gewünschten Produktes.
¹H-NMR (300 MHz, CDCl₃); δ = 1.83 (d, 3H), 2.64 (d, 1H), 2,89 (s, 3H), 3.18 (d, 1H), 4.60 (d, 1H), 5.53 (d, 1H), 5.95 (q,1H), 6.45 (dd, 1H), 6.80 (dd, 1H), 6.97 (d, 1H), 7.41 (dd, 1H), 9.34 (s, 1H).

**Beispiel 9A /9B** (*rac*,*cis*,*Z*)- 4-Ethyliden-6-fluor-1-[(8-fluor-2-methylchinazolin-5-yl)amino]-2-(trifluormethyl)-1,2,3,4-tetrahydronaphthalin-2,5-diol wird mittels präparativer chiraler HPLC (Chiracel OD 10µ) in die enanantiomerenreinen Verbindungen gespalten:
(+)-Enantiomer: analytische HPLC: Rₜ = 15.7 min (Chiralcel OD 10µ, 250x4.6 mm, Hexan / Ethanol 7%, 1 ml/min Fluß)
(-)-Enantiomer: analytische HPLC: Rₜ = 26.1 min (Chiralcel OD 10µ, 250x4.6 mm, Hexan / Ethanol 7%, 1 ml/min Fluß)

### Beispiel 10

### (cis,Z)-4-Ethyliden-6-fluor-1-[(8-fluor-2-methylchinazolin-5-yl)amino]-2-(trifluormethyl)-1,2,3,4-tetrahydronaphthalin-2,5-diol

Analog Beispiel 6 werden 420 mg des entsprechend gebildeten 3-[1-(3-Chlor 2-methoxyphenyl)-cyclopropyl]-1-[(8-fluor-2-methyl-chinazol-5-yl)imino]-2-(trifluormethyl)propan-2-ol mit BBr₃ Lösung behandelt und zwei Stunden refluxiert. Nach Aufarbeitung und Chromatographie an Kieselgel (Hexan /2-Propanol 20-30%) erhält man 13 mg gewünschtes Produkt.
¹H-NMR (300 MHz, CD₃OD); δ = 1.75 (d, 3H), 2.57 (d, 1 H), 2.87 (s, 3H), 3.14 (d, 1 H), 4.60 (s, 1H), 5.86 (q, 1H), 6.41 (dd, 1 H), 6.85 (d, 1 H), 7.25 (d, 1 H), 7.48 (dd, 1H), 9.68 (s, 1H).

### Beispiel 11

### (cis)-6-Fluor-1-[(8-fluor-2-methylchinazolin-5-yl)amino]-4-isopropyliden-2-(trifluormethyl)-1,2,3,4-tetrahydronaphthalin-2,5-diol

### 4-(3-Fluor-4-methoxy-phenyl)-2-hydroxy-5-methyl-2-(trifluoromethyl)-hex-4-enal

Zu 20 ml (214 mmol) 2-Fluorphenol in 150 ml Dichlormethan und 24 ml Pyridin werden bei 0°C 24.5 ml (235 mmol) 2-Methyl-propionsäurechlorid getropft. Man rüht die Mischung zwei Stunden lang und gibt 200 ml einer 1 M Salzsäure zu. Es wird mit Dichlormethan extrahiert und mit Wasser gewaschen. Nach dem Trocknen über Natriumsulfat und dem Entfernen des Lösungsmittels im Vakuum erhält man quantitativ 43 g 2-Methylpropionsäure-2-fluorphenyl-ester. 43 g (214 mmol) 2-Methylpropionsäure-2-fluorphenyl-ester in 20 ml 1,2-Dichlorbenzol werden zu 28 g Aluminiumtrichlorid in 25 ml 1,2-Dichlorbenzol getropft und die Mischung wird im Anschluss 24 Stunden lang bei 100°C gerührt. Man lässt abkühlen, verdünnt mit Dichlormethan und giesst vorsichtig auf eine Mischung von 4 M Salzsäure und Eis. Die Phasen werden getrennt, es wird mit Dichlormethan extrahiert, mit Wasser gewaschen und über Natriumsulfat getrocknet. Das Rohprodukt wird säulenchromatographisch an Kieselgel (Hexan / Ethylacetat 0-40%) gereinigt und man erhält 26.5 g 1-(3-Fluor-2-hydroxyphenyl)-2-methyl-propan-1-on und 12 g 1-(3-Fluor-4-hydroxyphenyl)-2-metyl-propan-1-on. 4.4 g (24 mmol) 1-(3-Fluor-4-hydroxyphenyl)-2-metyl-propan-1-on werden in 30 ml DMF gelöst und es werden 3.73 g (27 mmol) Kaliumcarbonat und 1.7 ml (27 mmol) Methyliodid zugegeben. Die Mischung wird 20 Stunden bei Raumtemperatur gerührt und man giesst in Wasser und extrahiert mit Diethylether /Hexan (1:1) und Ethylacetat. Es wird mit Sole gewaschen, über Natriumsulfat getrocknet und nach dem Entfernen des Lösungsmittels im Vakuum erhält man quantitativ 4.8 g 1-(3-Fluor-4-methoxyphenyl)-2-methyl-propan-1-on.
3.25 g (50 mmol) Zinkstaub und 139 mg (0.5 mmol) Blei(II)-chlorid werden in 50 ml THF suspendiert und bei Raumtemperatur werden 2.9 ml (26 mmol) Dibrommethan zugegeben. Man rührt weitere 30 Minuten und tropft 5.5 ml (5.5 mmol) einer 1 M Titan(IV)-chlorid Lösung in Dichlormethan zu, wobei sich die Mischung leicht erwärmt. Nach einer Stunde werden bei Raumtemperatur 1.0 g (5.1 mmol) 1-(3-Fluor-4-methoxyphenyl)-2-methyl-propan-1-on in 10 ml THF zugetropft. Man rührt weitere 13 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Isopropylether 0-20%) gereinigt und man erhält 0.47 g 2-Fluor-1-metoxy-6-(2-methyl-1-methylenpropyl)-benzen.
¹H-NMR (300 MHz, CDCl₃); δ = 1.09 (d, 6H), 2.76 (m, 1H), 3.89 (s, 3H), 5.00 (s, 1H), 5.11 (s, 1H), 6.90 (dd, 1H), 7.08-7.13 (m, 2H), 9.38.
Zu 69 mg (0.24 mmol) 1,1'-Bi-2-naphthol in 0.5 ml Toluol werden 0.25 ml (0.12 mmol) einer 0.5 M Titantetraisopropylat Lösung in Toluol gegeben und man rührt die rote Lösung für 2 Stunden bei Raumtemperatur. 0.45 g (2.3 mmol) 2-Fluor-1-methoxy-6-(2-methyl-1-methylenpropyl)-benzen und 780 mg (4.6 mmol) Ethyltrifluorpyruvat werden zugegeben und man rührt 24 Stunden bei 0°C, 24 Stunden bei Raumtemperatur und erhitzt die Mischung über 5 Stunden auf 100°C. Nach dem Abkühlen wird sofort säulenchromatographisch an Kieselgel (Hexan / Ethylacetat 0-20%)gereinigt und man erhält 250 mg 4-(3-Fluor-4-methoxyphenyl)-2-hydroxy-5-methyl-2-(trifluormetyl)-hex-4-ensäure-ethylester. 250 g (0.7 mmol) 4-(3-Fluor-4-methoxyphenyl)-2-hydroxy-5-methyl-2-(trifluormetyl)-hex-4-ensäure-ethylester werden in 10 ml Diethylether auf -5°C gekühlt und über 10 Minuten werden portionsweise 56 mg (1.4 mmol) Lithiumaluminiumhydrid fest zugegeben. Man rührt 2 Stunden bei 0° C und giesst in gesättigte Ammoniumchlorid Lösung. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Ethylacetat 30%) liefert 140 mg 4-(3-Fluor-4-methoxyphenyl)-2-hydroxy-5-methyl-2-(trifluormetyl)-hex-4-enal und 80 mg Alkohol.
¹H-NMR (300 MHz, CDCl₃); δ =1.56 (s, 3H), 1.88 (s, 3H), 3.09 (d, 1H), 3.26 (d, 1H), 3.67 (s, 1H), 3.89 (s, 3H), 6.68-6.90 (m, 3H), 9.14 (s, 1H).
Zu 75g (0.23mmol) 4-(3-Fluor-2-methoxyphenyl)-2-hydroxy-5-methyl-2-(triftuormetyl)-hex-4-enal und 41 (0.23mol) 5-Amino-8-fluor-2-methylchinazolin in 4ml Toluol werden 0.2 ml (1.0mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 115 5 mg 4-(3-Fluor-4-methoxyphenyl)1-[(8-fluor-2-methylchinazolin-5-yl)-imino]-5-methyl-2-(trifluormetyl)-hex-4-en-2-ol als Rohprodukt. Das Imin wird in 7 ml Dichlormethan aufgenommen und auf -78°C gekühlt. Man tropft über 5 Minuten 1.3 ml (1.3 mmol) einer 1 M Titantetrachlorid Lösung in Dichlormethan zu und entfernt nach 20 Minuten das Kühlbad. Nach weiteren 15 Minuten gießt man die auf 0°C erwärmte Lösung auf eine Mischung von Eis und gesättigter Natriumhydrogencarbonat Lösung und rührt 10 Minuten lang heftig. Es wird mit Ethylacetat extrahiert, mit gesättigter Natriumchloridlösung gewaschen und über Natriumsulfat getrocknet. Nach Einengen und Chromatographie an Kieselgel (Hexan / Ethylacetat 50%) erhält man 11 mg des gewünschten Produktes.
¹H-NMR (300 MHz, CDCl₃); δ = 1.93 (s, 3H), 2.03 (s, 3H), 2.69 (d, 1H), 2.90 (s, 3H), 3.17 (d, 1H), 3.68 (s, 3H), 4.68 (d, 1H), 5.52 (d, 1H), 6.54 (dd, 1H), 6.90 (d, 1H), 7.10 (d, 1 H), 7.43 (dd, 1H), 9.38 (s, 1 H).

### Beispiel 12 (cis,Z)-1-[(7,8-Difluor-2-methylchinazolin-5-yl)amino]-4-ethyliden-6-fluoro-2-(trifluormethyl)-1,2,3,4-tetrahydronaphthalin-2,5-diol

### 5-Amino-7,8-difluoro-2-methylchinazolin

Zu 41.7 g (180 mmol) 2,2-Dimethyl-N-(3,4,5-trifluorphenyl)-propionamid in 385 ml THF werden bei -70°C 156 ml (391 mmol) einer 2.5M Butyllithium Lösung in Hexan getropft. Man läßt eine Sunde rühren und tropft anschließend 38,6 ml DMF in 90 ml THF zu, wobei sich die Lösung auf -60°C erwärmen darf. Man rührt eine weitere Stunde bei - 70°C und gießt dann die kalte Reaktionslösung auf ein Gemisch von 2 kg Eis und 400 ml konzentrierter Salzsäure. Man rührt heftig und extrahiert nach einer Stunde mehrfach mit Diethylether. Die organische Phase wird mit Wasser neutral gewaschen und über Natriumsulfat getrocknet. Nach dem Einengen erhält man 49.3 g (188 mmol) rohen 4,5,6-Trifluor-2-N-pivaloylaminobenzaldehyd, der mit 26 g (275 mmol) Acetamidin Hydrochlorid, 38.3 g
(277 mmol) Kaliumcarbonat und 30 g Molekularsieb (4A) in 206 ml Butyronitril zusammengegeben wird. Man erhitzt unter heftigem Rühren 18 Stunden auf 145°C und entfernt das Lösungsmittel im Vakuum. Nach Chromatographie des Rückstands an Kieselgel mit Hexan/Ethylacetat (0-100%) erhält man 9.1 g 7,8-Difluoro-5-N-pivaloylamino-2-methylchinazolin.
2.0 g (7.2 mmol) 7,8-Difluoro-5-N-pivaloylamino-2-methychinazolin werden in 140 ml Toluol gelöst und auf -70°C gekühlt. Über 30 min werden 24 ml (28.8 mmol) einer 1,2 M Diisobutylaluminiumhydrid Lösung in Toluol zugetropft. Man lässt die Reaktionsmischung über 2 Stunden auf -25°C erwärmen und rührt 2 Stunden bei - 25°C. Es wird langsam Isopropanol und anschließend Wasser zugegeben und 12 Stunden bei Raumtemperatur gerührt bis sich ein Niederschlag bildet, der mittels Filtration durch Celite entfernt wird. Man wäscht gut mit einem Methylenchlorid Methanol Gemisch nach und engt ein. Der Rückstand wird in 200 ml Ethylacetat und 50 ml Methanol zusammen mit 100 g Kieselgel und 20 g Mangandioxid heftig gerührt. Man filtriert durch Celite, wäscht mit gut mit einem Methylenchlorid-Methanol-Gemisch nach und engt ein. Nach Chromatographie an Kieselgel (Hexan-Essigester 0-100%) werden 370 mg des Produkts erhalten.
¹H-NMR (300 MHz, CD₃OD): δ = 2.81 (s, 3H), 6.64 (dd, 1H), 9.52 (s, 1 H).
Analog Beispiel 6 werden 33 mg des entsprechend gebildeten 3-[1-(3-Chlor-2-methoxyphenyl)-cyclopropyl]-1-[(7,8-difluor-2-methyl-chinazol-5-yl)imino]-2-(trifluormethyl)propan-2-ol mit BBr₃ Lösung behandelt und eine Stunde refluxiert. Nach Aufarbeitung und Chromatographie an Kieselgel (Hexan / 2-Propanol 15%) erhält man 4.5 mg gewünschtes Produkt.
¹H-NMR (300 MHz, CDCl₃); δ = 1.83 (d, 3H), 2.61 (d, 1H), 2.87 (s, 3H), 3.22 (d, 1 H), 4.55 (d, 1 H), 5.90 (d, 1H), 5.95 (q, 1H), 6.30 (dd, 1H), 6.82 (dd, 1H), 7.01 (dd, 1H), 9.31 (s, 1 H).

### Beispiel 13 (cis,E)-1-[(7,8-Difluor-2-methylchinazolin-5-yl)amino]-4-ethyliden-6-fluoro-5-methoxy-2-(trifluormethyl)-1,2,3,4-tetrahydronaphthalin-2-ol

### E-4-(3-Fluor-2-methoxy-phenyl)-2-hydroxy-2-(trifluoromethyl)-pent 4-enal.

Zu 20 ml (214 mmol) 2-Fluorphenol in 150 ml Dichlormethan und 24 ml Pyridin werden bei 0°C 19.6ml (225 mmol) Propionsäurechlorid getropft. Man rüht die Mischung zwei Stunden lang und gibt 200 ml einer 1 M Salzsäure zu. Es wird mit Dichlormethan extrahiert und mit Wasser gewaschen. Nach dem Trocknen über Natriumsulfat und dem Entfernen des Lösungsmittels im Vakuum erhält man quantitativ 36 g Propionsäure-2-fluorphenyl-ester. 36 g (214 mmol) Propionsäure-2-fluorphenyl-ester in 20 ml 1,2-Dichlorbenzol werden zu 28 g Aluminiumtrichlorid in 25 ml 1,2-Dichlorbenzol getropft und die Mischung wird im Anschluss 24 Stunden lang bei 100°C gerührt. Man lässt abkühlen, verdünnt mit Dichlormethan und giesst vorsichtig auf eine Mischung von 4 M Salzsäure und Eis. Die Phasen werden getrennt, es wird mit Dichlormethan extrahiert, mit Wasser gewaschen und über Natriumsulfat getrocknet. Das Rohprodukt wird säulenchromatographisch an Kieselgel (Hexan / Ethylacetat 0-40%) gereinigt und man erhält 17 g 1-(3-Fluor-2-hydroxyphenyl)-propan-1-on und 12.7g 1-(3-Fluor-4-hydroxyphenyl)-propan-1-on. 17 g (102 mmol) 1-(3-Fluor-2-hydroxyphenyl)-propan-1-on werden in 160 ml Aceton gelöst und es werden 26 g Kaliumcarbonat und 11.5 ml Methyliodid zugegeben. Die Mischung wird 7 Stunden bei 70°C gerührt und das Lösungsmittel im Anschluss zu einem grossen Teil entfernt. Man giesst den Rückstand in Wasser und extrahiert mit Diethylether. Es wird mit Wasser gewaschen, über Natriumsulfat getrocknet und nach dem Entfernen des Lösungsmittels im Vakuum erhält man quantitativ 18.5 g 1-(3-Fluor-2-methoxyphenyl)-propan-1-on.
38.5 g (589 mmol) Zinkstaub und 804 mg (2.9 mmol) Blei(II)-chlorid werden in 595 ml THF suspendiert und bei Raumtemperatur werden 36.ml (513 mmol) Dibrommethan zugegeben. Man rührt weitere 30 Minuten und tropft bei 0°C 68.8 ml (68.8 mmol) einer 1 M Titan(IV)-chlorid Lösung in Dichlormethan zu. Das Kühlbad wird entfernt und nach 30 Minuten bei Raumtemperatur werden 12.5 g (1-(3-Fluor-2-methoxyphenyl)-propan-1-on in 128 ml THF zugetropft. Man rührt weitere 5 Stunden bei Raumtemperatur, wobei nach 2 Stunden eine leichte Erwärmung einsetzte, die mittels Wasserbad gekühlt wurde. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan /Isopropylether 0-3%) gereinigt und man erhält 8.1 g 2-Fluor-6-(1-methylenpropyl)-phenol.
Zu 896 mg (3.3 mmol) 1,1'-Bi-2-naphthol werden 3.3 ml (1.65 mmol) einer 0.5 M Titantetraisopropylat Lösung in Toluol gegeben und man rührt die rote Lösung für 2 Stunden bei Raumtemperatur. 5.07 g (28.1 mmol) 2-Fluor-6-(1-methylenpropyl)phenol und 7.3 ml (60.8 mmol) Ethyltrifluorpyruvat werden zugegeben und man erhitzt die Mischung über 17 Stunden auf 140°C. Nach dem Abkühlen wird sofort säulenchromatographisch an Kieselgel (Hexan / Ethylacetat 0-5%)gereinigt und man erhält 4.6 g 4-(3-Fluor-2-methoxyphenyl)-2-hydroxy-2-(trifluormetyl)-hex-4-ensäure-ethylester als E/Z Gemisch. 2.0 g (5.7 mmol) *E*-4-(3-Fluor-2-methoxyphenyl)-2-hydroxy-2-(trifluormetyl)-hex-4-ensäure-ethylester werden in 100 ml Diethylether auf -5°C gekühlt und über 10 Minuten werden portionsweise 498 mg (13.1 mmol) Lithiumaluminiumhydrid fest zugegeben. Man rührt 2 Stunden bei Raumtemperatur und giesst in gesättigte Ammoniumchlorid Lösung. Die Suspension wird durch Celite filtriert
wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Ethylacetat 0-15%) liefert 0.51g *E*-4-(3-Fluor-2-methoxyphenyl)-2-hydroxy-2-(trifluormethyl)-hex-4-enal und 1.2 g Alkohol.
¹H-NMR (300 MHz, CDCl₃); δ = 1.85 (d, 3H), 3.31 (d, 1H), 3.42 (d, 1H), 3.71 (s,1H), 3.89 (s, 3H), 5.78 (q, 1H), 6.67 (d, 1H), 6.92 (ddd, 1H), 7.00 (ddd, 1H), 9.31 (s, 1H).

Analog Beispiel 11 werden 77 mg des entsprechend gebildeten *E*-1-[(7,8-Difluor-2-methylchinazolin-5-yl)-imino]-4-(3-Fluor-2-methoxyphenyl)-2-(trifluormetyl)-hex-4-en-2-ol bei -78°C 30 Minuten mit Titantetrachlorid Lösung behandelt. Nach Aufarbeitung und Chromatographie an Kieselgel (Hexan /Ethylacetat 50%) erhält man 4 mg gewünschtes Produkt.
¹H-NMR (300 MHz, CDCl₃); δ = 1.94 (d, 3H), 2.89 (s, 3H), 2.92 (d, 1H), 2.99 (d, 1H), 3,88 (s, 3H), 4.79 (d, 1H), 5.69 (d, 1H), 6.45 (dd,1 H), 6.77 (q, 1H), 6.92 (dd, 1H), 6.93 (dd, 1H), 9.28 (s, 1H).

### Beispiel 14 (cis/trans, E)-4-Ethyliden-6-fluoro-5-methoxy-1-[(2-methylchinolin-5-yl)amino]-2-(trifluormethyl)-1,2,3,4-tetrahydronaphthalin-2-ol

Analog Beispiel 11 werden 150 mg (0.49 mmol) *E*-4-(3-Fluor-2-methoxyphenyl)-2-hydroxy-2-(trifluormetyl)-hex-4-enal mit 78 mg (0.49 mmol) 5-Amino-2-methylchinolin zum entsprechenden *E*-4-(3-Fluor-2-methoxyphenyl)-1-[(2-methylchinazolin-5-yl)imino]2-(trifluormetyl)-hex-4-en-2-ol umgesetzt. Das Imin wird in 15 ml Dichlormethan aufgenommen und bei -30°C mit 5 ml (5 mmol) einer 1M Bortribromid Lösung behandelt. Man läßt über 30 min auf Raumtemperatur erwämen und gießt die Lösung nach 4 Stunden auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und Chromatographie an Kieselgel (Hexan / Ethylacetat 30 - 60%) erhält man 44 mg der Titelverbindung und 82 mg der Verbindung aus Beispiel 15.
¹H-NMR (300 MHz, CDCl₃); δ = 1.86 (d, 1.5H), 1.96 (d, 1.5H), 2.65 (d, 0.5H), 2.72 (s, 3H), 2.92-2.97 (m, 1H), 3.04 (d, 0.5H), 2.99 (d, 1H), 4.70 (d, 0.5H), 4.77-4.85 (m, 1H) 4.94 (d, 0.5H), 5.99 (q, 0.5H), 6.61 (d,0.5H), 6.67-6.96 (m, 3H), 7.24 (d, 1H), 7.51 (m, 2H), 8.01 (d, 1H).

### Beispiel 15 (cis)-9-Fluor-3-methyl-1-[(2-methylchinolin-5-yl)amino]-5(trifluormethyl)-5,6-dihydro-4H-1-oxa-2-boraghenalen-2,5-diol

Darstellung ist in Beispiel 14 beschrieben.
¹H-NMR (300 MHz, CDCl₃); δ = 2.06 (s, 3H), 2.71 (s, 3H), 3.15 (d, 1 H), 3.32 (d, 1H), 4.89 (d, 1H), 5.19 (d, 1H), 6.81 (d, 1H), 7.05 (m, 2H), 7.23 (d, 1H), 7.48-7.56 (m, 2H), 8.08 (d, 1H).

### Beispiel 16 (cis)-9-Fluor-6-[(2-methylchinolin-5-yl)amino]-5-(trifluormethyl)-2,4,5,6-tetrahydrobenzo[de]chromen-5-ol

### 3-(8-Fluor-2H-chromen-4-yl)-2-hydroxy 2-(trifluormethyl)-propanal:

33.2 g (296 mmol) 2-Fluorphenol und 18.4 ml 281 mmol Acrylnitril werden zusammen mit 5.0g (29.6 mmol) Bezyltrimethylammoniumhydroxid 4 Tage bei 80°C gerührt. Bei Raumtemperatur wird mit Eis versetzt und 2N Salzsäure zugegeben. Man rührt 10 Minuten, extrahiert mit Ethylacetat, wäscht mit gesättigter Natriumhydogencarbonat und Natriumchlorid Lösung, trocknet über Natriumsulfat und entfernt das Lösungsmittel im Vakuum. Die säulenchromatographische Trennung an Kieselgel (Hexan / Ethylacetat 5-50%) liefert 6.4 g 3-(2-Fluorphenoxy)-propionitril. 6.4g (38.8 mmol) 3-(2-Fluorphenoxy)-propionitril werden in 38.6 ml konzentrierter Salzsäure über 2 Stunden refluxiert. Bei Raumtemperatur wird mit Eiswasser verdünnt und 10 Minuten gerührt. Man extrahiert mit Ethylacetat, wäscht mit gesättigter Ammoniumchlorid Lösung und trocknet über Natriumsulfat. Nach dem Entfernen des Lösungsmittels, im Vakuum erhält man 6.5 g 3-(2-Fluorphenoxy)-propionsäure. 6.5 g (35.6 mmol) 3-(2-Fluorphenoxy)-propionsäure werden zu 39 g Polyphosphorsäure gegeben und vier Stunden bei 70°C gerührt. Nach dem Abkühlen über Nacht wird auf Eiswasser gegossen. Man extrahiert mit Ethylacetat, wäscht mit gesättigter Natriumhydogencarbonat und Natriumchlorid Lösung und trocknet über Natriumsulfat. Nach dem Entfernen des Lösungsmittels im Vakuum erhält man 5.5 g 8-Fluorchroman-4-on als kristallinen Feststoff.
29.8 g (456 mmol) Zinkstaub und 710mg (2.5 mmol) Blei(II)chlorid werden in 450 ml THF suspendiert und bei Raumtemperatur werden 28.6 ml (253 mmol) Dibrommethan zugegeben. Man rührt weitere 60 Minuten und tropft 50.7 ml (50.7 mmol) einer 1 M Titan(IV)-chlorid Lösung in Dichlormethan über 40 Minuten unter Eiskühlung zu. Nach einer Stunde werden bei Raumtemperatur 8.4 g (50.7 mmol) 8-Fluorchroman-4-on in 500 ml THF zugetropft. Man rührt weitere 18 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Isopropylether 0-20%) gereinigt und man erhält 0.81 g 8-Fluor-4-methylen-chroman.
Zu 281 mg (0.98 mmol) 1,1'-Bi-2-naphthol werden 0.98 ml (0.49 mmol) einer 0.5 M Titantetraisopropylat Lösung in Toluol gegeben und man rührt die rote Lösung für 2 Stunden bei Raumtemperatur. 0.81 g (4.9 mmol) 8-Fluor-4-methylen-chroman und 1.21 ml (9.8 mmol) Ethyltrifluorpyruvat werden zugegeben und man erhitzt die Mischung über 3 Stunden auf 120°C. Nach dem Abkühlen wird sofort säulenchromatographisch an Kieselgel (Hexan / Ethylacetat 0-20%)gereinigt und man erhält 0.69 g 3-(8-Fluor-2H-chromen-4-yl)-2-hydroxy-2-(trifluormethyl)-propionsäure-ethylester. 345 mg (1.0 mmol) 3-(8-Fluor-2H-chromen-4-yl)-2-hydroxy-2-(trifluormethyl)-propionsäure-ethylester werden in 10 ml Diethylether auf -5°C gekühlt und über 10 Minuten werden portionsweise 78 mg (2.0 mmol) Lithiumaluminiumhydrid fest zugegeben. Man rührt 2 Stunden bei Raumtemperatur und giesst in gesättigte Ammoniumchlorid Lösung. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die chromatographische Trennung an Kieselgel (Hexan / Ethylacetat 0-15%) liefert 46 mg 3-(8-Fluor-2H-chromen-4-yl)-2-hydroxy-2-(trifluormethyl)-propanal und 71 mg Alkohol.
¹H-NMR (300 MHz, CDCl₃); δ = 3.07 (d, 1H), 3.25 (d, 1H), 3,89 (s, 1H), 4.77 (m, 2H), 5.79 (t, 1H), 6.85 (ddd, 1H), 6.95-7.01 (m, 2H), 9.64 (s, 1H).

Analog Beispiel 11 werden 46 mg (0.16 mmol) 3-(8-Fluor-2H-chromen-4-yl)-2-hydroxy-2-(trifluormethyl)-propanal mit 26 mg (0.17 mmol) 5-Amino-2-methylchinolin zum entsprechenden 3-(8-Fluor-2H-chromen-4-yl)-1-[(2-methylchinazolin-5-yl)imino]-2-(trifluormethyl)-propan-2-ol umgesetzt. Das Imin wird in 1.9 ml Dichlormethan aufgenommen und bei -30°C mit 0.95 ml (0.95 mmol) einer 1 M Bortribromid Lösung behandelt. Man läßt über 30 min auf Raumtemperatur erwämen und gießt die Lösung auf eine Mischung von Eis und gesättigter Natriumhydrogencarbonat Lösung und rührt 5 Minuten lang heftig. Es wird mit Dichlormethan extrahiert, mit gesättigter Natriumchloridlösung gewaschen und über Natriumsulfat getrocknet. Nach Einengen und Chromatographie an Kieselgel (Hexan / Ethylacetat 30%) erhält man 7 mg gewünschtes Produkt.
¹H-NMR (300 MHz, CDCl₃); δ = 2.74 (s, 3H), 2.92 (d, 1H), 3.25 (d, 1H), 4.51 (d, 1H), 4.84-4.98 (m, 2H), 5.02 (d, 1H), 6.74 (d, 1H), 6.83 (dd, 1H), 6.86 (dd, 1H), 7.00 (ddd, 1H), 7.28 (d, 1H), 7.55 (t, 1H), 7.59 (d, 1H), 8.12 (d, 1H).
analog können hergestellt werden:

### Beispiel 17

### (cis,Z)-2-Fluor-5-[(-2-methylchinazolin-5-yl)amino]-8-(propyliden)-6-(trifluormethyl)-5,6,7,8-tetrahydronaphthalin-1,6-diol

¹H-NMR (300 MHz, CD₃OD); δ = 1.03 (t, 3H), 2.05 (ddq, 1H), 2.20 (ddq, 1H), 2.49 (d, 1H), 2,77 (s, 3H), 3.08 (d, 1H), 4.59 (s, 1H), 5.67 (dd, 1H), 6.43 (d, 1H), 6.73 (dd, 1H), 6.93 (dd, 1H), 7.13 (d, 1H), 7.65 (t, 1H), 9.59 (s, 1H).

### Beispiel 18

### (cis,E)-2-Fluor-5-[(2-methylchinazolin-5-yl)amino]-8-propyliden-6-(trifluormethyl)-5,6,7,8-tetrahydronaphthalin-1,6-diol

¹H-NMR (300 MHz, CD₃OD); δ = 1.12 (t, 3H), 2.28 (m, 2H), 2.76 (d, 1H), 2,77 (s, 3H), 2.99 (d, 1H), 4.96 (d, 1H), 6.69 - 6.73 (m, 3H), 6.84 (dd, 1H), 7.14 (d, 1H), 7.69 (t, 1H), 9.59 (s, 1H).

### Beispiel 19

### (cis,Z)-2-Fluor-5-[(7-fluor-2-methylchinazolin-5-yl)amino]-8-propyliden-6-(trifluormethyl)-5,6,7,8-tetrahydronaphthalin-1,6-diol

¹H-NMR (300 MHz, CDCl₃); δ = 1.08 (t, 3H), 2.15 (ddq, 1H), 2.25 (ddq, 1H), 2.63 (d, 1H), 2,79 (s, 3H), 3.23 (d, 1H), 4.56 (d, 1H), 5.82 (dd, 1H), 6.21 (m, 2H), 6.80 (dd, 1H), 6.88 (d, 1H), 6.93 (dd, 1H), 9.29 (s, 1H).

### Beispiel 20

### (cis,E)-2-Fluor-5-[(7-fluor-2-methylchinazolin-5-yl)amino]-8-propyliden-6-(trifluormethyl)-5,6,7,8-tetrahydronaphthalin-1,6-diol

¹H-NMR (300 MHz, CDCl₃); δ = 1.08 (t, 3H), 2.12 (ddq, 1H), 2.21 (ddq, 1H), 2,93 (s, 3H), 3.00 (m, 2H), 4.93 (d, 1H), 6.44 (m, 2H), 6.87 - 6.96 (m, 3H), (d, 1H), 7.01 (dd, 1H), 9.28 (s, 1H).

### Beispiel 21

### (cis,E)-3-Chlor-8-ethyliden-2-fluor-5-[(2-methylchinolin-5-yl)amino]-6-(trifluormethyl)-5,6,7,8-tetrahydronaphthalin-1,6-diol

¹H-NMR (300 MHz, CD₃OD); δ = 1.90 (d, 3H), 2,74 (s, 3H), 2.80 (d, 1H), 3.04 (d, 1H), 4.92 (s, 1H), 6.38 (d, 1H), 6.80 (d, 1H), 6.82 (q, 1H), 7.33 (d, 1H), 7.43 (d, 1H), 7.49 (t, 1H), 8.48 (d, 1H).

### Beispiel 22

### (cis,Z)-3-Chlor-8-ethyliden-2-fluor-5-[(2-methylchinolin-5-yl)amino]-6-(trifluormethyl)-5,6,7,8-tetrahydronaphthalin-1,6-diol

¹H-NMR (300 MHz, CD₃OD); δ = 1.77 (d, 3H), 2.54 (d, 1H), 2,75 (s, 3H), 3.14 (d, 1H), 4.56 (s, 1H), 5.86 (q, 1H), 6.61 (d, 1H), 6.81 (d, 1H), 7.34 (d, 1H), 7.44 (d, 1H), 7.53 (t, 1H), 8.50 (s, 1H).

### Beispiel 23

### (cis,Z)-3-Chlor-8-ethyliden-2-fluor-5-[(2-methylchinazolin-5-yl)amino]-6-(trifluormethyl)-5,6,7,8-tetrahydronaphthalin-1,6-diol

¹H-NMR (300 MHz, CD₃OD); δ = 1.78 (d, 3H), 2.58 (d, 1H), 2,77 (s, 3H), 3.10 (d, 1H), 4.62 (s, 1H), 5.89 (q, 1H), 6.45 (d, 1H), 6.93 (d, 1H), 7.13 (d, 1H), 7.65 (t, 1H), 9.59 (s, 1H).

### Beispiel 24

### (cis,Z)-3-Chlor-8-ethyliden-2-fluor-5-[(7-fluor-2-methylchinazolin-5-yl)amino]-6-(trifluormethyl)-5,6,7,8-tetrahydronaphthalin-1,6-diol

¹H-NMR (300 MHz, CD₃OD); δ = 1.77 (d, 3H), 2.57 (d, 1H), 2.81 (s, 3H), 3.14 (d, 1H), 4.64 (s, 1H), 5.90 (q, 1H), 6.30 (d, 1H), 6.82 (d, 1H), 6.88 (d, 1H), 7.13 (d, 1H), 9.58 (s, 1H).

### Beispiel 25

### (cis,Z)-3-Chlor-8-ethyliden-2-fluor-5-[(8-fluor-2-methylchinazolin-5-yl)amino]-6-(trifluormethyl)-5,6,7,8-tetrahydronaphthalin-1,6-diol

¹H-NMR (300 MHz, CD₃OD); δ = 1.72 (d, 3H), 2.52 (d, 1H), 2.84 (s, 3H), 3.10 (d, 1H), 4.54 (s, 1H), 5.83 (q, 1H), 6.39 (dd, 1H), 6.86 (d, 1H), 7.47 (dd, 1H), 9.65 (s, 1H).

### Beispiel 26

### (cis, Z)-3-Chlor-5-[(7,8-difluor-2-methylchinazolin-5-yl)amino]-8-ethyliden-2-fluor-6-(trifluormethyl)-5,6,7,8-tetrahydronaphthalin-1,6-diol

¹H-NMR (300 MHz, CD₃OD); δ = 1.75 (d, 3H), 2.54 (d, 1H), 2.80 (s, 3H), 3.11 (d, 1H), 4.58 (s, 1H), 5.82 (q, 1H), 6.35 (dd, 1H), 6.86 (d, 1H), 7.13 (d, 1H), 9.58 (s, 1H).

### Beispiel 27

### (cis, Z)-5-{[7-Chlor-2,5-dihydroxy-4-ethyliden-6-fluor-2-trifluormethyl-1,2,3,4-tetrahydronaphthalin-1-yl]amino}-chinolin-2(1H)-on

¹H-NMR (300 MHz, CD₃OD); δ = 1.74 (d, 3H), 2.48 (d, 1H), 3.07 (d, 1 H), 4.45 (s, 1H), 5.82 (q, 1H), 6.12 (d, 1H), 6.52 (d, 1H), 6.66 (d, 1H), 6.78 (d, 1H), 7.26 (t, 1H), 8.20 (d, 1H).

### Beispiel 28

### (cis,E)-5-{[7-Chlor-2,5-dihydroxy-4-ethyliden-6-fluor-2-trifluormethyl-1,2,3,4-tetrahydronaphthalin-1-yl]amino}-chinolin-2(1H)-on

¹H-NMR (300 MHz, CD₃OD); δ = 1.84 (d, 3H), 2.74 (d, 1H), 2.95 (d, 1H), 4.82 (s, 1H), 6.36 (d, 1H), 6.52 (d, 1H), 6.70 (d, 1H), 6.78 (d, 1H), 6.80 (q, 1H), 7.32 (t, 1H), 8.22 (d, 1H).

### Beispiel 29

### (cis,Z)-5-{[7-Chlor-2,5-dihydroxy-4-ethyliden-6-fluor-2-trifluormethyl-1,2,3,4-tetrahydronaphthalin-1-yl]amino}-2-methylphthalazin-1-on

¹H-NMR (300 MHz, CD₃OD); δ = 1.71 (d, 3H), 2.50 (d, 1H), 3.09 (d, 1H), 3.81 (s, 3H), 4.52 (s, 1H), 5.81 (q, 1H), 6.76 (d, 1H), 6.82 (d, 1H), 7.55 (t, 1H), 7.62 (d, 1 H), 8.53 (s, 1H).

### Beispiel 30 (cis, E)-3-Chlor-8-ethyliden-2-fluor-5-[(2-methylchinazolin-5-yl)amino]-6-(trifluormethyl)-5,6,7,8-tetrahydronaphthalin-1,6-diol

¹H-NMR (300 MHz, CD₃OD); δ = 1.86 (d, 3H), 2,78 (s, 3H), 2.80 (d, 1H), 2.97 (d, 1 H), 4.97 (s, 1H), 6.71 (d, 1H), 6.78 (d, 1H), 6.85 (q, 1H), 7.17 (d, 1H), 7.71 (t, 1H), 9.60 (s, 1H).

### Beispiel 31

### (cis,E)-3-Chlor-8-ethyliden-2-fluor-5-[(7-fluor-2-methylchinazolin-5-yl)amino]-6-(trifluormethyl)-5,6,7,8-tetrahydronaphthalin-1,6-diol

¹H-NMR (300 MHz, CD₃OD); δ = 1.91 (d, 3H), 2,80 (s, 3H), 2.87 (d, 1 H), 3.01 (d, 1H), 5.07 (s, 1H), 6.61 (d, 1H), 6.82 (d, 1H), 6.83 (d, 1H), 6.95 (q, 1H), 9.59 (s, 1H).

### Beispiel 32

### (cis,E)-3-Chlor-5-[(7,8-difluor-2-methylchinazolin-5-yl)amino]- 8-ethyliden-2-fluor-6-(trifluormethyl)-5,6,7,8-tetrahydronaphthalin-1,6-diol

¹H-NMR (300 MHz, CD₃OD): δ = 1.87 (d, 3H), 2,78 (s, 3H), 2.81 (d, 1H), 2.97 (d, 1H), 4.98 (s, 1H), 6.64 (dd, 1H), 6.79 (d, 1H). 6.89 (q, 1H). 9.57 (s, 1H).

### Beispiel 33

### (cis, E)-3-Chlor-8-ethyliden-[(2-methylchinazolin-5-yl)amino]-1-methoxy-6-(trifluormethyl)-5,6,7,8-tetrahydronaphthalin-6-ol

¹H-NMR (300 MHz, CD₃OD); δ = 1.89 (d, 3H), 2.78 (d, 1H), 2,80 (s, 3H), 3.08 (d, 1H), 3.70 (s, 3H), 4.96 (s, 1H), 6.68 (d, 1H), 6.83 (q, 1H), 6.98 (d, 1H), 7.14 (d, 1H), 7.18 (d, 1H), 7.69 (t, 9.57 (s, 1H).

### Beispiel 34

### (cis,Z)-5,6-Difluor-4-ethyliden-1-[(2-methylchinazolin-5-yl)amino]-2-(trifluormethyl)-1,2,3,4-tetrahydronaphthalin-2-ol

¹H-NMR (300 MHz, CDCl₃); δ = 1.83 (m, 3H), 2.72 (d, 1H), 2,82 (s, 3H), 3.24 (d, 1H), 4.69 (d, 1H), 5.98 (q, 1H), 6.08 (d, 1H), 6.54 (d, 1H), 7.07 - 7.14 (m, 2H), 7.28 (d, 1H), 7.68 (t, 1H), 9.41 (s, 1H).

### Beispiel 35

### (cis,E)-5,6-Difluor-4-ethyliden-1-[(2-methylchinazolin-5-yl)amino]-2-(trifluormethyl)-1,2,3,4-tetrahydronaphthalin-2-ol

¹H-NMR (300 MHz, CD₃OD); δ = 1.90 (d, 3H), 2,77 (s, 3H), 2.94 (m, 2H), 5.15 (s, 1H), 6.63 (q, 1H), 6.80 (d, 1H), 6.98 - 7.09 (m, 2H), 7.17 (d, 1H), 7.72 (t, 1H), 9.60 (s, 1H).

### Beispiel 36

### (cis,Z)-5-Fluor-4-ethyliden-1-[(2-methylchinazolin-5-yl)amino]-2-(trifluormethyl)-1,2,3,4-tetrahydronaphthalin-2,6-diol

¹H-NMR (300 MHz, CD₃OD); δ = 1.74 (m, 3H), 2.57 (d, 1H). 2.77 (s, 3H), 3.08 (d, 1H), 4.71 (s, 1H), 5.86 (q, 1H), 6.54 (d, 1H), 6.80 (dd, 1H), 6.92 (d, 1H), 7.13 (d, 1H), 7.67 (t, 1H), 9.58 (s, 1H).

### Beispiel 37

### (cis,Z)-5-{[2 6-Dihydroxy-4-ethyliden-5-fluor-2-(trifluormethyl)-1,2,3,4-tetrahydronaphthalin-1-yl]amino}-chinolin-2(1H)-on

¹H-NMR (300 MHz, CD₃OD); δ = 1.66 (m, 3H), 2.45 (d, 1H), 2.99 (d, 1H), 4.49 (s, 1H), 5.76 (q, 1H), 6.14 (d, 1H), 6.43 (d, 1H), 6.57 (d, 1H), 6.72 (t, 1H), 6.81 (d, 1H), 7.18 (t, 1H), 8.11 (d, 1H).

### Beispiel 38

### (cis, Z)-4-Ethyliden-7-fluor-1-[(7-fluor-2-methylchinazolin-5-yl)amino]-2-(trifluormethyl)-1,2,3,4-tetrahydronaphthalin-2,6-diol

¹H-NMR (300 MHz, CD₃OD); δ = 1.97 (d, 3H), 2.66 (d, 1H), 2.74 (s, 3H), 2.94 (d, 1H), 5.11 (s, 1H), 5.73 (q, 1H), 6.62 (d, 1H), 6.73 (d, 1H), 6.95 (d, 1H), 7.10 (d, 1H), 9.52 (s, 1H).

### Beispiel 39

### (cis,E)-4-Ethyliden-7-fluor-1-[(7-fluor-2-methylchinazolin-5-yl)amino]-2-(trifluormethyl)-1,2,3,4-tetrahydronaphthalin-2,6-diol

¹H-NMR (300 MHz, CD₃OD); δ = 1.83 (d, 3H), 2.73 (s, 3H), 2.76 (d, 1H), 3.05 (d, 1H), 5.22 (s, 1H), 6.27 (q, 1H), 6.69 (d, 1H), 6.74 (d, 1H), 6.86 (d, 1H), 7.18 (d, 1H), 9.53 (s, 1H).

### Beispiel 40

### (cis,Z)-5-{[2,6-Dihydroxy-4-ethyliden-7-fluor-2-(trifluormethyl)-1,2,3,4-tetrahydronaphthalin-1-yl]amino}-chinolin-2(1H)-on

¹H-NMR (300 MHz, CD₃OD); δ = 1.94 (d, 3H), 2.62 (d, 1H), 2.92 (d, 1H), 4.90 (s, 1H), 5.71 (q, 1H), 6.49 (d, 1H), 6.60 (d. 1H), 6.68 (d, 1H), 6.92 (d, 1H), 7.03 (d, 1H), 7.33 (t, 1H), 8.18 (d, 1H).

### Beispiel 41

### (cis,E)-5-{[2,6-Dihydroxy-4-ethyliden-7-fluor-2-(trifluormethyl)-1,2,3,4-tetrahydronaphthalin-1-yl]amino}-chinolin-2(1H)-on

¹H-NMR (300 MHz, CD₃OD); δ = 1.84 (d, 3H), 2.74 (d, 1 H), 3.03 (d, 1H), 5.06 (s, 1H), 6.24 (q, 1H), 6.50 (d, 1H), 6.57 (d, 1H), 6.69 (d, 1H), 6.87 (d, 1H), 7.17 (d, 1H), 7.35 (t, 1H), 8.20 (d, 1H).

### Beispiel 42

### (cis,E)-7-Chlor-4-ethyliden-1-[(7-fluor-2-methylchinazolin-5-yl)amino]-2-(trifluormethyl)-1,2,3,4-tetrahydronaphthalin-2,6-diol

¹H-NMR (300 MHz, CD₃OD); δ = 1.85 (d, 3H), 2.69 (d, 1H), 2.75 (s, 3H), 3.04 (d, 1H), 5.23 (s, 1H), 6.34 (q, 1H), 6.70 (d, 1H), 6.78 (d, 1H), 7.11 (s, 1H), 7.18 (s, 1H), 9.52 (s, 1H).

### Beispiel 43

### (cis,Z)-7-Chlor-4-ethyliden-1-[(8-fluor-2-methylchinazolin-5-yl)amino]-2-(trifluormethyl)-1,2,3,4-tetrahydronaphthalin-2,6-diol

¹H-NMR (300 MHz, CD₃OD); δ = 1.96 (d, 3H), 2.67 (d, 1H), 2.80 (s, 3H), 2.95 (d, 1H), 5.00 (s, 1H), 5.77 (q, 1H), 6.72 (dd, 1H), 7.07 (s, 1H), 7.21 (s, 1H), 7.51 (dd, 1H), 9.61 (s, 1H).

### Beispiel 44

### (cis, E)-7-Chlor-4-ethyliden-1-[(8-fluor-2-methylchinazolin-5-yl)amino]-2-(trifluormethyl)-1,2,3,4-tetrahydronaphthalin-2,6-diol

¹H-NMR (300 MHz, CD₃OD); δ = 1.83 (d, 3H), 2.75 (d, 1H), 2.80 (s, 3H), 3.01 (d, 1H), 5.12 (s, 1H), 6.31 (q, 1H), 6.78 (dd, 1H), 7.13 (s. 1H), 7.15 (s, 1H), 7.51 (dd, 1H), 9.62 (s, 1 H).

### Beispiel 45

### (cis,Z)-7-Chlor-1-[(7,8-difluor-2-methylchinazolin-5-yl)amino]-4-ethyliden-2-(trifluormethyl)-1,2,3,4-tetrahydronaphthalin-2,6-diol

¹H-NMR (300 MHz, CD₃OD); δ = 1.97 (d, 3H), 2.68 (d, 1H), 2.78 (s, 3H), 2.94 (d, 1H), 5.06 (s, 1H), 5.78 (q, 1H), 6.71 (dd, 1H), 7.09 (s, 1H), 7.19 (s, 1H), 9.55 (s, 1H).

### Beispiel 46

### (cis,E)-7-Chlor-1-[(7,8-difluor-2-methylchinazolin-5-yl)amino]-4-ethyliden-2-(trifluormethyl)-1,2,3,4-tetrahydronaphthalin-2,6-diol

¹H-NMR (300 MHz, CD₃OD); δ = 1.84 (d, 3H), 2.77 (d, 1H), 2.79 (s, 3H), 3.02 (d, 1H), 5.16 (s, 1H), 6.32 (q, 1H), 6.79 (dd, 1H), 7.12 (s, 1H), 7.17 (d, 1H), 9.56 (s, 1H).

### Beispiel 47

### (cis,Z)-5-{[7-Chlor-2,6-dihydroxy-4-ethyliden-2-(trifluormethyl)-1,2,3,4-tetrahydronaphthalin-1-yl]amino}-2-methylphthalazin-1-on

¹H-NMR (300 MHz, CD₃OD); δ = 1.96 (d, 3H), 2.65 (d, 1H), 2.94 (d, 1 H), 3.80 (s, 3H), 4.99 (s, 1H), 5.77 (q, 1H), 7.04 (s, 1H), 7.06 (d, 1H), 7.17 (s, 1H), 7.59 (d, 1H), 7.60 (t, 1H), 8.46 (s, 1H).

### Beispiel 48

### (cis,E)-5-{[7-Chlor-2,6-dihydroxy-4-ethyliden-2-(trifluormethyl)-1,2,3,4-tetrahydronaphthalin-1-yl]amino}-2-methylphthalazin-1-on

¹H-NMR (300 MHz, CD₃OD); δ = 1.82 (d, 3H), 2.72 (d, 1H), 2.99 (d, 1H), 3.79 (s, 3H), 5.12 (s, 1H), 6.32 (q, 1H), 7.06 (s, 1H), 7.13 (s, 1H), 7.14 (d, 1H), 7.60 (m, 2H), 8.50 (s, 1H).

### Beispiel 49

### (cis, E)-5-{[7-Chlor-2,6-dihydroxy-4-ethyliden-2-(trifluormethyl)-1,2,3,4-tetrahydronaphthalin-1-yl]amino}-chinolin-2(1H)-on

¹H-NMR (300 MHz, DMSO-d₆); δ = 1.75 (m, 3H), 2.65 (d, 1H), 2.81 (d. 1H), 5.14 (d, 1H), 6.08 - 6.19 (m. 2H), 6.37 (d, 1H), 6.45 (d, 1H), 6.55 (d, 1H), 6.95 (s, 1H), 7.18 (t, 1H), 7.19 (s, 1H), 8.14 (d, 1H), 10.07 (s, 1H), 11.54 (s, 1H).

### Beispiel 50

### (cis)-2-Fluor-8-isoproyliden-5-[(2-methylchinazolin-5-yl)amino]-6-(trifluormethyl)-5,6,7,8-tetrahydronaphthalin-1,6-diol

¹H-NMR (300 MHz, CDCl₃); δ = 1.86 (s, 3H), 1.94 (s, 3H), 2.23 (d, 1H), 2.73 (s, 3H), 3.62 (d, 1H), 4.53 (d, 1H), 5.46 (d, 1H), 6.34 (d, 1H), 6.80 (dd, 1H), 6.87 (dd, 1H), 7.20 - 7.42 (m, 3H), 8.21 (d, 1H).

### Beispiel 51

### (cis)-2-Fluor-8-isoproyliden-5-[(2-methylchinazolin-5-yl)amino]-6-(trifluormethyl)-5,6,7,8-tetrahydronaphthalin-1,6-diol

¹H-NMR (300 MHz, CD₃OD); δ = 1.83 (s, 3H), 1.95 (s, 3H), 2.22 (d, 1H), 2.83 (s, 3H), 3.55 (d, 1H), 4.55 (s, 1H), 6.45 (d, 1H), 6.77 (dd, 1H), 6.95 (dd, 1H), 7.18 (d, 1H), 7.70 (t, 1H), 9.65 (s, 1H).

### Beispiel 52 (cis)-8-Chlor-9-fluor-3-methyl-1-[(2-methylchinolin-5-yl)amino]-5-(trifluormethyl)-5,6-dihydro-4H-1-oxa-2-boraphenalen-2,5-diol

¹H-NMR (300 MHz, CD₃OD); δ = 2.03 (s, 3H), 2,74 (s, 3H), 3.05 (d, 1H), 3.44 (d, 1H), 5.46 (s, 1H), 6.84 (d, 1H), 7.15 (d, 1H), 7.36 (d, 1H), 7.42 (d, 1H), 7.58 (t, 1H), 8.51 (d, 1H).

## Patentansprüche

1. Stereoisomere der allgemeinen Formel (I), 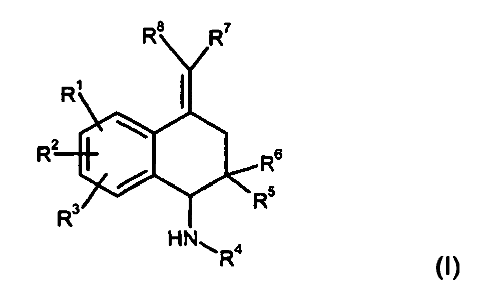 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe
oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus Hydroxygruppen, Halogenatome, (C₁-C₅)-Alkoxygruppen substituierte C₁-C₁₀-Alkylgruppe,
eine gegebenenfalls substituierte (C₃-C₇)-Cycloalkylgruppe,
eine gegebenenfalls substituierte Heterocyclylgruppe,
eine gegebenenfalls substituierte Arylgruppe,
eine gegebenenfalls durch eine oder mehrere Gruppen ausgewählt aus (C₁-C₅)-Alkylgruppen (welche gegebenenfalls substituiert sein können durch 1-3 Hydroxy oder 1-3 COOR¹²-Gruppen), (C₁-C₅)-Alkoxygruppen, Hydroxygruppen, Halogenatomen, 1-2 (C₁-C₃)-Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R¹² eine (C₁-C₅)-Alkyl- oder eine Benzyl-Gruppe bedeutet,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe,
(C₃-C₇)Cycloalkyl(C₂-C₈)alkenylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C2-C₈)alkinylgruppe, eine gegebenenfalls durch eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe, eine Heteroaryl(C₁-C₈)alkylgruppe oder eine
Heteroaryl(C₂-C₈)alkenylgruppe
wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann,
oder gemeinsam mit dem Kohlenstoffatom der Methylengruppe einen (C₃-C₆)-Cycloalkylring, oder
R¹ und R⁸ gemeinsam einen annelierten fünf- bis achtgliedrigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-2 Hydroxygruppen, 1-4 Halogenatome substituiert ist,
bedeuten.

2. Stereoisomere der allgemeinen Formel I, worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus Hydroxygruppen, Halogenatome, (C₁-C₅)-Alkoxygruppen substituierte C₁-C₁₀-Alkylgruppe,
eine gegebenenfalls substituierte (C₃-C₇)-Cycloalkylgruppe,
eine gegebenenfalls substituierte Heterocyclylgruppe,
eine gegebenenfalls substituierte Arylgruppe,
eine gegebenenfalls durch eine oder mehrere Gruppen ausgewählt aus (C₁-C₅)-Alkylgruppen (welche gegebenenfalls substituiert sein können durch 1-3 Hydroxy oder 1-3 COOR¹²-Gruppen), (C₁-C₅)-Alkoxygruppen, Hydroxygruppen, Halogenatomen, 1-2 (C₁-C₃)-Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R¹² eine (C₁-C₅)-Alkyl- oder eine Benzyl-Gruppe bedeutet
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe,
(C₃-C₇)Cycloalkyl(C₂-C₈)alkenylgruppe , eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinylgruppe, eine gegebenenfalls durch eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe, eine Heteroaryl(C₁-C₈)alkylgruppe oder eine
Heteroaryl(C₂-C₈)alkenylgruppe
wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann,
oder gemeinsam mit dem Kohlenstoffatom der Methylengruppe einen (C₃-C₆)-Cycloalkylring, oder
R¹ und R⁸ gemeinsam einen annelierten fünf- bis achtgliedrigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-2 Hydroxygruppen 1-4 Halogenatome substituiert ist,
bedeuten.

3. Stereoisomere der allgemeinen Formel I gemäß Anspruch 1, worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR⁶-Gruppen), (C₁-C₅)-Alkoxygruppen, Hydroxygruppen, Halogenatome, (C₁-C₃)Exoalkylidengruppen substituierte, gegebenenfalls 1-4 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinyl-gruppe eine gegebenenfalls durch eine oder mehrere Ketogruppen, (C₁-C₅)-Alkylgruppen, (C₁-C₅)-Alkoxygruppen, Halogenatome, (C₁-C₃)Ecoalkylidengruppen substituierte, ein oder mehrere Stickstoffatome und/oder Sauerstoffatome und/oder Schwefelatome enthaltende mono-oder bizyklische Heteroarylgruppe,
eine Heteroaryl(C₁-C₈)alkylgruppe oder eine
Heteroaryl(C₂-C₈)alkenylgruppe
wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann,
oder gemeinsam mit dem Kohlenstoffatom der Methylengruppe einen (C₃-C₆)-Cycloalkylring, oder
R¹ und R⁸ gemeinsam einen annelierten fünf- bis achtgliedrigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist, bedeuten.

4. Stereoisomere der allgemeinen Formel (I) gemäß Anspruch 1, worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkythiogruppe, eine (C₁-C₅)-Perfluoralkyl gruppe, eine Cyanogruppe, eine Nitrogruppe
oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, wobei n = 1 oder 2 ist und die endständigen Atome mit direkt benachbarten Ring-Kohlenstoffatomen verknüpft sind,
oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁴ eine C₁-C₁₀-Alkylgruppe, eine durch eine oder mehrere Gruppen ausgewählt aus 1-3 Hydroxygruppen, Halogenatome, 1-3 (C₁-C₅)-Alkoxygruppen substituierte C₁-C₁₀-Alkylgruppe, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Hydroxygruppen, 1-3 Halogenatome, 1-2 (C₁-C₃)-Exoalkylidengruppen substituierte 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppen über eine beliebige Position mit dem Amin des Tetrahydronaphthalinsystems verknüpft sein und gegebenenfalls an einer oder mehreren Stellen hydriert sein können
R⁵ eine Hydroxygruppe
R⁶ eine (C₁-C₅)-Alkylgruppe oder eine gegebenenfalls teilweise oder vollständig fluorierte (C₁-C₅)-Alkylgruppe, eine Arylgruppe, eine Aryl(C₁-C₈)alkylgruppe, Aryl(C₂-C₈)alkenylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe
R⁷ und R⁸ unabhängig voneinander ein Wasserstoffatom, ein Halogenatom, eine Methyl- oder Ethylgruppe, die mit OR¹⁰, SR¹⁰, N(R¹⁰)₂ substituiert sein dürfen, oder gemeinsam mit dem Kohlenstoffatom der Methylengruppe einen (C₃-C₆)-Cycloalkylring oder
R¹ und R⁸ gemeinsam einen annelierten fünf- bis achtgliedrigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist,
bedeuten.

5. Stereoisomere der allgemeinen Formel (I), gemäß Anspruch 1, worin
R¹ und R² unabhängig voneinander ein Wasserstoffatom, eine Hydroxy-Gruppe, ein Halogenatom, eine gegebenenfalls substituierte (C₁-C₅)-Alkylgruppe, eine (C₁-C₅)-Alkoxygruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Atome mit direkt benachbarten Ring-Kohlenstoffatomen verknüpft sind,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe,
R⁴ eine C₁-C₁₀-Alkylgruppe, eine durch eine oder mehrere Gruppen ausgewählt aus 1-3 Hydroxygruppen oder Halogenatome substituierte C₁-C₁₀-Alkylgruppe, eine gegebenenfalls durch eine oder mehrere Gruppen ausgewählt aus 1-2 Ketogruppen, 1-2-(C₁-C₅)-Alkylgruppen, 1-2-(C₁-C₅)-Alkoxygruppen, 1-3 Hydroxygruppen, 1-3 Halogenatome, 1-2 (C₁-C₃)-Exoalkylidengruppen substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazol- oder Indolylgruppe,
wobei diese Gruppen über eine beliebige Position mit dem Amin des Tetrahydronaphthalinsystems verknüpft sein können und gegebenenfalls an einer oder mehreren Stellen hydriert sein können
R⁵ eine Hydroxygruppe
R⁶ eine (C₁-C₅)-Alkylgruppe oder eine gegebenenfalls teilweise oder vollständig fluorierte (C₁-C₅)-Alkylgruppe
R⁷ und R⁸ unabhängig voneinander ein Wasserstoffatom, ein Halogenatom, eine Methyl- oder Ethylgruppe, die mit OR¹⁰, SR¹⁰, N(R⁹R¹⁰) substituiert sein dürfen, oder gemeinsam mit dem Kohlenstoffatom der Methylengruppe einen (C₃-C₆)-Cycloalkylring oder
R¹ und R⁸ gemeinsam einen annelierten fünf- bis achtgliedrigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-4 Halogenatome substituiert ist,
bedeuten.

6. Stereoisomere der allgemeinen Formel (I) gemäß Anspruch 1,
worin
R¹ und R² unabhängig voneinander ein Wasserstoffatom, eine Hydroxy-Gruppe, ein Halogenatom, eine (C₁-C₅)-Alkylgruppe, eine (C₁-C₅)-Alkoxygruppe, oder zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Atome mit direkt benachbarten Ring-Kohlenstoffatomen verknüpft sind,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe,
R⁴ eine gegebenenfalls durch eine oder mehrere Gruppen ausgewählt aus 1-2 Ketogruppen, 1-2-(C₁-C₅)Alkylgruppen, 1-2-(C₁-C₅)-Alkoxygruppen, 1-3 Hydroxygruppen, 1-3 Halogenatome, 1-2 (C₁-C₃)-Exoalkylidengruppen substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl-, Dihydroisoindolonyl-, Benzimidazol- oder Indolylgruppe,
wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine (C₁-C₅)-Alkylgruppe oder eine gegebenenfalls teilweise oder vollständig fluorierte (C₁-C₅)-Alkylgruppe
R⁷ und R⁸ unabhängig voneinander ein Wasserstoffatom, ein Halogenatom, eine Methyl-oder Ethylgruppe, oder gemeinsam mit dem Kohlenstoffatom der Methylengruppe einen (C₃-C₆)-Cycloalkylring oder
R¹ und R⁸ gemeinsam einen annelierten fünf- bis achtgliedrigen gesättigten oder ungesättigten Carbo- oder Heterozyklus,
bedeuten.

7. Stereoisomere der allgemeinen Formel (I) gemäß Anspruch 1, worin
R¹ und R² unabhängig voneinander ein Wasserstoffatom, eine Hydroxy-Gruppe, ein Halogenatom, eine (C₁-C₅)-Alkoxygruppe,
R³ ein Wasserstoffatom, ein Halogenatom,
R⁴ eine gegebenenfalls mit C₁-C₅-Alkyl, Halogen, Ketogruppen, substituierte Chinolinyl-, Chinolonyl-, Phthalazinyl-, Phthalazinonyl-, Chinazolinyl- oder Chinazolonylgruppe
wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine gegebenenfalls teilweise oder vollständig fluorierte (C₁-C₅)-Alkylgruppe
R⁷ und R⁸ unabhängig voneinander ein Wasserstoffatom, eine Methyl- oder Ethylgruppe, oder
R¹ und R⁸ gemeinsam einen annelierten fünf- bis achtgliedrigen gesättigten oder ungesättigten Carbo- oder Heterozyklus, der gegebenenfalls durch
eine Hydroxygruppe substituiert ist, bedeuten.

8. Stereoisomere der allgemeinen Formel (I) gemäß Anspruch 1, worin
R¹ und R² unabhängig voneinander ein Wasserstoffatom, eine Hydroxy-Gruppe, ein Fluor- oder Chloratom, eine Methoxygruppe,
R³ ein Wasserstoffatom oder ein Chloratom,
R⁴ eine gegebenenfalls mit einer oder mehren Gruppen ausgewählt aus Methyl-, Hydroxy-, Ketogruppe oder Fluoratom, substituierte Chinolinyl-, Chinolonyl-, Chinazolinyl- oder Phtalazinonylgruppe
wobei diese Gruppen über eine beliebige Position mit dem Amin des Tetrahydronaphthalinsystems verknüpft sein können und gegebenenfalls an einer oder mehreren Stellen hydriert sein können,
R⁵ eine Hydroxygruppe
R⁶ eine Trifluormethylgruppe
R⁷ und R⁸ unabhängig voneinander ein Wasserstoffatom, eine Methyl- oder Ethylgruppe, oder
R¹ und R⁸ gemeinsam einen annelierten sechsgliedrigen Heterozyklus,
bedeuten, der gegebenenfalls durch eine Hydroxygruppe substituiert ist.

9. Stereoisomere der allgemeinen Formel (I) gemäß Anspruch 1, worin R¹ und R⁸ gemeinsam einen annelierten sechsgliedrigen Heterozyklus; bedeuten, der ein Sauerstoffatom und ein Boratom enthält und der gegebenenfalls durch eine Hydroxygruppe substituiert ist.

10. Verwendung der Stereoisomere gemäß einem der vorhergehenden Ansprüche zur Herstellung eines Arzneimittels.

11. Verwendung der Stereoisomere der Ansprüche 1-4 zur Herstellung eines Arzneimittels für die Behandlung von entzündlichen Erkrankungen

12. Pharmazeutische Präparate enthaltend mindestens ein Stereoisomeres nach Anspruch 1-4 oder deren Gemische sowie pharmazeutisch verträgliche Träger.

13. Verfahren zur Herstellung der Stereoisomere der allgemeinen Formel I, worin die Reste, wenn nicht anders erwähnt, die in Anspruch 1 definierten Bedeutungen haben, **dadurch gekennzeichnet, dass** Stereoisomere der allgemeinen Formel (III) 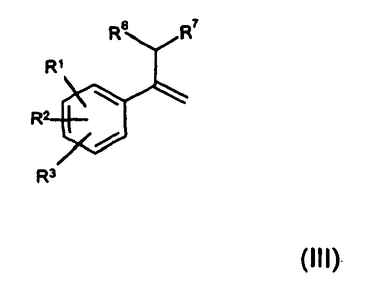 worin R¹, R², R³, R⁷ und R⁸ die in Anspruch 1 genannten Bedeutungen haben, durch eine gegebenenfalls enantioselektiv geführte En-Reaktion mit α-Ketosäuren R⁶(CO)COOR¹² mit R¹² in der Bedeutung von (C₁-C₅)-Alkyl, Benzyl, in Gegenwart von gegebenenfalls chiralen Lewissäuren in Verbindungen der allgemeinen Formel (IV) 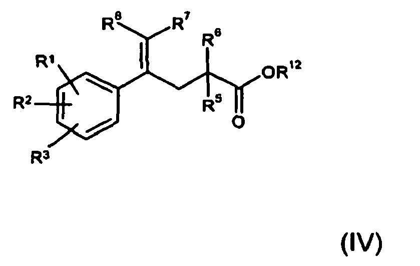 überführt werden, wobei R⁵ eine Hydroxygruppe bedeutet, die gegebenenfalls gemäß der anderen für R⁵ in Anspruch 1 definierten Bedeutungen in eine Schutzgruppe überführt werden kann,
durch Reduktion und Umsetzung mit Aminen der Formel R⁴-NH₂,
wobei R⁴ die in Anspruch 1 angegebenen Bedeutung hat,
die Verbindungen der allgemeinen Formel (V) hergestellt werden 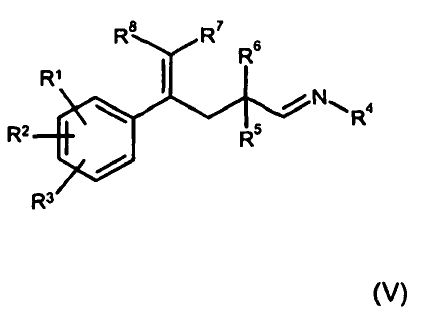 worin R¹, R², R³, R⁴, R⁵, R⁶, R⁷ und R⁸ die in Anspruch 1 angegebenen Bedeutungen haben, dann entweder ohne weiteres Reagenz oder durch Zusetzen von anorganischen oder organischen Säuren oder Lewissäuren bei Temperaturen von -70°C bis 80°C zu Verbindungen der allgmeinen Formel (I) zyklisiert werden.

14. Verfahren zur Herstellung der Stereoisomere der allgemeinen Formel I, **dadurch gekennzeichnet, dass** Stereoisomere der allgemeinen Formel V 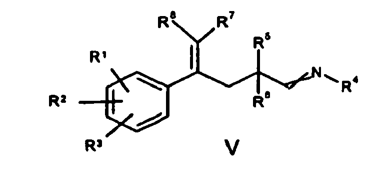 worin die Reste R¹, R², R³, R⁴, R⁵, R⁶, R⁷ und R⁸ die in Anspruch 1 angegebenen Bedeutungen haben, gegebenenfalls unter Zugabe von anorganischen oder organischen Säuren oder Lewissäuren zyklisiert werden oder **dadurch gekennzeichnet, dass** Stereoisomere der allgemeinen Formel II 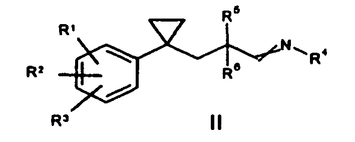 worin die Reste R¹, R², R³, R⁴, R⁵, und R⁶ die in Anspruch 1 angegebenen Bedeutungen haben, gegebenenfalls unter Zugabe von anorganischen oder organischen Säuren oder Lewissäuren zyklisiert und umgelagert werden.

15. Verfahren zur Herstellung der Stereoisomere der allgemeinen Formel I gemäß Anspruch 13, **dadurch gekennzeichnet, dass** Stereoisomere der allgemeinen Formel (III) 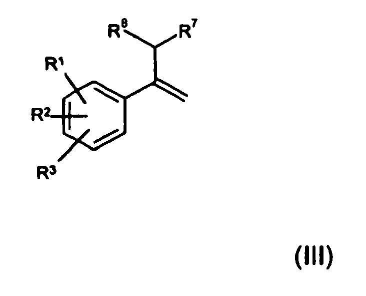 worin R¹, R², R³, R⁷ und R⁸ die in Anspruch 1 genannten Bedeutungen haben, durch eine gegebenenfalls enantioselektiv geführte En-Reaktion mit α-Ketosäuren R⁶(CO)COOR¹² mit R¹² in der Bedeutung von (C₁-C₅)-Alkyl, Benzyl, in Gegenwart von gegebenenfalls chiralen Lewissäuren in Verbindungen der allgemeinen Formel (IV) 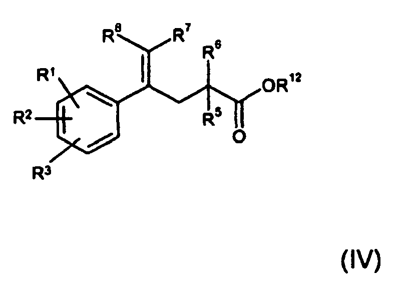 überführt werden, wobei R⁵ eine Hydroxygruppe bedeutet, die gegebenenfalls gemäß der anderen für R⁵ in Anspruch 1 definierten Bedeutungen in eine Schutzgruppe überführt werden kann.

16. Stereoisomere der Formel IV 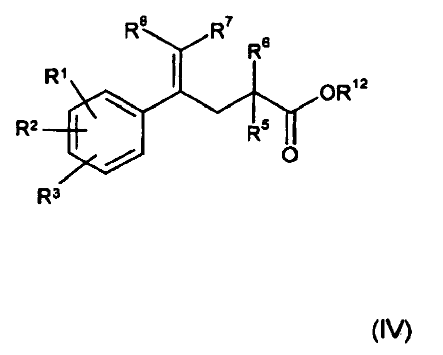 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe, eine Nitrogruppe oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂,-NH-(CH₂)ₙ₊₁, N(C₁-C₃-alkyl)-(CH₂)ₙ₊₁, -NH-N=CH-, wobei n = 1 oder 2 ist und die endständigen Atome mit direkt benachbarten Ring-Kohlenstoffatomen verknüpft sind,
oder NR⁹R¹⁰, wobei R⁹ und R¹⁰ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe, eine Cyanogruppe,
R⁵ eine Hydroxygruppe, eine Gruppe OR¹¹ oder eine O-(CO)R¹¹ -Gruppe, wobei R¹¹ eine beliebige Hydroxyschutzgruppe oder eine C₁-C₁₀-Alkylgruppe bedeutet,
R⁶ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₃-C₇)Cycloalkyl(C₁-C₈)alkylgruppe, (C₃-C₇)Cycloalkyl(C₂-C₈)alkenylgruppe, eine Heterocyclylgruppe, eine Heterocyclyl(C₁-C₈)alkylgruppe, Heterocyclyl(C₂-C₈)alkenylgruppe, eine Arylgruppe, eine Aryl(C₁-C₈)alkylgruppe, Aryl(C₂-C₈)alkenylgruppe, Aryl(C₂-C₈)alkinylgruppe, eine gegebenenfalls durch eine oder mehrere Ketogruppen, (C₁-C₅)-Alkylgruppen, (C₁-C₅)-Alkoxygruppen, Halogenatome, (C₁-C₃)Exoalkylidengruppen substituierte, ein oder mehrere Stickstoffatome und/oder Sauerstoffatome und/oder Schwefelatome enthaltende mono- oder bizyklische Heteroarylgruppe, eine Heteroaryl(C₁-C₈)alkylgruppe oder eine Heteroaryl(C₂-C₈)alkenylgruppe
wobei diese Gruppen über eine beliebige Position mit dem Tetrahydronaphthalinsystem verknüpft sein können und gegebenenfalls an einer oder mehreren Stellen hydriert sein können,
R⁷ und R⁸ unabhängig voneinander ein Wasserstoffatom, ein Halogenatom, eine (C₁-C₅)Alkylgruppe, die mit OR¹⁰, SR¹⁰, N(R⁹R¹⁰) substituiert sein kann,
oder gemeinsam mit dem Kohlenstoffatom der Methylengruppe einen (C₃-C₆)-Cycloalkylring, oder
R¹ und R⁸ gemeinsam einen annelierten fünf- bis achtglierigen gesättigten oder ungesättigten Carbo- oder Heterozyklus, der gegebenenfalls durch 1-2 Ketogruppen, 1-2-(C₁-C₅)-Alkylgruppen, 1-2-(C₁-C₅)-Alkoxygruppen, 1-2 Hydroxygruppen, 1-4 Halogenatome substituiert ist,
bedeuten,
wobei mindestens einer der Reste R⁷ und R⁸ kein Wasserstoffatom ist und R¹² (C₁-C₅)-Alkyl oder Benzyl bedeutet.

17. Verfahren zur Herstellung der Stereoisomere der allgemeinen Formel I gemäß Anspruch 12, **dadurch gekennzeichnet, dass** Stereoisomere der allgemeinen Formel (IV) 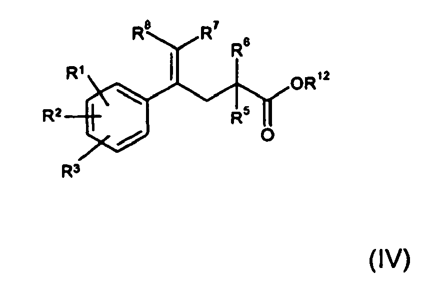 wobei R⁵ eine Hydroxygruppe bedeutet, die gegebenenfalls gemäß der anderen für R⁵ in Anspruch 1 definierten Bedeutungen in eine Schutzgruppe überführt werden kann, die Reste R¹, R², R³, R⁶, R⁷ und R⁸ die in Anspruch 1 angegebenen Bedeutungen haben und R¹³ (C₁-C₅)-Alkyl, Benzyl bedeutet, durch Reduktion und Umsetzung mit Aminen der Formel R⁴-NH₂,
wobei R⁴ die in Anspruch 1 angegebenen Bedeutung hat,
in Verbindungen der allgemeinen Formel (V) überführt werden, 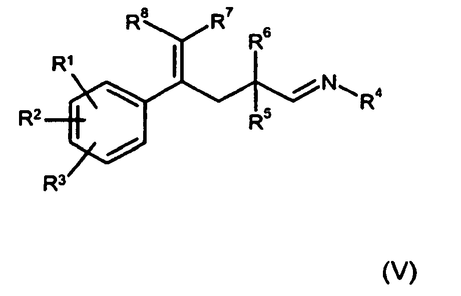 worin R¹, R², R³, R⁴, R⁵, R⁶, R⁷ und R⁸ die in Anspruch 1 angegebenen Bedeutungen haben.

18. Stereoisomere der Formel V gemäß Anspruch 17, 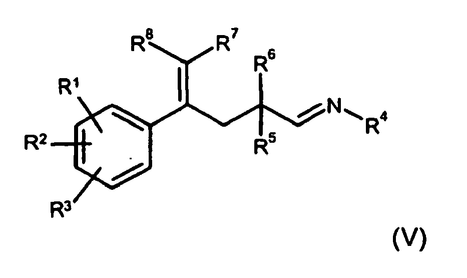 worin R¹, R², R³, R⁴, R⁵, R⁶, R⁷ und R⁸ die in Anspruch 1 angegebenen Bedeutungen haben.

19. Stereoisomere der allgemeinen Formel I, gemäß einem der Ansprüche 1-9, 16 und 18 in Form der Salze mit physiologisch verträglichen Anionen.

## Claims

1. Stereoisomers of general formula (I), 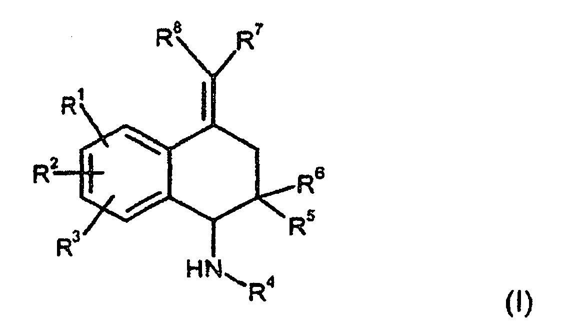 in which
R¹ and R², independently of one another, mean a hydrogen atom, a hydroxy group, a halogen atom, an optionally substituted (C₁-C₁₀)-alkyl group, (C₁-C₁₀)-alkoxy group, a (C₁-C₁₀)-alkylthio group, a (C₁-C₅)-perfluoroalkyl group, a cyano group, or a nitro group or
R¹ and R² together mean a group that is selected from the groups -O-(CH₂)ₙ-O-, -O- (CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁ and -NH-N=CH-, where n = 1 or 2, and the terminal atoms are linked to directly adjacent ring carbon atoms, or NR⁹R¹⁰, where R⁹ and R¹⁰, independently of one another, can be hydrogen, C₁-C₅-alkyl or (CO)-C₁-C₅-alkyl,
R³ means a hydrogen atom, a hydroxy group, a halogen atom, an optionally substituted (C₁-C₁₀)-alkyl group, a (C₁-C₁₀)-alkoxy group, a (C₁-C₁₀)-alkylthio group, a (C₁-C₅)-perfluoroalkyl group, or a cyano group,
R⁴ means a C₁-C₁₀-alkyl group that is substituted by one or more groups selected from hydroxy groups, halogen atoms, and (C₁-C₅)-alkoxy groups,
an optionally substituted (C₃-C₇)-cycloalkyl group,
an optionally substituted heterocyclyl group,
an optionally substituted aryl group, a monocyclic or bicyclic heteroaryl group that optionally is substituted by one or more groups selected from (C₁-C₅)-alkyl groups (which optionally can be substituted by 1-3 hydroxy or 1-3 COOR¹² groups), (C₁-C₅) -alkoxy groups, hydroxy groups, halogen atoms, or 1-2 (C₁-C₃)-exoalkylidene groups and that optionally contains 1-4 nitrogen atoms and/or 1-2 oxygen atoms and/or 1-2 sulphur atoms and/or 1-2 keto groups, where this heteroaryl group can be linked to the amine of the tetrahydronaphthalene system via any position and optionally can be hydrogenated at one or more sites, and R¹² means a (C₁-C₅)-alkyl group or a benzyl group,
R⁵ means a hydroxy group, a group OR¹¹ or an O-(CO)R¹¹ group, where R¹¹ means any hydroxy-protective group or a C₁-C₁₀-alkyl group,
R⁶ means a (C₁-C₅)-alkyl group or an optionally partially or completely fluorinated (C₁-C₅)-alkyl group, a (C₃-C₇)cycloalkyl group, a (C₃-C₇)cycloalkyl(C₁-C₈)alkyl group, a (C₃-C₇)cycloalkyl(C₂-C₈)alkenyl group, a heterocyclyl group, a heterocyclyl(C₁-C₈)alkyl group, a heterocyclyl(C₂-C₈)alkenyl group, an aryl group, an aryl(C₁-C₈)alkyl group, an aryl(C₂-C₈)alkenyl group, an aryl (C₂-C₈) alkynyl group; a monocyclic or bicyclic heteroaryl group that optionally is substituted by one or more keto groups, (C₁-C₅)-alkyl groups, (C₁-C₅)-alkoxy groups, halogen atoms, or (C₁-C₃)-exoalkylidene groups and that contains one or more nitrogen atoms and/or oxygen atoms and/or sulphur atoms; a heteroaryl(C₁-C₈)alkyl group or a heteroaryl(C₂-C₈)alkenyl group, where these groups can be linked to the tetrahydronaphthalene system via any position and optionally can be hydrogenated at one or more sites,
R⁷ and R⁸ independently of one another, mean a hydrogen atom, a halogen atom, a (C₁-C₅)-alkyl group, which can be substituted with OR¹⁰, SR¹⁰, or N(R⁹R¹⁰),
or together with the carbon atom of the methylene group mean a (C₃-C₆)-cycloalkyl ring, or
R¹ and R⁸ together mean an annelated five- to eight-membered saturated or unsaturated carbocycle or heterocycle, which optionally is substituted by 1-2 keto groups, 1-2 (C₁-C₅)-alkyl groups, 1-2 (C₁-C₅)-alkoxy groups, 1-2 hydroxy groups, or 1-4 halogen atoms.

2. Stereoisomers of general formula I, in which
R¹ and R², independently of one another, mean a hydrogen atom, a hydroxy group, a halogen atom, an optionally substituted (C₁-C₁₀)-alkyl group, (C₁-C₁₀)-alkoxy group, a (C₁-C₁₀)-alkylthio group, a (C₁-C₅)-perfluoroalkyl group, a cyano group, or a nitro group or
R¹ and R² together mean a group that is selected from the groups -O-(CH₂)ₙ-O-, -O-(CH₂)ₙ-CH₂-, -O-CH=CH-, - (CH₂)ₙ₊₂-, -NH-(CH₂)ₙ₊₁, N(C₁-C₃-alkyl)-(CH₂)ₙ₊₁ and -NH-N=CH-, where n = 1 or 2, and the terminal atoms are linked to directly adjacent ring carbon atoms, or NR⁹R¹⁰, where R⁹ and R¹⁰, independently of one another, can be hydrogen, C₁-C₅-alkyl or (CO) -C₁-C₅-alkyl ,
R³ means a hydrogen atom, a hydroxy group, a halogen atom, an optionally substituted (C₁-C₁₀)-alkyl group, a (C₁-C₁₀)-alkoxy group, a (C₁-C₁₀)-alkylthio group, a (C₁-C₅)-perfluoroalkyl group, or a cyano group,
R⁴ means a C₁-C₁₀-alkyl group that is substituted by one or more groups selected from hydroxy groups, halogen atoms, and (C₁-C₅)-alkoxy groups,
an optionally substituted (C₃-C₇)-cycloalkyl group,
an optionally substituted heterocyclyl group,
an optionally substituted aryl group, a monocyclic or bicyclic heteroaryl group that optionally is substituted by one or more groups selected from (C₁-C₅)-alkyl groups (which optionally can be substituted by 1-3 hydroxy or 1-3 COOR¹² groups), (C₁-C₅)-alkoxy groups, hydroxy groups, halogen atoms, or 1-2 (C₁-C₃)-exoalkylidene groups and that optionally contains 1-4 nitrogen atoms and/or 1-2 oxygen atoms and/or 1-2 sulphur atoms and/or 1-2 keto groups, where this heteroaryl group can be linked to the amine of the tetrahydronaphthalene system via any position and optionally can be hydrogenated at one or more sites, and R¹² means a (C₁-C₅) -alkyl group or a benzyl group,
R⁵ means a hydroxy group, a group OR¹¹ or an O- (CO) R¹¹ group, where R¹¹ means any hydroxy-protective group or a C₁-C₁₀-alkyl group,
R⁶ means a (C₁-C₅) -alkyl group or an optionally partially or completely fluorinated (C₁-C₅)-alkyl group, a (C₃-C₇)-cycloalkyl group, a (C₃-C₇) cycloalkyl (C₁-C₈) alkyl group, a (C₃-C₇)cycloalkyl(C₂-C₈)alkenyl group, a heterocyclyl group, a heterocyclyl(C₁-C₈)alkyl group, a heterocyclyl(C₂-C₈)alkenyl group, an aryl group, an aryl (C₁-C₈) alkyl group, an aryl (C₂-C₈) alkenyl group, an aryl (C₂-C₈) alkynyl group; a monocyclic or bicyclic heteroaryl group that optionally is substituted by one or more keto groups, (C₁-C₅)-alkyl groups, (C₁-C₅)-alkoxy groups, halogen atoms, or (C₁-C₃)-exoalkylidene groups and that contains one or more nitrogen atoms and/or oxygen atoms and/or sulphur atoms; a heteroaryl(C₁-C₈)alkyl group or a heteroaryl(C₂-C₈)alkenyl group, where these groups can be linked to the tetrahydronaphthalene system via any position and optionally can be hydrogenated at one or more sites,
R⁷ and R⁸, independently of one another, mean a hydrogen atom, a halogen atom, a (C₁-C₅)-alkyl group, which can be substituted with OR¹⁰, SR¹⁰, or N(R⁹R¹⁰),
or together with the carbon atom of the methylene group mean a (C₃-C₆)-cycloalkyl ring, or
R¹ and R⁸ together mean an annelated five- to eight-membered saturated or unsaturated carbocycle or heterocycle, which optionally is substituted by 1-2 keto groups, 1-2 (C₁-C₅)-alkyl groups, 1-2 (C₁-C₅)-alkoxy groups, 1-2 hydroxy groups, or 1-4 halogen atoms.

3. Stereoisomers of general formula I according to Claim 1, in which
R¹ and R², independently of one another, mean a hydrogen atom, a hydroxy group, a halogen atom, an optionally substituted (C₁-C₁₀)-alkyl group, (C₁-C₁₀)-alkoxy group, a (C₁-C₁₀)-alkylthio group, a (C₁-C₅)-perfluoroalkyl group, a cyano group, or a nitro group or
R¹ and R² together mean a group that is selected from the groups -O-(CH₂)ₙ-O-, -O-(CH₂)ₙ-CH₂-, -O-CH=CH-, - (CH₂)ₙ₊₂-, -NH- (CH₂)ₙ₊₁, N(C₁-C₃-alkyl)-(CH₂)ₙ₊₁ and -NH-N=CH-, where n = 1 or 2, and the terminal atoms are linked to directly adjacent ring carbon atoms, or NR⁹R¹⁰, where R⁹ and R¹⁰, independently of one another, can be hydrogen, C₁-C₅-alkyl or (CO)-C₁-C₅-alkyl,
R³ means a hydrogen atom, a hydroxy group, a halogen atom, an optionally substituted (C₁-C₁₀)-alkyl group, a (C₁-C₁₀)-alkoxy group, a (C₁-C₁₀) -alkylthio group, a (C₁-C₅) -perfluoroalkyl group, or a cyano group,
R⁴ means a C₁-C₁₀-alkyl group that is substituted by one or more groups selected from 1-3 hydroxy groups, halogen atoms, and 1-3 (C₁-C₅)-alkoxy groups,
an optionally substituted (C₃-C₇)-cycloalkyl group,
an optionally substituted heterocyclyl group,
an optionally substituted aryl group; a monocyclic or bicyclic heteroaryl group that optionally is substituted by one or more groups selected from (C₁-C₅)-alkyl groups (which optionally can be substituted by 1-3 hydroxy or 1-3 COOR⁶ groups), (C₁-C₅)-alkoxy groups, hydroxy groups, halogen atoms, or (C₁-C₃)-exo-alkylidene groups and that optionally contains 1-4 nitrogen atoms and/or 1-2 oxygen atoms and/or 1-2 sulphur atoms and/or 1-2 keto groups, where this heteroaryl group can be linked to the amine of the tetrahydronaphthalene system via any position and optionally can be hydrogenated at one or more sites,
R⁵ means a hydroxy group, a group OR¹¹ or an O- (CO) R¹¹ group, where R¹¹ means any hydroxy-protective group or a C₁-C₁₀-alkyl group,
R⁶ means a (C₁-C₅)-alkyl group or an optionally partially or completely fluorinated (C₁-C₅) -alkyl group, a (C₃-C₇)-cycloalkyl group, a (C₃-C₇)cycloalkyl(C₁-C₈)alkyl group, a (C₃-C₇)cycloalkyl(C₂-C₈)alkenyl group, a heterocyclyl group, a heterocyclyl(C₁-C₈)alkyl group, a heterocyclyl(C₂-C₈)alkenyl group, an aryl group, an aryl(C₁-C₈)alkyl group, an aryl(C₂-C₈)alkenyl group, an aryl(C₂-C₈)alkynyl group; a monocyclic or bicyclic heteroaryl group that optionally is substituted by one or more keto groups, (C₁-C₅) -alkyl groups, (C₁-C₅)-alkoxy groups, halogen atoms, or (C₁-C₃)-exoalkylidene groups and that contains one or more nitrogen atoms and/or oxygen atoms and/or sulphur atoms,
a heteroaryl (C₁-C₈)alkyl group or a heteroaryl (C₂-C₈) alkenyl group, where these groups can be linked to the tetrahydronaphthalene system via any position and optionally can be hydrogenated at one or more sites,
R⁷ and R⁸, independently of one another, mean a hydrogen atom, a halogen atom, a (C₁-C₅)-alkyl group, which can be substituted with OR¹⁰, SR¹⁰, or N(R⁹R¹⁰),
or together with the carbon atom of the methylene group mean a (C₃-C₆)-cycloalkyl ring, or
R¹ and R⁸ together mean an annelated five- to eight-membered saturated or unsaturated carbocycle or heterocycle, which optionally is substituted by 1-2 keto groups, 1-2 (C₁-C₅)-alkyl groups, 1-2 (C₁-C₅)-alkoxy groups, or 1-4 halogen atoms.

4. Stereoisomers of general formula (I) according to Claim 1, in which
R¹ and R², independently of one another, mean a hydrogen atom, a hydroxy group, a halogen atom, an optionally substituted (C₁-C₁₀)-alkyl group, (C₁-C₁₀)-alkoxy group, a (C₁-C₁₀)-alkylthio group, a (C₁-C₅)-perfluoroalkyl group, a cyano group, or a nitro group or
R¹ and R² together mean a group that is selected from the groups -O-(CH₂)ₙ-O-, -O-(CH₂)ₙ-CH₂-, -O-CH=CH-, or -(CH₂)ₙ₊₂-, where n = 1 or 2, and the terminal atoms are linked to directly adjacent ring carbon atoms, or NR⁹R¹⁰, where R⁹ and R¹⁰, independently of one another, can be hydrogen, C₁-C₅-alkyl or (CO)-C₁-C₅-alkyl,
R³ means a hydrogen atom, a hydroxy group, a halogen atom, an optionally substituted (C₁-C₁₀)-alkyl group, a (C₁-C₁₀)-alkoxy group, a (C₁-C₁₀) -alkylthio group, a (C₁-C₅)-perfluoroalkyl group, or a cyano group,
R⁴ means a C₁-C₁₀-alkyl group that is substituted by one or more groups selected from 1-3 hydroxy groups, halogen atoms, and 1-3 (C₁-C₅)-alkoxy groups,
an optionally substituted phenyl group,
an optionally substituted heterocyclyl group,
a monocyclic or bicyclic heteroaryl group that optionally is substituted by 1-2 keto groups, 1-2 (C₁-C₅)-alkyl groups, 1-2 (C₁-C₅)-alkoxy groups, 1-3 hydroxy groups, 1-3 halogen atoms, or 1-2 (C₁-C₃)-exoalkylidene groups and that contains 1-4 nitrogen atoms and/or 1-2 oxygen atoms and/or 1-2 sulphur atoms and/or 1-2 keto groups, where these groups can be linked to the amine of the tetrahydronaphthalene system via any position and optionally can be hydrogenated at one or more sites,
R⁵ means a hydroxy group,
R⁶ means a (C₁-C₅)-alkyl group or an optionally partially or completely fluorinated (C₁-C₅)-alkyl group, an aryl group, an aryl(C₁-C₈)alkyl group, an aryl (C₂-C₈) alkenyl group, a (C₃-C₇)-cycloalkyl group, a (C₃-C₇)cycloalkyl(C₁-C₈)alkyl group, or a (C₃-C₇)cycloalkyl(C₂-C₈)alkenyl group,
R⁷ and R⁸, independently of one another, mean a hydrogen atom, a halogen atom, a methyl or ethyl group, which should be substituted with OR¹⁰, SR¹⁰, or N(R¹⁰)₂, or together with the carbon atom of the methylene group mean a (C₃-C₆)-cycloalkyl ring, or
R¹ and R⁸ together mean an annelated five- to eight-membered saturated or unsaturated carbocycle or heterocycle, which optionally is substituted by 1-2 keto groups, 1-2 (C₁-C₅) - alkyl groups, 1-2 (C₁-C₅)-alkoxy groups, or 1-4 halogen atoms.

5. Stereoisomers of general formula (I), according to Claim 1, in which
R¹ and R², independently of one another, mean a hydrogen atom, a hydroxy group, a halogen atom, an optionally substituted (C₁-C₅)-alkyl group, or a (C₁-C₅)-alkoxy group, or
R¹ and R² together mean a group that is selected from the groups -O-(CH₂)ₙ-O-, -O-(CH₂)ₙ-CH₂-, -O-CH=CH-, or -(CH₂)ₙ₊₂-, where n = 1 or 2, and the terminal atoms are linked to directly adjacent ring carbon atoms,
R³ is a hydrogen atom, a hydroxy group, a halogen atom, an optionally substituted (C₁-C₁₀)-alkyl group, or a (C₁-C₁₀)-alkoxy group,
R⁴ is a C₁-C₁₀-alkyl group, a C₁-C₁₀-alkyl groups substituted by one or more groups selected from 1-3 hydroxy groups or halogen atoms, or a phenyl, phthalidyl, isoindolyl, dihydroindolyl, dihydroisoindolyl, dihydroisoquinolinyl, thiophthalidyl, benzoaxazinonyl, phthalazinonyl, quinolinyl, isoquinolinyl, quinolonyl, isoquinolonyl, indazolyl, benzothiazolyl, quinazolinyl, quinoxalinyl, cinnolinyl, phthalazinyl, 1,7- or 1,8-naphthyridinyl, dihydroindolonyl, dihydroisoindolonyl, benzimidazole or indolyl group that optionally is substituted by one or more groups selected from 1,2 keto groups, 1-2 (C₁-C₅)-alkyl groups, 1-2 (C₁-C₅)-alkoxy groups, 1-3 hydroxy groups, 1-3 halogen atoms, or 1-2 (C₁-C₃)-exoalkylidene groups,
where these groups can be linked via any position to the amine of the tetrahydronaphthalene system and optionally be hydrogenated at one or more sites,
R⁵ means a hydroxy group,
R⁶ means a (C₁-C₅)-alkyl group or an optionally partially or completely fluorinated (C₁-C₅)-alkyl group,
R⁷ and R⁸, independently of one another, mean a hydrogen atom, a halogen atom, a methyl or ethyl group, which should be substituted with OR¹⁰, SR¹⁰, or N(R⁹R¹⁰), or together with the carbon atom of the methylene group mean a (C₃-C₆)-cycloalkyl ring, or
R¹ and R⁸ together mean an annelated five- to eight-membered, saturated or unsaturated carbocycle or heterocycle, which optionally is substituted by 1-2 keto groups, 1-2 (C₁-C₅)-alkyl groups, 1-2 (C₁-C₅)-alkoxy groups, or 1-4 halogen atoms.

6. Stereoisomers of general formula (I) according to Claim 1, in which
R¹ and R², independently of one another, mean a hydrogen atom, a hydroxy group, a halogen atom, a (C₁-C₅)-alkyl group, a (C₁-C₅)-alkoxy group, or together a group that is selected from the groups -OH-(CH₂)ₙ-O-, -O- (CH₂)ₙ-CH₂-, -O-CH=CH-, - (CH₂)ₙ₊₂-,
where n = 1 or 2, and the terminal atoms are linked to directly adjacent ring carbon atoms,
R³ means a hydrogen atom, a hydroxy group, a halogen atom, an optionally substituted (C₁-C₁₀)-alkyl group, or a (C₁-C₁₀)-alkoxy group,
R⁴ means a phenyl, phthalidyl, isoindolyl, dihydroindolyl, dihydroisoindolyl, dihydroisoquinolinyl, thiophthalidyl, benzoaxazinonyl, phthalazinonyl, quinolinyl, isoquinolinyl, quinolonyl, isoquinolonyl, indazolyl, benzothiazolyl, quinazolinyl, quinoxalinyl, cinnolinyl, phthalazinyl, 1,7- or 1,8-naphthyridinyl, dihydroindolonyl, dihydroisoindolonyl, benzimidazole or indolyl group that optionally is substituted by one or more groups selected from 1,2 keto groups, 1-2 (C₁-C₅)-alkyl groups, 1-2 (C₁-C₅)-alkoxy groups, 1-3 hydroxy groups, 1-3 halogen atoms, or 1-2 (C₁-C₃)-exoalkylidene groups,
where these groups can be linked via any position to the amine of the tetrahydronaphthalene system and optionally be hydrogenated at one or more sites,
R⁵ means a hydroxy group,
R⁶ means a (C₁-C₅)-alkyl group or an optionally partially or completely fluorinated (C₁-C₅)-alkyl group,
R⁷ and R⁸, independently of one another, mean a hydrogen atom, a halogen atom, a methyl or ethyl group, or together with the carbon atom of the methylene group mean a (C₃-C₆)-cycloalkyl ring, or
R¹ and R⁸ together mean an annelated five- to eight-membered saturated or unsaturated carbocycle or heterocycle.

7. Stereoisomers of general formula (I) according to Claim 1, in which
R¹ and R², independently of one another, mean a hydrogen atom, a hydroxy group, a halogen atom, or a (C₁-C₅)-alkoxy group,
R³ means a hydrogen atom, or a halogen atom,
R⁴ means a quinolinyl, quinolonyl, phthalazinyl, phthalazinonyl, quinazolinyl or quinazolonyl group that optionally is substituted with C₁-C₅-alkyl, halogen, or keto groups, and where these groups can be linked to the amine of the tetrahydronaphthalene system via any position and optionally can be hydrogenated at one or more sites,
R⁵ means a hydroxy group,
R⁶ means an optionally partially or completely fluorinated (C₁-C₅)-alkyl group,
R⁷ and R⁸, independently of one another, mean a hydrogen atom, a methyl or ethyl group, or
R¹ and R⁸ together mean an annelated, five- to eight-membered saturated or unsaturated carbocycle or heterocycle, which optionally is substituted by a hydroxy group.

8. Stereoisomers of general formula (I) according to Claim 1, in which
R¹ and R², independently of one another, mean a hydrogen atom, a hydroxy group, a fluorine or chlorine atom, or a methoxy group,
R³ means a hydrogen atom, or a chlorine atom,
R⁴ means a quinolinyl, quinolonyl, quinazolinyl or phthalzinonyl group that optionally is substituted with one or more groups selected from methyl, hydroxy, keto group or fluorine atom, and where these groups can be linked to the amine of the tetrahydronaphthalene system via any poisiton and optionally can be hydrogenated at one or more sites,
R⁵ means a hydroxy group,
R⁶ means a trifluoromethyl group,
R⁷ and R⁸, independently of one another, mean a hydrogen atom, a methyl or ethyl group, or
R¹ and R⁸ together mean an annelated, six-membered heterocycle, which optionally is substituted by a hydroxy group.

9. Stereoisomers of general formula (I) according to Claim 1, in which R¹ and R⁸ together mean an annelated, six-membered heterocycle, which contains an oxygen atom and a boron atom and which optionally is substituted by a hydroxy group.

10. Use of the stereoisomers according to any one of the preceding claims for the production of a pharmaceutical.

11. Use of the stereoisomers of Claims 1-4 for the production of a pharmaceutical for the treatment of inflammatory diseases.

12. Pharmaceutical preparations comprising at least one stereoisomer according to Claim 1-4 or mixtures thereof and also pharmaceutically compatible vehicles.

13. Process for the production of stereoisomers of general formula I, in which the radicals, unless otherwise indicated, have the meanings that are defined in Claim 1, **characterized in that** stereoisomers of general (III) 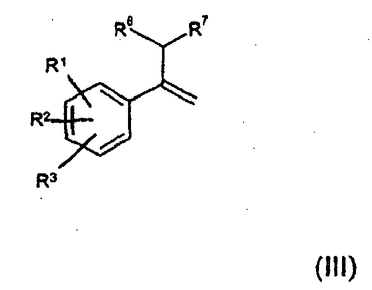 in which R¹, R², R³, R⁷ and R⁸ have the meanings that are mentioned in Claim 1, are converted by an optionally enantioselectively conducted ene reaction with α-keto acids R⁶ (CO) COOR¹² with R¹² in the meaning of (C₁-C₅)-alkyl or benzyl, in the presence of optionally chiral Lewis acids, into compounds of general formula (IV) 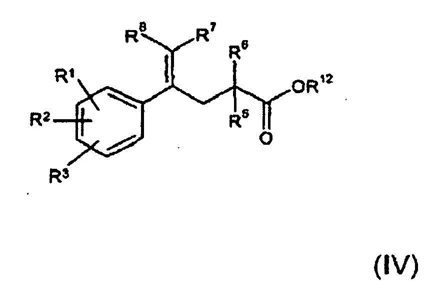 where R⁵ means a hydroxy group, which optionally can be converted into a protective group according to the other meanings that are defined for R⁵ in Claim 1, by reduction and reaction with amines of the formula R⁴-NH₂, where R⁴ has the meaning indicated in Claim 1, the compounds of general formula (V) are produced 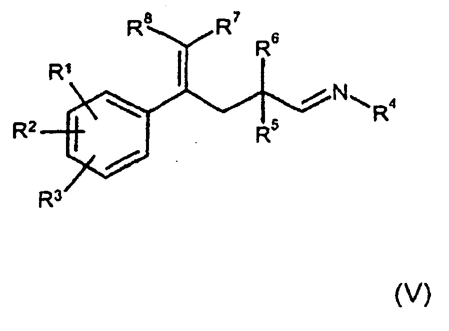 in which R¹, R², R³, R⁴ , R⁵, R⁶, R⁷ and R⁸ have the meanings that are indicated in Claim 1, then they are cyclized to compounds of general formula (I) either without additional reagent or by adding inorganic or organic acids or Lewis acids at temperatures of -70°C to 80°C.

14. Process for the production of stereoisomers of general formula I, **characterized in that** stereoisomers of general formula V 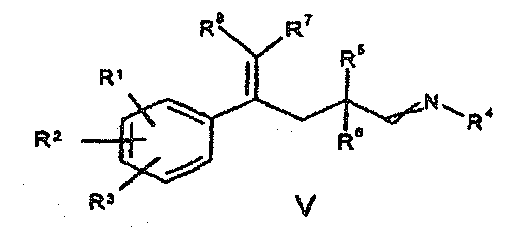 in which the radicals R¹, R², R³, R⁴, R⁵, R⁶, R⁷ and R⁸ have the meanings that are indicated in Claim 1, are cyclized optionally with the addition of inorganic or organic acids or Lewis acids, or **characterized in that** stereoisomers of general formula II 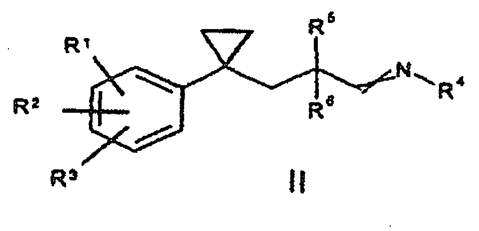 in which the radicals R¹, R², R³, R⁴, R⁵, and R⁶ have the meanings that are indicated in Claim 1, are cyclized and rearranged optionally with the addition of inorganic or organic acids or Lewis acids.

15. Process for the production of stereoisomers of general formula I according to Claim 13,
**characterized in that** stereoisomers of general formula (III) 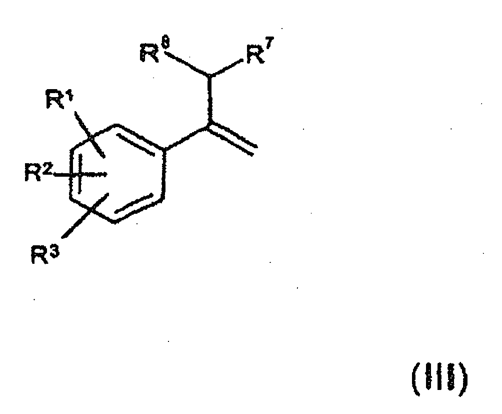 in which the radicals R¹, R², R³, R⁷ and R⁸ have the meanings that are indicated in Claim 1, are converted by an optionally enantioselectively conducted ene reaction with α-keto acids R⁶(CO)COOR¹² with R¹² in the meaning of (C₁-C₅)-alkyl or benzyl, in the presence of optionally chiral Lewis acids, into compounds of general formula (IV) 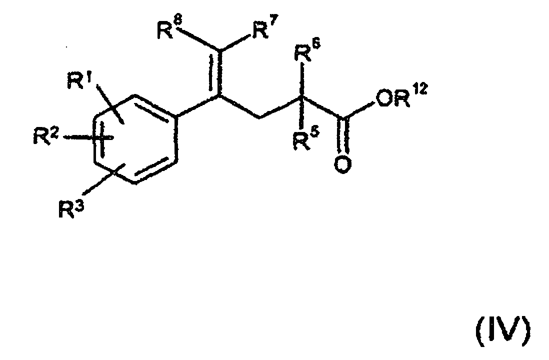 where R⁵ means a hydroxy group, which optionally can be converted into a protective group according to the other meanings that are defined for R⁵ in Claim 1.

16. Stereoisomers of formula IV 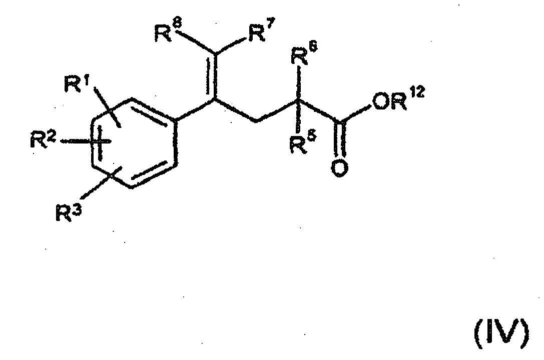 in which
R¹ and R², independently of one another, mean a hydrogen atom, a hydroxy group, a halogen atom, an optionally substituted (C₁-C₁₀)-alkyl group, (C₁-C₁₀) -alkoxy group, a (C₁-C₁₀)-alkylthio group, a (C₁-C₅)-perfluoroalkyl group, a cyano group, or a nitro group or
R¹ and R² together mean a group that is selected from the groups -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁ and -NH-N=CH-, where n = 1 or 2, and the terminal atoms are linked to directly adjacent ring carbon atoms, or NR⁹R¹⁰, where R⁹ and R¹⁰, independently of one another, can be hydrogen, C₁-C₅-alkyl or (CO) -C₁-C₅-alkyl,
R³ means a hydrogen atom, a hydroxy group, a halogen atom, an optionally substituted (C₁-C₁₀)-alkyl group, a (C₁-C₁₀)-alkoxy group, a (C₁-C₁₀) -alkylthio group, a (C₁-C₅)-perfluoroalkyl group, or a cyano group,
R⁵ means a hydroxy group, a group OR¹¹ or an O-(C¹¹)R¹¹ group, where R¹¹ means any hydroxy-protective group or a C₁-C₁₀-alkyl group,
R⁶ means a (C₁-C₅)-alkyl group or an optionally partially or completely fluorinated (C₁-C₅)-alkyl group, a (C₃-C₇)-cycloalkyl group, a (C₃-C₇)cycloalkyl(C₁-C₈)alkyl group, a (C₃-C₇)cycloalkyl(C₂-C₈)alkenyl group, a heterocyclyl group, a heterocyclyl(C₁-C₈)alkyl group, a heterocyclyl(C₂-C₈)alkenyl group, an aryl group, an aryl(C₁-C₈)alkyl group, an aryl(C₂-C₈)alkenyl group, an aryl(C₂-C₈)alkynyl group; a monocyclic or bicyclic heteroaryl group that optionally is substituted by one or more keto groups, (C₁-C₅)-alkyl groups, (C₁-C₅)-alkoxy groups, halogen atoms, or (C₁-C₃)-exoalkylidene groups and that contains one or more nitrogen atoms and/or oxygen atoms and/or sulphur atoms; a heteroaryl(C₁-C₈)alkyl group or a heteroaryl(C₂-C₈)alkenyl group, where these groups can be linked to the tetrahydronaphthalene system via any position and optionally can be hydrogenated at one or more sites,
R⁷ and R⁸, independently of one another, mean a hydrogen atom, a halogen atom, a (C₁-C₅)-alkyl group, which can be substituted with OR¹⁰, SR¹⁰, or N(R⁹R¹⁰),
or together with the carbon atom of the methylene group mean a (C₃-C₆)-cycloalkyl ring, or
R¹ and R⁸ together mean an annelated five- to eight-membered saturated or unsaturated carbocycle or heterocycle, which optionally is substituted by 1-2 keto groups, 1-2 (C₁-C₅)-alkyl groups, 1-2 (C₁-C₅)-alkoxy groups, 1-2 hydroxy groups, or 1-4 halogen atoms,
where at least one of the radicals R⁷ and R⁸ is not a hydrogen atom and R¹² means (C₁-C₅)-alkyl or benzyl.

17. Process for the production of stereoisomers of general formula I according to Claim 12, **characterized in that** stereoisomers of general formula (IV) 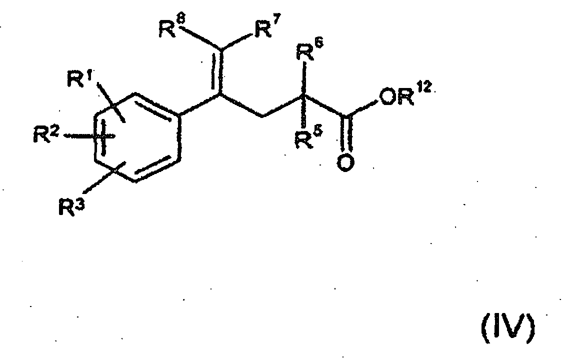 where R⁵ means a hydroxy group, which optionally can be converted into a protective group according to the other meanings that are defined from R⁵ in Claim 1, the radicals R¹, R², R³, R⁶, R⁷ and R⁸ have the meanings that are mentioned in Claim 1, and R¹³ means (C₁-C₅)-alkyl or benzyl, are converted by reduction and reaction with amines of the formula R⁴-NH₂, where R⁴ has the meaning indicated in Claim 1, into compounds of general formula (V) 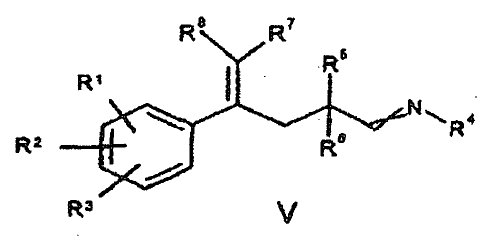 in which R¹, R², R³, R⁴, R⁵, R⁶, R⁷ and R⁸ have the meanings that are mentioned in Claim 1.

18. Stereoisomers of formula V according to Claim 17, 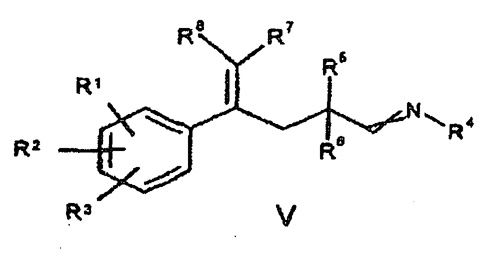 in which R¹, R², R³, R⁴, R⁵, R⁶, R⁷ and R⁸ have the meanings that are indicated in Claim 1.

19. Stereoisomers of general formula I, according to any one of Claims 1-9, 16 and 18, in the form of salts with physiologically compatible anions.

## Revendications

1. Stéréoisomères de formule générale (I), 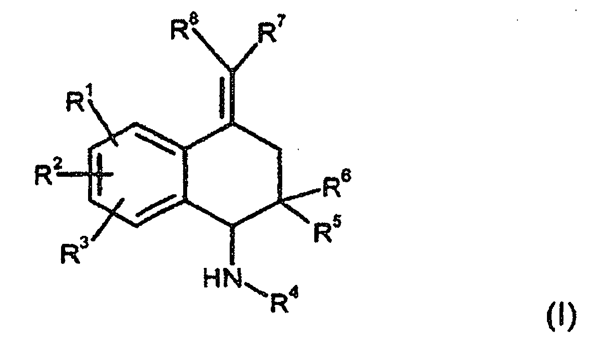 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl (C₁-C₁₀) thio, un groupe perfluoroalkyle en C₁-C₅, un groupe cyano, un groupe nitro, ou R¹ et R² forment ensemble un groupe choisi parmi les groupes -O-(CH₂)ₙ-O-, -O- (CH₂)ₙ-CH₂-,-O-CH=CH-, -(CH₂)ₙ₊₂-, -NH-(CH₂)ₙ₊₁-, -N[alkyle(C₁-C₃)]-(CH₂)ₙ₊₁-, -NH-N=CH-, n étant 1 ou 2 et les atomes en bout de chaîne étant liés à des atomes de carbone immédiatement voisins formant le cycle,
ou NR⁹R¹⁰, R⁹ et R¹⁰ pouvant être, indépendamment l'un de l'autre, un atome d'hydrogène, un groupe alkyle en C₁-C₅ ou (CO)-alkyle(C₁-C₅),
R³ représente un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano,
R⁴ représente un groupe alkyle en C₁-C₁₀, un groupe alkyle en C₁-C₁₀ substitué par un ou plusieurs groupes choisis parmi des groupes hydroxy, des atomes d'halogène, des groupes alcoxy en C₁-C₅,
un groupe cycloalkyle en C₃-C₇ éventuellement substitué,
un groupe hétérocyclyle éventuellement substitué,
un groupe aryle éventuellement substitué,
un groupe hétéroaryle mono- ou bicyclique éventuellement substitué par un ou plusieurs groupes choisis parmi des groupes alkyle en C₁-C₅ (qui peuvent éventuellement être substitués par 1-3 groupes hydroxy ou 1-3 groupes COOR¹²), des groupes alcoxy en C₁-C₅, des groupes hydroxy, des atomes d'halogène, 1-2 groupes exoalkylidène en C₁-C₃, et contenant éventuellement 1-4 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions, et R¹² représentant un groupe alkyle en C₁-C₅ ou un groupe benzyle,
R⁵ représente un groupe hydroxy, un groupe OR¹¹ ou un groupe O(CO)R¹¹, R¹¹ représentant un groupe protecteur quelconque de fonction hydroxy ou un groupe alkyle en C₁-C₁₀,
R⁶ représente un groupe alkyle en C₁-C₅ ou un groupe alkyle en C₁-C₅ éventuellement partiellement ou totalement fluoré, un groupe cycloalkyle en C₃-C₇, un groupe cycloalkyl(C₃-C₇)-alkyle(C₁-C₈), un groupe cycloalkyl(C₃-C₇)-alcényle(C₂-C₈), un groupe hétérocyclyle, un groupe hétérocyclylalkyle(C₁-C₈), un groupe hétérocyclylalcényle(C₂-C₈), un groupe aryle, un groupe aryl-alkyle(C₁-C₈), un groupe arylalcényle (C₂-C₈), un groupe arylalcynyle (C₂-C₈), un groupe hétéroaryle mono-ou bicyclique éventuellement substitué par un ou plusieurs groupes céto, groupes alkyle en C₁-C₅, groupes alcoxy en C₁-C₅, atomes d'halogène, groupes exoalkylidène en C₁-C₃, et contenant un ou plusieurs atomes d'azote et/ou atomes d'oxygène et/ou atomes de soufre,
un groupe hétéroaryl-alkyle(C₁-C₈) ou
un groupe hétéroaryl-alcényle(C₂-C₈),
ces groupes pouvant être liés par une position quelconque au système tétrahydronaphtalène et pouvant éventuellement être hydrogénés en une ou plusieurs positions,
R⁷ et R⁸ représentent, indépendamment l'un de l'autre, un atome d'hydrogène, un atome d'halogène, un groupe alkyle en C₁-C₅ qui peut être substitué par OR¹⁰, SR¹⁰, N(R⁹R¹⁰), ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-2 groupes hydroxy, 1-4 atomes d'halogène.

2. Stéréoisomères de formule générale I, dans lesquels
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano, un groupe nitro ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-, -NH- (CH₂)ₙ₊₁-, -N[alkyle (C₁-C₃)]- (CH₂)ₙ₊₁-, -NH-N=CH-, n étant 1 ou 2 et les atomes en bout de chaîne étant liés à des atomes de carbone immédiatement voisins formant le cycle,
ou NR⁹R¹⁰, R⁹ et R¹⁰ pouvant être, indépendamment l'un de l'autre, un atome d'hydrogène, un groupe alkyle en C₁-C₅ ou (CO)-alkyle(C₁-C₅),
R³ représente un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano,
R⁴ représente un groupe alkyle en C₁-C₁₀, un groupe alkyle en C₁-C₁₀ substitué par un ou plusieurs groupes choisis parmi des groupes hydroxy, des atomes d'halogène, des groupes alcoxy en C₁-C₅,
un groupe cycloalkyle en C₃-C₇ éventuellement substitué,
un groupe hétérocyclyle éventuellement substitué,
un groupe aryle éventuellement substitué,
un groupe hétéroaryle mono- ou bicyclique éventuellement substitué par un ou plusieurs groupes choisis parmi des groupes alkyle en C₁-C₅ (qui peuvent éventuellement être substitués par 1-3 groupes hydroxy ou 1-3 groupes COOR¹²), des groupes alcoxy en C₁-C₅, des groupes hydroxy, des atomes d'halogène, 1-2 groupes exoalkylidène en C₁-C₃, et contenant éventuellement 1-4 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions, et R¹² représentant un groupe alkyle en C₁-C₅ ou un groupe benzyle,
R⁵ représente un groupe hydroxy, un groupe OR¹¹ ou un groupe O(CO)R¹¹, R¹¹ représentant un groupe protecteur quelconque de fonction hydroxy ou un groupe alkyle en C₁-C₁₀,
R⁶ représente un groupe alkyle en C₁-C₅ ou un groupe alkyle en C₁-C₅ éventuellement partiellement ou totalement fluoré, un groupe cycloalkyle en C₃-C₇, un groupe cycloalkyl(C₃-C₇)-alkyle(C₁-C₈), un groupe cycloalkyl(C₃-C₇)-alcényle(C₂-C₈), un groupe hétérocyclyle, un groupe hétérocyclylalkyle(C₁-C₈), un groupe hétérocyclylalcényle(C₂-C₈), un groupe aryle, un groupe aryl-alkyle(C₁-C₈), un groupe arylalcényle(C₂-C₈), un groupe arylalcynyle(C₂-C₈), un groupe hétéroaryle mono-ou bicyclique éventuellement substitué par un ou plusieurs groupes céto, groupes alkyle en C₁-C₅, groupes alcoxy en C₁-C₅, atomes d'halogène, groupes exoalkylidène en C₁-C₃, et contenant un ou plusieurs atomes d'azote et/ou atomes d'oxygène et/ou atomes de soufre,
un groupe hétéroaryl-alkyle(C₁-C₈) ou
un groupe hétéroaryl-alcényle (C₂-C₈) ces groupes pouvant être liés par une position quelconque au système tétrahydronaphtalène et pouvant éventuellement être hydrogénés en une ou plusieurs positions,
R⁷ et R⁸ représentent, indépendamment l'un de l'autre, un atome d'hydrogène, un atome d'halogène, un groupe alkyle en C₁-C₅ qui peut être substitué par OR¹⁰, SR¹⁰, N(R⁹R¹⁰), ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-2 groupes hydroxy, 1-4 atomes d'halogène.

3. Stéréoisomères de formule générale I selon la revendication 1, dans lesquels
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano, un groupe nitro ou R¹ et R² forment ensemble un groupe choisi parmi les groupes -O-(CH₂)ₙ-O-, -O- (CH₂)ₙ-CH₂-, -O-CH=CH-, - (CH₂)n+₂-, -NH-(CH₂)ₙ₊₁-, -N[alkyle (C₁-C₃)]-(CH₂)ₙ₊₁-, -NH-N=CH-, n étant 1 ou 2 et les atomes en bout de chaîne étant liés à des atomes de carbone immédiatement voisins formant le cycle,
ou NR⁹R¹⁰, R⁹ et R¹⁰ pouvant être, indépendamment l'un de l'autre, un atome d'hydrogène, un groupe alkyle en C₁-C₅ ou (CO)-alkyle(C₁-C₅),
R³ représente un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano,
R⁴ représente un groupe alkyle en C₁-C₁₀, un groupe alkyle en C₁-C₁₀ substitué par un ou plusieurs groupes choisis parmi 1-3 groupes hydroxy, des atomes d'halogène, 1-3 groupes alcoxy en C₁-C₅, un groupe cycloalkyle en C₃-C₇ éventuellement substitué, un groupe hétérocyclyle éventuellement substitué, un groupe aryle éventuellement substitué, un groupe hétéroaryle mono- ou bicyclique éventuellement substitué par un ou plusieurs groupes choisis parmi des groupes alkyle en C₁-C₅ (qui peuvent éventuellement être substitués par 1-3 groupes hydroxy ou 1-3 groupes COOR⁶), des groupes alcoxy en C₁-C₅, des groupes hydroxy, des atomes d'halogène, des groupes exoalkylidène en C₁-C₃, et contenant éventuellement 1-4 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions,
R⁵ représente un groupe hydroxy, un groupe OR¹¹ ou un groupe O(CO)R¹¹, R¹¹ représentant un groupe protecteur quelconque de fonction hydroxy ou un groupe alkyle en C₁-C₁₀,
R⁶ représente un groupe alkyle en C₁-C₅ ou un groupe alkyle en C₁-C₅ éventuellement partiellement ou totalement fluoré, un groupe cycloalkyle en C₃-C₇, un groupe cycloalkyl(C₃-C₇)-alkyle(C₁-C₈), un groupe cycloalkyl(C₃-C₇)-alcényle(C₂-C₈), un groupe hétérocyclyle, un groupe hétérocyclylalkyle(C₁-C₈), un groupe hétérocyclylalcényle(C₂-C₈), un groupe aryle, un groupe aryl-alkyle(C₁-C₈), un groupe arylalcényle (C₂-C₈) , un groupe arylalcynyle(C₂-C₈), un groupe hétéroaryle mono-ou bicyclique éventuellement substitué par un ou plusieurs groupes céto, groupes alkyle en C₁-C₅, groupes alcoxy en C₁-C₅, atomes d'halogène, groupes exoalkylidène en C₁-C₃, et contenant un ou plusieurs atomes d'azote et/ou atomes d'oxygène et/ou atomes de soufre,
un groupe hétéroaryl-alkyle(C₁-C₈) ou
un groupe hétéroaryl-alcényle(C₂-C₈),
ces groupes pouvant être liés par une position quelconque au système tétrahydronaphtalène et pouvant éventuellement être hydrogénés en une ou plusieurs positions,
R⁷ et R⁸ représentent, indépendamment l'un de l'autre, un atome d'hydrogène, un atome d'halogène, un groupe alkyle en C₁-C₅ qui peut être substitué par OR¹⁰, SR¹⁰, N(R⁹R¹⁰), ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-4 atomes d'halogène.

4. Stéréoisomères de formule générale (I) selon la revendication 1, dans lesquels
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano, un groupe nitro ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-, n étant 1 ou 2 et les atomes en bout de chaîne étant liés à des atomes de carbone immédiatement voisins formant le cycle,
ou NR⁹R¹⁰, R⁹ et R¹⁰ pouvant être, indépendamment l'un de l'autre, un atome d'hydrogène, un groupe alkyle en C₁-C₅ ou (CO)-alkyle(C₁-C₅),
R³ représente un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano,
R⁴ représente un groupe alkyle en C₁-C₁₀, un groupe alkyle en C₁-C₁₀ substitué par un ou plusieurs groupes choisis parmi 1-3 groupes hydroxy, des atomes d'halogène, 1-3 groupes alcoxy en C₁-C₅, un groupe phényle éventuellement substitué, un groupe hétéroaryle mono- ou bicyclique éventuellement substitué par 1-2 groupes céto, 1-2 groupes alkyle en C₁-C₅, 1-2 groupes alcoxy en C₁-C₅, 1-3 groupes hydroxy, 1-3 atomes d'halogène, 1-2 groupes exoalkylidène en C₁-C₃, et contenant 1-4 atomes d'azote et/ou 1-2 atomes d'oxygène et/ou 1-2 atomes de soufre et/ou 1-2 groupes céto, ces groupes pouvant être liés par une position quelconque à l'amine du système tétrahydronaphtalène et pouvant éventuellement être hydrogénés en une ou plusieurs positions,
R⁵ représente un groupe hydroxy,
R⁶ représente un groupe alkyle en C₁-C₅ ou un groupe alkyle en C₁-C₅ éventuellement partiellement ou totalement fluoré, un groupe aryle, un groupe aryl-alkyle(C₁-C₈), un groupe aryl-alcényle(C₂-C₈), un groupe cycloalkyle en C₃-C₇, un groupe cycloalkyl(C₃-C₇)-alkyle(C₁-C₈), un groupe cycloalkyl (C₃-C₇)-alcényle (C₂-C₈),
R⁷ et R⁸ représentent, indépendamment l'un de l'autre, un atome d'hydrogène, un atome d'halogène, un groupe méthyle ou éthyle qui peut être substitué par OR¹⁰, SR¹⁰, N(R¹⁰)₂, ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-4 atomes d'halogène.

5. Stéréoisomères de formule générale (I) selon la revendication 1, dans lesquels
R¹ et R² représentent, indépendamment l'un de l'autre, un atome d'hydrogène, un groupe hydroxy, un atome d'halogène, un groupe alkyle en C₁-C₅ éventuellement substitué, un groupe alcoxy en C₁-C₅, ou
R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-,
n étant 1 ou 2 et les atomes en bout de chaîne étant liés à des atomes de carbone immédiatement voisins formant le cycle,
R³ représente un atome d'hydrogène, un groupe hydroxy, un atome d'halogène, un groupe alkyle en C₁-C₁₀ éventuellement substitué, un groupe alcoxy en C₁-C₁₀,
R⁴ représente un groupe alkyle en C₁-C₁₀, un groupe alkyle en C₁-C₁₀ substitué par un ou plusieurs groupes choisis parmi 1-3 groupes hydroxy ou atomes d'halogène, un groupe phényle, phtalidyle, iso-indolyle, dihydroindolyle, dihydro-iso-indolyle, dihydroisoquinolinyle, thiophtalidyle, benzoxazinonyle, phtalazinonyle, quinolinyle, isoquinolinyle, quinolonyle, isoquinolonyle, indazolyle, benzothiazolyle, quinazolinyle, quinoxalinyle, cinnolinyle, phtalazinyle, 1,7- ou 1,8-naphtyridinyle, dihydroindolonyle, dihydro-iso-indolonyle, benzimidazole ou indolyle éventuellement substitué par un ou plusieurs groupes choisis parmi 1-2 groupes céto, 1-2 groupes alkyle en C₁-C₅, 1-2 groupes alcoxy en C₁-C₅, 1-3 groupes hydroxy, 1-3 atomes d'halogène, 1-2 groupes exoalkylidène en C₁-C₃,
ces groupes pouvant être liés par une position quelconque à l'amine du système tétrahydronaphtalène et pouvant éventuellement être hydrogénés en une ou plusieurs positions,
R⁵ représente un groupe hydroxy,
R⁶ représente un groupe alkyle en C₁-C₅ ou un groupe alkyle en C₁-C₅ éventuellement partiellement ou totalement fluoré,
R⁷ et R⁸ représentent, indépendamment l'un de l'autre, un atome d'hydrogène, un atome d'halogène, un groupe méthyle ou éthyle qui peut être substitué par OR¹⁰, SR¹⁰, (R⁹R¹⁰), ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-4 atomes d'halogène.

6. Stéréoisomères de formule générale (I) selon la revendication 1, dans lesquels
R¹ et R² représentent, indépendamment l'un de l'autre, un atome d'hydrogène, un groupe hydroxy, un atome d'halogène, un groupe alkyle en C₁-C₅, un groupe alcoxy en C₁-C₅, ou forment ensemble un groupe choisi parmi les groupes -O-(CH₂)ₙ-O-, -O-(CH₂)ₙ-CH₂-, -O-CH=CH-, -(CH₂)ₙ₊₂-,
n étant 1 ou 2 et les atomes en bout de chaîne étant liés à des atomes de carbone immédiatement voisins formant le cycle,
R³ représente un atome d'hydrogène, un groupe hydroxy, un atome d'halogène, un groupe alkyle en C₁-C₁₀ éventuellement substitué, un groupe alcoxy en C₁-C₁₀,
R⁴ représente un groupe phényle, phtalidyle, iso-indolyle, dihydro-indolyle, dihydro-iso-indolyle, dihydro-isoquinolinyle, thiophtalidyle, benzoxazinonyle, phtalazinonyle, quinolinyle, isoquinolinyle, quinolonyle, isoquinolonyle, indazolyle, benzothiazolyle, quinazolinyle, quinoxalinyle, cinnolinyle, phtalazinyle, 1,7- ou 1,8-naphtyridinyle, dihydro-indolonyle, dihydro-iso-indolonyle, benzimidazole ou indolyle éventuellement substitué par un ou plusieurs groupes choisis parmi 1-2 groupes céto, 1-2 groupes alkyle en C₁-C₅, 1-2 groupes alcoxy en C₁-C₅, 1-3 groupes hydroxy, 1-3 atomes d'halogène, 1-2 groupes exoalkylidène en C₁-C₃,
ces groupes pouvant être liés par une position quelconque à l'amine du système tétrahydronaphtalène et pouvant éventuellement être hydrogénés en une ou plusieurs positions,
R⁵ représente un groupe hydroxy,
R⁶ représente un groupe alkyle en C₁-C₅ ou un groupe alkyle en C₁-C₅ éventuellement partiellement ou totalement fluoré,
R⁷ et R⁸ représentent, indépendamment l'un de l'autre, un atome d'hydrogène, un atome d'halogène, un groupe méthyle ou éthyle, ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons.

7. Stéréoisomères de formule générale (I) selon la revendication 1, dans lesquels
R¹ et R² représentent, indépendamment l'un de l'autre, un atome d'hydrogène, un groupe hydroxy, un atome d'halogène, un groupe alcoxy en C₁-C₅,
R³ représente un atome d'hydrogène, un atome d'halogène,
R⁴ représente un groupe quinolinyle, quinolonyle, phtalazinyle, phtalazinonyle, quinazolinyle ou quinazolonyle éventuellement substitué par alkyle en C₁-C₅, halogène, des groupes céto,
ces groupes pouvant être liés par une position quelconque à l'amine du système tétrahydronaphtalène et pouvant éventuellement être hydrogénés en une ou plusieurs positions,
R⁵ représente un groupe hydroxy,
R⁶ représente un groupe alkyle en C₁-C₅ éventuellement partiellement ou totalement fluoré,
R⁷ et R⁸ représentent, indépendamment l'un de l'autre, un atome d'hydrogène, un groupe méthyle ou éthyle, ou
R¹ et R⁸ forment ensemble un cycle carbocyclique ou hétérocyclique condensé, saturé ou insaturé, à cinq à huit chaînons, qui est éventuellement substitué par un groupe hydroxy.

8. Stéréoisomères de formule générale (I) selon la revendication 1, dans lesquels
R¹ et R² représentent, indépendamment l'un de l'autre, un atome d'hydrogène, un groupe hydroxy, un atome de fluor ou de chlore, un groupe méthoxy,
R³ représente un atome d'hydrogène ou un atome de chlore,
R⁴ représente un groupe quinolinyle, quinolonyle, quinazolinyle ou phtalazinonyle éventuellement substitué par un ou plusieurs groupes choisis parmi les groupes méthyle, hydroxy, céto ou l'atome de fluor,
ces groupes pouvant être liés par une position quelconque à l'amine du système tétrahydronaphtalène et pouvant éventuellement être hydrogénés en une ou plusieurs positions,
R⁵ représente un groupe hydroxy,
R⁶ représente un groupe trifluorométhyle,
R⁷ et R⁸ représentent, indépendamment l'un de l'autre, un atome d'hydrogène, un groupe méthyle ou éthyle, ou
R¹ et R⁸ forment ensemble un cycle hétérocyclique condensé à six chaînons, qui est éventuellement substitué par un groupe hydroxy.

9. Stéréoisomères de formule générale (I) selon la revendication 1, dans lesquels R¹ et R⁸ forment ensemble un cycle hétérocyclique condensé à six chaînons, qui contient un atome d'oxygène et un atome de bore et qui est éventuellement substitué par un groupe hydroxy.

10. Utilisation des stéréoisomères selon l'une quelconque des revendications précédentes, pour la fabrication d'un médicament.

11. Utilisation des stéréoisomères selon les revendications 1 à 4, pour la fabrication d'un médicament destiné au traitement de maladies inflammatoires.

12. Préparations pharmaceutiques contenant au moins un stéréoisomère selon l'une quelconque des revendications 1 à 4 ou des mélanges de tels stéréoisomères ainsi que des véhicules pharmaceutiquement acceptables.

13. Procédé pour la préparation des stéréoisomères de formule générale I, dans lesquels les radicaux, à moins d'indication contraire, ont les significations définies dans la revendication 1,
**caractérisé en ce qu'**on convertit des stéréoisomères de formule générale (III) 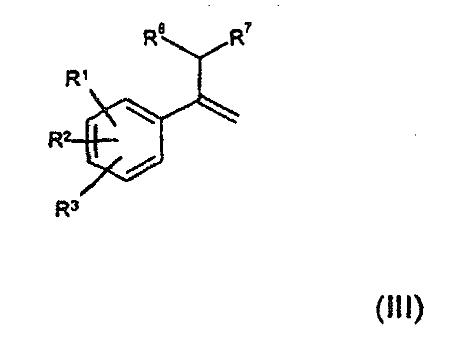 dans laquelle les radicaux R¹, R², R³, R⁷ et R⁸ ont les significations données dans la revendication 1, par une réaction d'ène éventuellement effectuée de façon énantiosélective, avec des α-cétoacides R⁶(CO)COOR¹², où R¹² a la signification d'un groupe alkyle en C₁-C₅ ou d'un groupe benzyle, en présence d'acides de Lewis éventuellement chiraux, en composés de formule générale (IV) 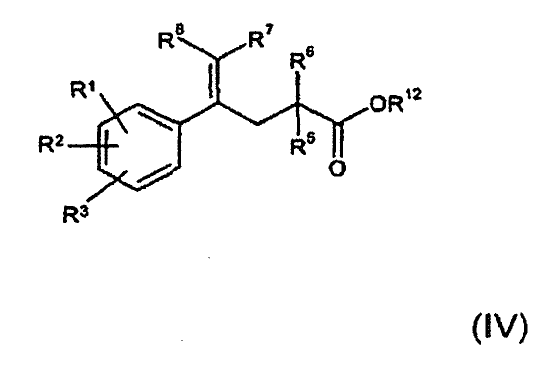 R⁵ représentant un groupe hydroxy qui peut éventuellement être converti en un groupe protecteur selon les autres significations définies pour R⁵ dans la revendication 1,
par réduction et réaction avec des amines de formule R⁴-NH₂,
R⁴ ayant la signification indiquée dans la revendication 1,
on prépare les composés de formule générale (V) 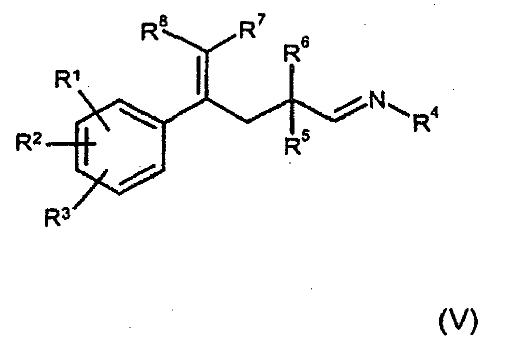 dans laquelle R¹, R², R³ R⁴, R⁵ R⁶, R⁷ et R⁸ ont les significations indiquées dans la revendication 1, puis on les cyclise en composés de formule générale (I) soit sans autre réactif, soit par addition d'acides organiques ou minéraux ou d'acides de Lewis, à des températures de -70 °C à 80 °C.

14. Procédé pour la préparation des stéréoisomères de formule générale I, **caractérisé en ce qu'**on cyclise des stéréoisomères de formule générale V 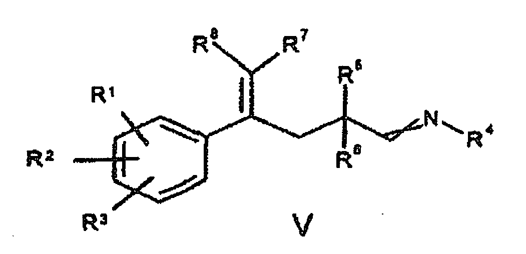 dans laquelle les radicaux R¹, R², R³, R⁴, R⁵, R⁶, R⁷ et R⁸ ont les significations indiquées dans la revendication 1, éventuellement avec addition d'acides organiques ou minéraux ou d'acides de Lewis, ou **caractérisé en ce qu'**on cyclise et transpose des stéréoisomères de formule générale II 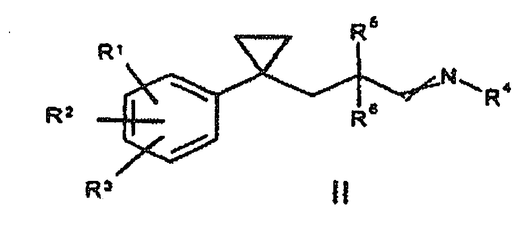 dans laquelle les radicaux R¹, R², R³, R⁴, R⁵ et R⁶ ont les significations indiquées dans la revendication 1, éventuellement avec addition d'acides organiques ou minéraux ou d'acides de Lewis.

15. Procédé pour la préparation des stéréoisomères de formule générale I selon la revendication 13,
**caractérisé en ce qu'**on convertit des stéréoisomères de formule générale (III) 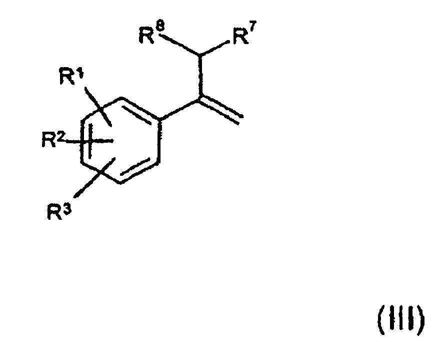 dans laquelle les radicaux R¹, R², R³, R⁷ et R⁸ ont les significations données dans la revendication 1, par une réaction d'ène éventuellement effectuée de façon énantiosélective, avec des α-cétoacides R⁶(CO)COOR¹², où R¹² a la signification d'un groupe alkyle en C₁-C₅ ou d'un groupe benzyle, en présence d'acides de Lewis éventuellement chiraux,
en composés de formule générale (IV) 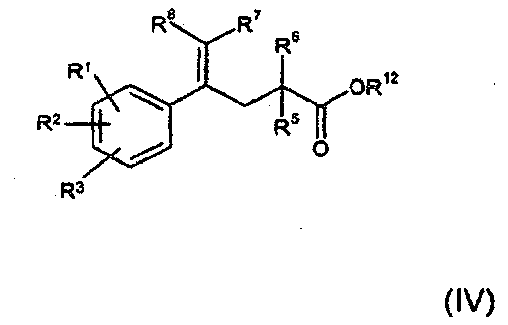 R⁵ représentant un groupe hydroxy qui peut éventuellement être converti en un groupe protecteur selon les autres significations définies pour R⁵ dans la revendication 1,

16. Stéréoisomères de formule IV 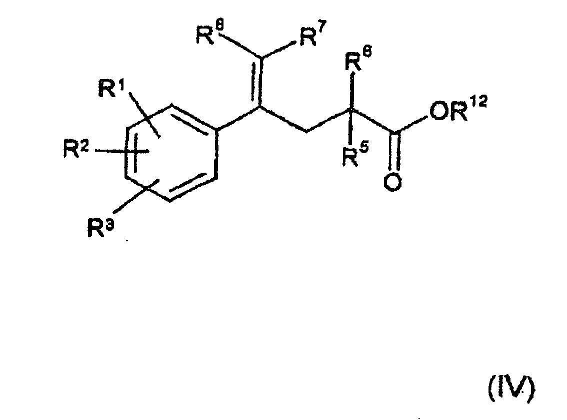 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano, un groupe nitro, ou R¹ et R² forment ensemble un groupe choisi parmi les groupes -O-(CH₂)ₙ-O-, -O- (CH₂)ₙ-CH₂-, -O-CH=CH-, - (CH₂)ₙ₊₂-, -NH-(CH₂)ₙ₊₁-, -N [alkyle (C₁-C₃)]- (CH₂)ₙ₊₁-, -NH-N=CH-, n étant 1 ou 2 et les atomes en bout de chaîne étant liés à des atomes de carbone immédiatement voisins formant le cycle,
ou NR⁹R¹⁰, R⁹ et R¹⁰ pouvant être, indépendamment l'un de l'autre, un atome d'hydrogène, un groupe alkyle en C₁-C₅ ou (CO)-alkyle(C₁-C₅),
R³ représente un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano,
R⁵ représente un groupe hydroxy, un groupe OR¹¹ ou un groupe O(CO)R¹¹, R¹¹ représentant un groupe protecteur quelconque de fonction hydroxy ou un groupe alkyle en C₁-C₁₀,
R⁶ représente un groupe alkyle en C₁-C₅ ou un groupe alkyle en C₁-C₅ éventuellement partiellement ou totalement fluoré, un groupe cycloalkyle en C₃-C₇, un groupe cycloalkyl (C₃-C₇)-alkyle (C₁-C₈), un groupe cycloalkyl (C₃-C₇)-alcényle (C₂-C₈), un groupe hétérocyclyle, un groupe hétérocyclylalkyle(C₁-C₈), un groupe hétérocyclylalcényle(C₂-C₈), un groupe aryle, un groupe aryl-alkyle (C₁-C₈), un groupe arylalcényle (C₂-C₈), un groupe arylalcynyle (C₂-C₈), un groupe hétéroaryle mono-ou bicyclique éventuellement substitué par un ou plusieurs groupes céto, groupes alkyle en C₁-C₅, groupes alcoxy en C₁-C₅, atomes d'halogène, groupes exoalkylidène en C₁-C₃, et contenant un ou plusieurs atomes d'azote et/ou atomes d'oxygène et/ou atomes de soufre,
un groupe hétéroaryl-alkyle(C₁-C₈) ou
un groupe hétéroaryl-alcényle(C₂-C₈),
ces groupes pouvant être liés par une position quelconque au système tétrahydronaphtalène et pouvant éventuellement être hydrogénés en une ou plusieurs positions,
R⁷ et R⁸ représentent, indépendamment l'un de l'autre, un atome d'hydrogène, un atome d'halogène, un groupe alkyle en C₁-C₅ qui peut être substitué par OR¹⁰, SR¹⁰, N(R⁹R¹⁰) , ou forment ensemble, conjointement avec l'atome de carbone du groupe méthylène, un cycle cycloalkyle en C₃-C₆, ou
R¹ et R⁸ forment ensemble un cycle carbocyclique ou hétérocyclique condensé, saturé ou insaturé, à cinq à huit chaînons, qui est éventuellement substitué par 1-2 groupes céto, 1-2 groupes alkyle en C₁-C₅, 1-2 groupes alcoxy en C₁-C₅, 1-2 groupes hydroxy, 1-4 atomes d'halogène,
au moins l'un des radicaux R⁷ et R⁸ n'étant pas un atome d'hydrogène et R¹² représentant un groupe alkyle en C₁-C₅ ou benzyle.

17. Procédé pour la préparation des stéréoisomères de formule générale I selon la revendication 12, **caractérisé en ce qu'**on convertit des stéréoisomères de formule générale (IV) 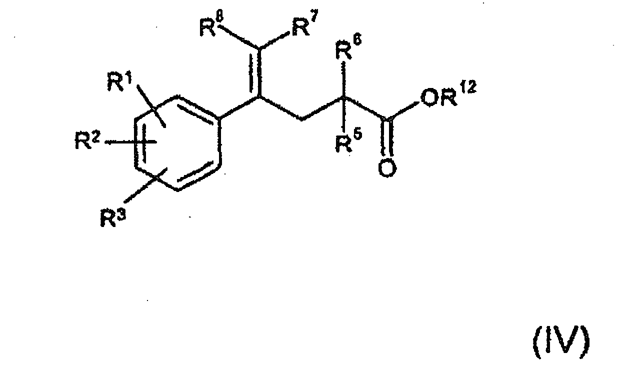 dans laquelle R⁵ représente un groupe hydroxy qui peut éventuellement être converti en un groupe protecteur selon les autres significations définies pour R⁵ dans la revendication 1, les radicaux R¹, R², R³, R⁶, R⁷ et R⁸ ont les significations indiquées dans la revendication 1 et R¹³ a la signification d'un groupe alkyle en C₁-C₅ ou d'un groupe benzyle, par réduction et réaction avec des amines de formule R⁴-NH₂,
R⁴ ayant les significations indiquées dans la revendication 1, en composés de formule générale (V) 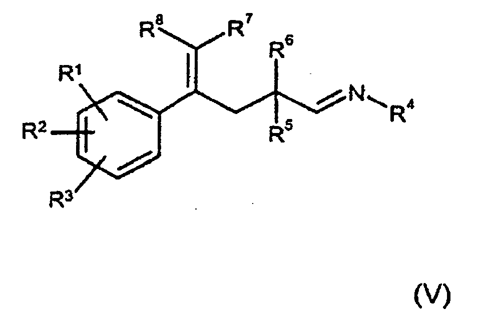 dans laquelle R¹, R², R³, R⁴, R⁵, R⁶, R⁷ et R⁸ ont les significations indiquées dans la revendication 1.

18. Stéréoisomères de formule V selon la revendication 17, 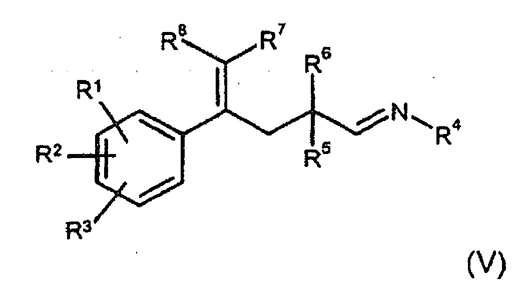 dans laquelle R¹, R², R³, R⁴, R⁵, R⁶, R⁷ et R⁸ ont les significations indiquées dans la revendication 1.

19. Stéréoisomères de formule générale I selon l'une quelconque des revendications 1-9, 16 et 18, sous forme des sels avec des anions physiologiquement acceptables.
